# EUROPEAN PATENT SPECIFICATION

(11) **EP 0 533 350 B1**
(45) Date of publication and mention of the grant of the patent: **26.05.1999**
(21) Application number: 92307479.3
(22) Date of filing: 14.08.1992
(51) Int. Cl.: C12N 15/57, C12N 9/64, C12P 21/02, C12P 21/08

(54) **DNA encoding precursor interleukin 1B converting enzyme**
DNS, welche das Interleukin-1B-Vorläufer-Converting-Enzym kodiert
ADN codant l'enzyme de conversion du précurseur de l'interleukine 1B

(30) Priority: 16.08.1991 US 746454
(43) Date of publication of application: 24.03.1993
(73) Proprietor: Merck & Co., Inc., Rahway New Jersey 07065-0900 (US)
(72) Inventor: Howard, Andrew D., Park Ridge, NJ 07656 (US); Calaycay, Jimmy R., Clark, NJ 07066 (US); Molineaux, Susan M., New York, NY 10003 (US); Miller, Douglas K., Westfield, NJ 07090 (US); Tocci, Michael J., Bridgewater, NJ 08807 (US)
(74) Representative: Barrett-Major, Julie Diane

(56) References cited:
- EP-A- 0 165 654
- EP-A- 0 364 778
- WO-A-91/15577
- NATURE vol. 356, no. 6372, 30 April 1992, LONDON GB pages 768 - 776 N.A. THORNBERRY ET AL. 'A novel heterodimeric cysteine protease is required for interleukin-1 beta processing in monocytes'
- SCIENCE vol. 256, no. 5053, 3 April 1992, WASHINGTON DC, US pages 97 - 100 D.P. CERRETTI ET AL. 'Molecular cloning of the interleukin-1 beta converting enzyme'
- ARCHIVES OF BIOCHEMISTRY AND BIOPHYSICS vol. 296, no. 2, 1 August 1992, US pages 698 - 703 S.R. KRONHEIM ET AL. 'Purification of interleukin-1 beta converting enzyme, the protease that cleaves the interleukin-1 beta precursor'
- JOURNAL CELLULAR BIOCHEMISTRY SUPPLEMENT vol. 0, no. 15, 1991, PART G, NEW YORK, US page 128 R.A. BLACK ET AL. 'Purification and molecular cloning of the IL-1-Beta processing enzyme'
- PROCEEDINGS OF THE NATIONAL ACADEMY OF SCIENCES OF USA. vol. 86, no. 14, July 1989, WASHINGTON US pages 5227 - 5231 M.J. KOSTURA ET AL. 'Identification of a monocyte specific pre-interleukin 1beta convertase activity'
- FEBS LETTERS vol. 247, no. 2, April 1989, AMSTERDAM, NL pages 386 - 390 R.A. BLACK ET AL. 'Activation of interleukin-1beta by a co-induced protease'
- JOURNAL OF BIOLOGICAL CHEMISTRY vol. 264, no. 10, 5 April 1989, BALTIMORE, US pages 5323 - 5326 R.A BLACK ET AL 'A pre-aspartate-specific protease from human leukocytes that cleaves pro-interleukin-1beta'
- NATURE vol. 315, 20 June 1985, LONDON GB pages 641 - 647 C.J.MARCH ET AL. 'Cloning, sequence and expression of two distinct human interleukin-1 complementary DNAs'

## Description

### BACKGROUND OF THE INVENTION

Mammalian interleukin-1 (IL-1) is an immunoregulatory protein secreted by certain cell types as part of the general inflammatory response. The primary cell type responsible for IL-1 production is the peripheral blood monocyte. Other non-transformed cell types have, however, been described as releasing or containing IL-1 or IL-1-like molecules. These include epithelial cells (Luger et al., J. Immunol. 127: 1493-1498 [1981], Le et al., J. Immunol. 138: 2520-2526 [1987] and Lovett and Larsen, J. Clin. Invest. 82: 115-122 [1988], connective tissue cells (Ollivierre et al., Biochem. Biophys. Res. Comm. 141: 904-911 [1986], Le et al., J. Immunol. 138: 2520-2526 [1987], cells of neuronal origin (Giulian et al., J. Exp. Med. 164: 594-604 [1986] and leukocytes (Pistoia et al., J. Immunol. 136: 1688-1692 [1986], Acres et al., Mol. Immuno. 24: 479-485 [1987], Acres et al., J. Immunol. 138: 2132-2136 [1987] and Lindemann et al., J. Immunol. 140: 837-839 [1988]. Transformed cell lines have also been shown to produce IL-1. These include monocytic leukemia lines P388D, J774, THP.1, U-937 (Krakauer and Oppenheimer, Cell. Immunol. 80: 223-229 [1983] and Matsushima et al., Biochem. 25: 3242-3429 [1986], EBV-transformed human B lymphoblastoid lines (Acres, et al., J. Immunol. 138: 2132-2136 [1987]) and transformed murine keratinocytes (Luger et al., J. Immunol. 125: 2147-2152 [1982]).

Biologically active IL-1 exists in two distinct forms, IL-1α with an isoelectric point of about 5.2 and IL-1β with an isoelectric point of about 7.0 with both forms having a molecular mass of about 17,500 (Bayne et al., J. Exp. Med. 163: 1267-1280 [1986] and Schmidt, J. Exp. Med. 160: 772 [1984]). The polypeptides appear evolutionarily conserved, showing about 27-33% homology at the amino acid level (Clark et al., Nucleic Acids Res. 14: 7897-7914 [1986]).

Mammalian IL-1β is synthesized as a cell associated precursor polypeptide of about 31.5 kDa (Limjuco et al., Proc. Natl. Acad. Sci. USA 83: 3972-3976 [1986]). Precursor IL-1β is unable to bind to IL-1 receptors and is biologically inactive (Mosley et al., J. Biol. Chem. 262: 2941-2944 [1987]). Biological activity appears dependent upon some form of proteolytic processing which results in the conversion of the precursor 31.5 kDA form to the mature 17.5 kDa form.

Recent studies suggest that the processing and secretion of IL-1β is specific to monocytes and monocytic cell lines (Matsushima et al., J. Immunol. 135:1132 [1985]) For example, fibroblasts and keratinocytes synthesize the IL-1β precursor, but have not been shown to actively process the precursor or secrete mature IL-1β (Young et al., J. Cell Biol. 107:447 (1988) and Corbo et al., Eur. J. Biochem. 169:669 [1987]).

Several observations support the hypothesis that the processing and secretion of IL-1β occurs by a unique pathway distinct from that used by classical secretory proteins. IL-1β molecules from five different species do not contain hydrophobic signal sequences (Lomedico et al., Nature 312:458 [1984], Auron et al., Proc. Natl. Acad. Sci. USA 81; 7907 [1984], Gray et al., J. Immunol. 137:3644 [1986], Maliszewski et al., Mol. Immunol. 25:429 [1988], Mori et al., Biochem. Biophys. Res. Commun. 150:1237 [1988], and Furutani et al., Nucleic Acid Res. 13:5869 [1985]. Furthermore, neither the human nor the murine IL-1β precursors, when synthesized in vitro, translocate across competent microsomal membranes, and despite the presence of N-linked carbohydrate addition sites, do not contain N-linked carbohydrate. Finally, light and electron microscopy studies immunolocalize IL-1β to the cytoplasm and fail to demostrate IL-1β in organelles that are involved in classical secretion (Bayne et al., J. Exp. Med. 163: 1267-1280 [1986] and Singer et al., J Exp. Med. 167: 389 [1988]).

In activated monocytes, pulse-chase experiments suggest that IL-1β secretion may be linked to processing. These experiments show that the intracellular pool of unprocessed precursor is chased to extracellular mature IL-1β (Hazuda et al., J. Biol. Chem. 263: 8473 [1989]. IL-1β precursor is occasionally found extracellularly but does not appear to contribute to the formation of 17 kDa IL-1β unless incubated at high concentrations in the presence of excess trypsin, chymotrypsin or collagenase (Hazuda et al., J. Biol. Chem. 264: 1689 [1989], Black et al., J. Biol. Chem. 263: 9437 [1988] and Hazuda et al., J. Biol. Chem. 265: 6318 [1990]). However, none of these proteinases appear capable of generating mature IL-1β terminating with Ala117.

Proteolytic maturation of precursor IL-1β to mature, 17 kDa IL-1β apparently results from cleavage between Asp116 and Ala117. An endoproteinase, termed Interleukin-1 Coverting Enzyme (ICE), that is capable of cleaving the IL-1β precursor at Asp116-Ala11, as well as at a homologous site at Asp27-Gly28, and generating mature IL-1β with the appropriate amino terminus at Ala117 has now been identified. The Asp at position 116 has been found to be essential for cleavage, since substitution of Ala (Kostura et al., Proc. Natl. Acad. Sci 86: 5227-5231 [1989] or other amino acids (Howard et al., J. Immunol., 147, 2964-9, 1991) for Asp inhibits this cleavage event.

ICE activity has been obtainable only from cells naturally producing the enzyme. Crude cell lysates with ICE activity are available from these cells (Black, R.A. et al., 1989, FEBS Lett., 247, pp 386-90; and Kostura, M.J. et al., 1989, P.N.A.S. USA, 86, pp 5227-31, Howard, A. et al., 1991, J.Immunol., in press). However, purification of ICE from natural sources does not yield the quantities required for extensive study or practical application of the enzyme.

### OBJECTS OF THE INVENTION

It is, accordingly, an object of the present invention to provide a cDNA encoding ICE, the recombinantly produced ICE being capable of converting pre-IL-1β to biologically active mature IL-1β with Ala¹¹⁷ as the amino-terminal amino acid. An additional object of the present invention is to provide expression vectors containing cDNA encoding full length ICE, or the individual 20 kDa and 10 kDa subunits of the enzyme. A further object of the present invention is to provide recombinant host cells containing cDNA encoding full length pre-IL-1β, ICE and/or the individual 20 kDa and 10 kDa subunits of the enzyme. An additional object is to provide a method for the coexpression of ICE and IL-1β in a recombinant host cell to produce biologically active IL-1β. A-further object of the present invention is to provide isolated 20 kDa-ICE subunit, and isolated 10 kDa ICE subunit. An additional object of the present invention is to provide full length ICE. Another object is to provide monospecific antibodies which bind to either the ICE 20 kDa or the 10 kDa subunit, and the use of these antibodies as diagnostic reagents.

### SUMMARY OF THE INVENTION

Complimentary DNAs (cDNAs) are identified from a monocytic cell line cDNA library, which encode the full length form, from which the individual 20 kDa and 10 kDa subunits of ICE are derived. The cDNAs are fully sequenced and cloned into expression vectors for expression in a recombinant host. The cDNAs are useful to produce recombinant full length ICE, as well as the individual 20 kDa and 10 kDa subunits of the enzyme.

### BRIEF DESCRIPTION OF THE DRAWING

Figure 1. Sodium dodecyl sulfate polyacrylamide gel electrophoregrams of purified ICE (panel A) and associated pre-IL-1β cleavage (enzymatic) activity (panel B). A.S. = ammonium sulfate; DE-F.T. = DEAE flow through; S.P. = sulfopropyl cation exchange; HIC = propyl hydrophobic interaction chromatography; TSK = TSK-125 size exclusion chromatography; HAP = hydroxyapatite column chromatography.

Figure 2. ICE activity which had been purified through an alternate purification scheme (A.S., DEAE, SP, HAP, TSK) was applied to a Propyl hydrophobic interaction column. Protein was eluted with a reverse salt gradient. Eluted proteins were dialysed and analyzed by SDS-PAGE and silver staining (panel A) as well as converting enzyme activity (panel B). Note the correlation between appearance in the eluate of the 22 and 10 kDa proteins (arrows) and ICE activity.

Figure 3. A. Illustrates the pH optimum of THP-1 S-300 Interleukin-1β converting enzyme activity. B. Illustrates a salt titration of Interleukin-1β converting enzyme activity.

Figure 4. Active site labeling of ICE by 14-C-iodoacetate.

Figure 5. Isoelectric point of native ICE.

Figure 6. Interleukin-1β converting enzyme equilibrium characteristics.

Figure 7. Interleukin-1β converting enzyme ss-glutathione stability.

Figure 8. Interleukin-1β converting enzyme ss-glutathione stability.

Figure 9. Rate constant for ICE association.

Figure 10. Molecular weight estimation of ICE: Size exclusion chromatography of GSSG treated or cystamine treated enzyme.

Figure 11. Panel A. C-4 reverse phase HPLC chromatogram of the 22 kDa and 10 kDa ICE subunits. Panel B. SDS-PAGE fractionation of the 22 kDa and 10 kDa ICE subunits following reverse phase HPLC.

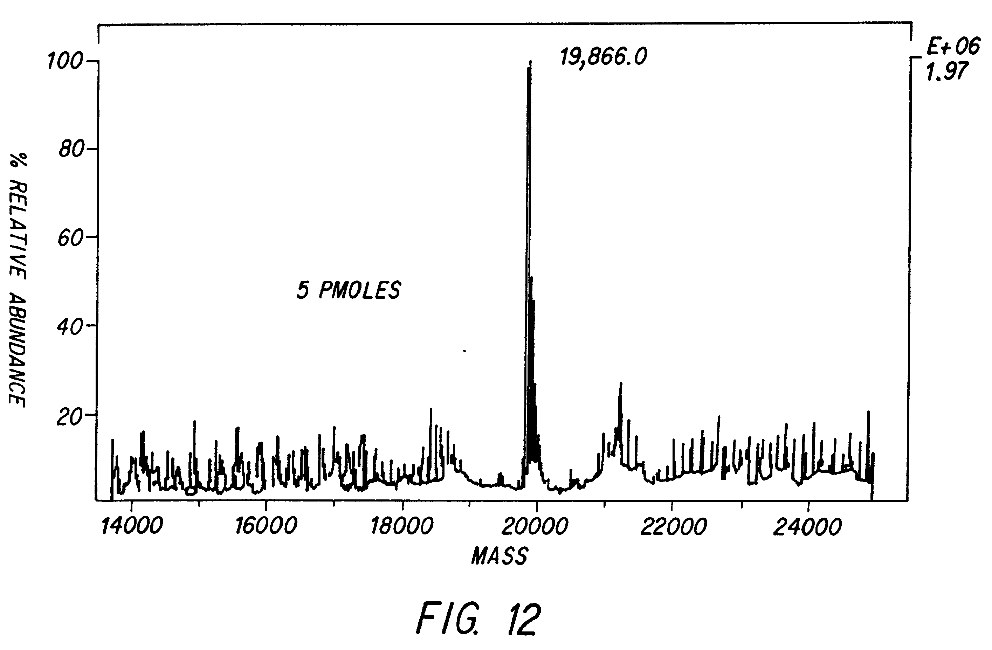

Figure 12. Capillary LC electrospray ionization mass spectrum of the 20 kDa ICE subunit.

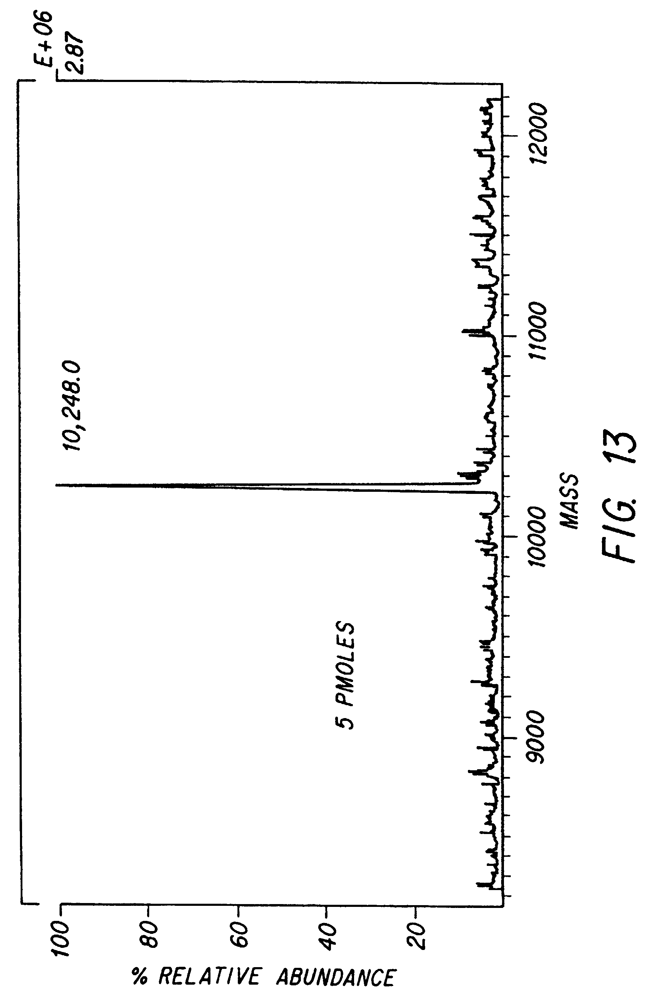

Figure 13. Capillary LC electrospray ionization mass spectrum of the 10 kDa ICE subunit.

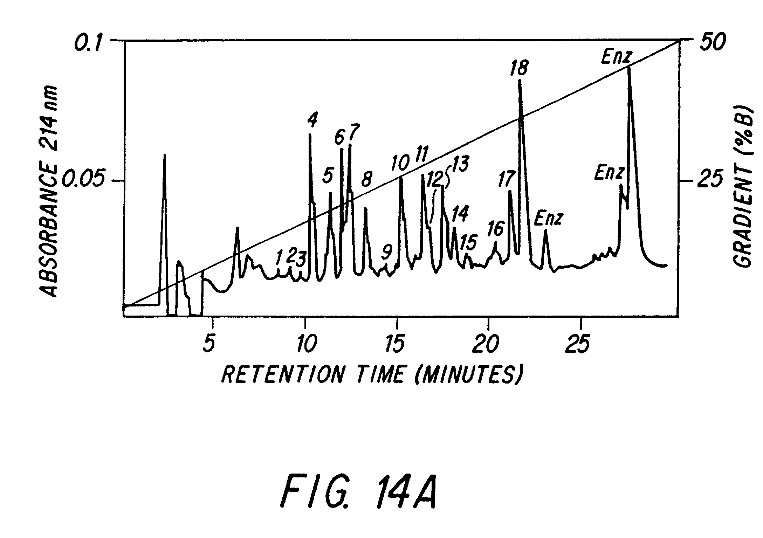

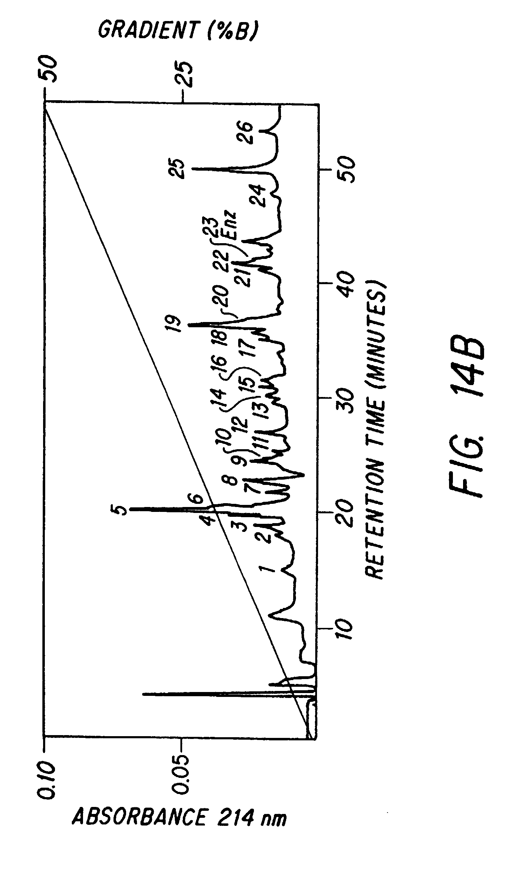

Figure 14. Comparative tryptic and Asp.N maps of the 20 kDa ICE subunit.

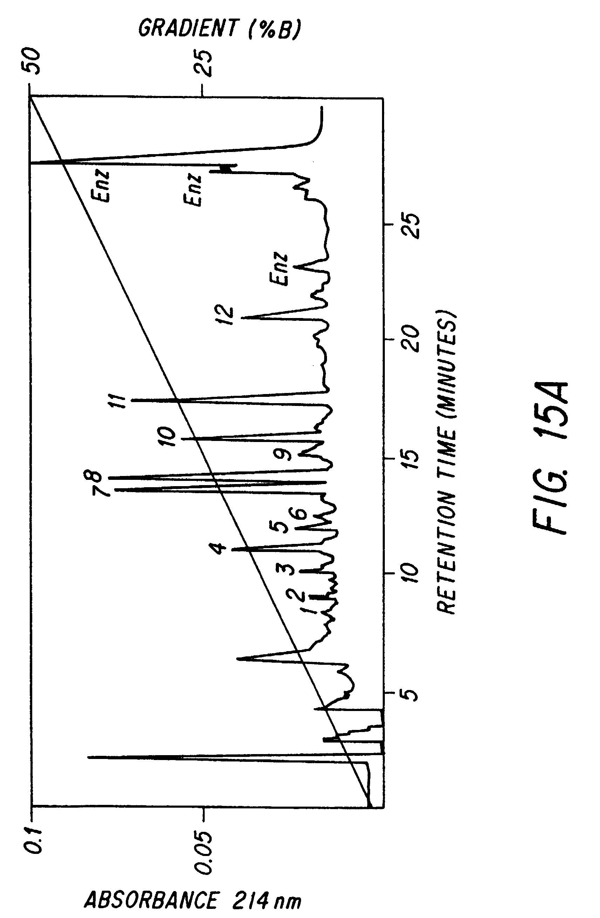

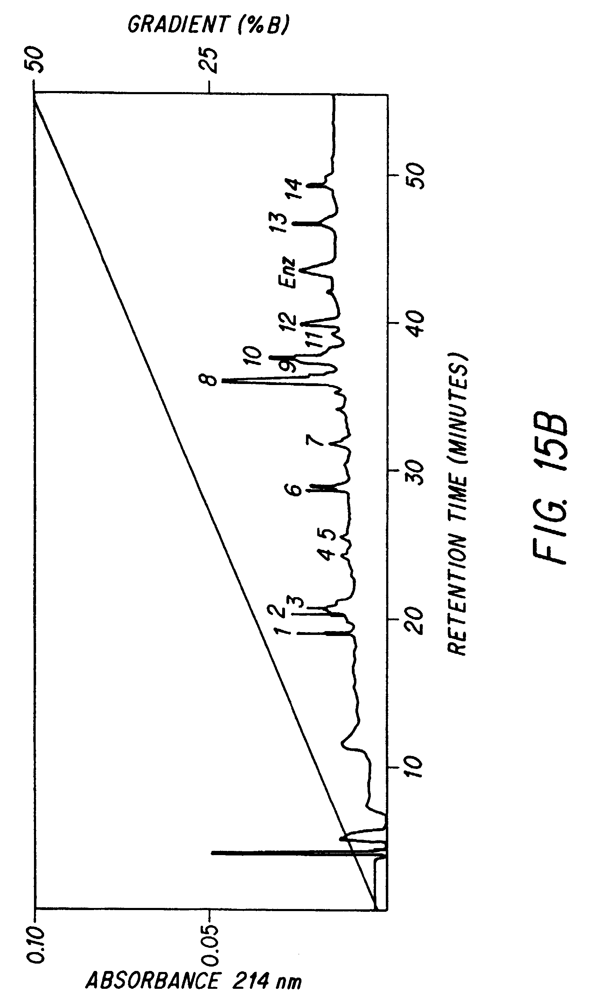

Figure 15. Comparative tryptic and Asp.N maps of the 10 kDa ICE subunit.

Figure 16. Comparison of amino terminal sequences from the 20 kDa ICE subunit and the 24 kDa protein.

Figure 17. Design of degenerate oligonucleotides for PCR of DNA fragments to the 20 kDa ICE subunit.

Figure 18. Design of degenerate oligonucleotides for PCR of DNA fragments to the 10 kDa ICE subunit.

Figure 19. Sequence of the PCR product for the 20 kDa ICE subunit.

Figure 20. Sequence of the PCR product for the 10 kDa ICE subunit.

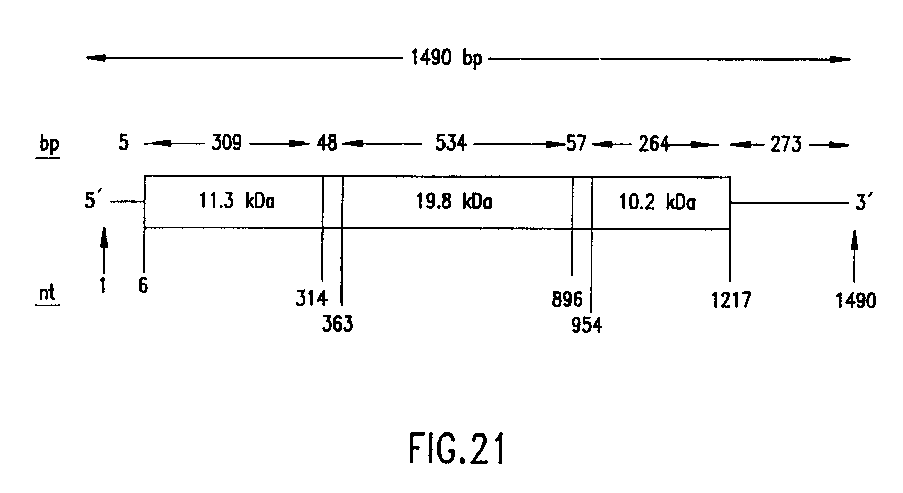

Figure 21. Structural organization of the human ICE cDNA.

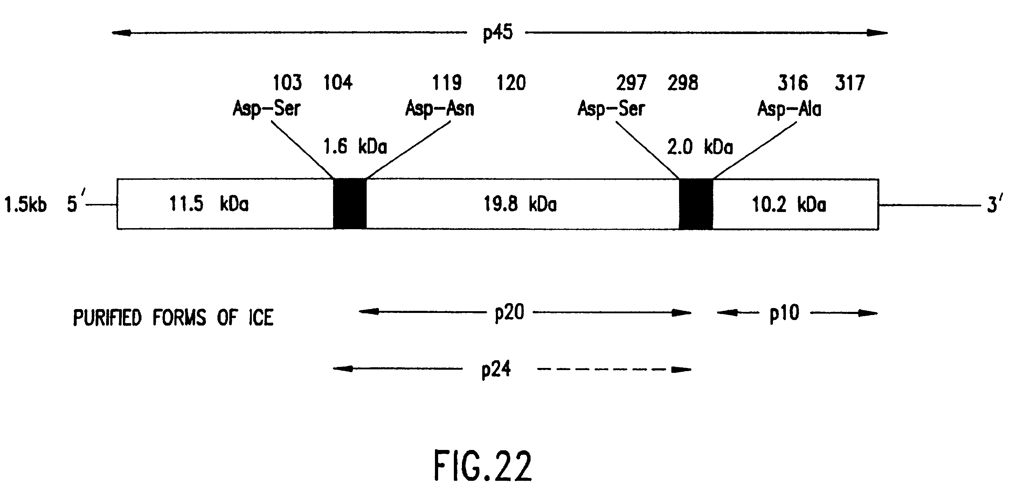

Figure 22. Structural organization of the human ICE precursor protein.

Figure 23. In vitro translation of ICE cDNA in rabbit reticulocyte lysates.

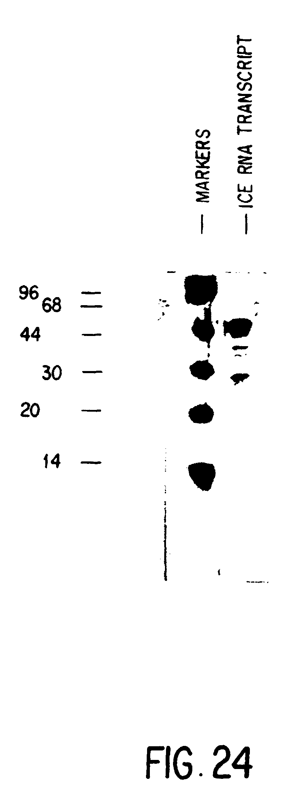

Figure 24. Location of the 24 kDa protein and the 20 kDa and 10 kDa ICE subunits in the ICE precursor.

Figure 25. Functional expression of ICE cDNA in transfected COS-7 cells.

### DETAILED DESCRIPTION

The present invention relates to cDNA encoding pre-IL-1β converting enzyme (ICE) which is isolated from IL-1 producing cells. ICE, as used herein, refers to an enzyme which can specifically cleave the peptide bond between the aspartic acid at position 116 (Asp¹¹⁶) and the alanine at position 117 (Ala¹¹⁷) of precursor Il-1β, and the peptide bond Asp at position 27 (Asp²⁷) and Gly at position 28 (Gly²⁸).

The amino acid sequence of human IL-1β is known, (March et al., Nature 315: 641-647 [1985]). Mammalian cells capable of producing IL-1β include, but are not limited to, keratinocytes, endothelial cells, mesangial cells, thymic epithelial cells, dermal fibroblasts, chondrocytes, astrocytes, glioma cells, monomuclear phagocytes, granulocytes, T and B lymphocytes and NK cells. Transformed mammalian cell lines which produce IL-1β include, but are not limited to, monocytic leukemia lines such as P388D1, J774, THP.1, Mono Mac 6 and U-937; EBV-transformed human B lymphoblastoid lines and transformed murine keratinocytes. The preferred cells for the present invention include normal human peripheral blood monocytes and THP.1 cells and the most preferred cells are THP.1 cells.

Other cells and cell lines may also be suitable for use to isolate ICE cDNA. Selection of suitable cells may be done by screening for ICE activity in cell extracts or conditioned medium. Methods for detecting ICE activity are well known in the art (Kostura, M.J. et al., 1989, P.N.A.S. USA, 86, pp.5227-5231) and measure the conversion of precursor IL-1β to mature IL-1β. Cells which possess ICE activity in this assay may be suitable for the isolation of ICE cDNA.

Human peripheral blood monocytes are obtained from healthy donors by leukophoresis and purified by sedimentation through Lymphocyte Separation Media (Organon Teknika) followed by elutriation on a Beckman counterflow centrifuge as described by Wicker, et al., Cell. Immunol. 106: 318-329 (1987). Monocytes are identified by labeling with anti-MACl antibody followed by FACS analysis using standard procedures known in the art.

The present invention relates to a unique pre-IL-1β converting enzyme (ICE), also described as pre-IL-1β convertase, which is isolated from IL-1 producing cells. Pre-IL-1β converting enzyme or covertase, as used herein, refers to an enzyme which can specifically cleave the peptide bond between aspartic acid at position 116 (Aspll6) and alanine at position 117 (Ala117) of the pre-IL-1β molecule.

Interleukin-1β producing cells such as human THP-1 cells (American Type Culture Collection, ATCC TIB 202) described by Tsuchiya et al., Int. J. Cancer 26: 171-176 (1980) are grown in suspension at about 37o C in, for example, Dulbecco's modified minimal essential medium (Hazelton Research Products) with about 10% fetal calf serum (HyClone; defined sera with no detectable endotoxin) or Iscove's Modified Dulbecco's Medium (JRH Biosciences) with about 9% horse serum. The cells are grown in roller bottles, Wheaton turbolift 46 liter suspension flasks (Wheaton), or 75, 200, or 300 liter fermenters with weekly harvests of about 1-2 x 106 cells/ml (3-4 doublings/week). Media for use in suspension flasks or fermenters may contain about 0.1-0.3 % F6 pluronic to reduce shear force on the cells. Cells are typically grown for no more than 3-4 months following initial culturing.

Cell-free extracts are prepared from human peripheral blood monocytes or THP.1 cells by disruption of the cells by nitrogen cavitation , hypotonic lysis or the like. The cells are collected by centrifugation and may be washed in an isotonic buffer solution such as phosphate buffered saline, pH about 7.4. Hypotonic lysis is accomplished by washing the cells in about 10 volumes of hypotonic buffer (about 10 mM KCl, about 20 mM HEPES, about pH 7.4, about 1.5 mM MgC12, about 0.1 mM EDTA) or (about 25 mM HEPES, about pH 7.5, about 5 mM MgC12, and 1 mM EGTA) and collected by centrifugation. The lysis buffer may also contain a reducing agent such as dithiothreitol (DTT). The hypotonic buffer will generally contain protease inhibitors such as PMSF, leupeptin and pepstatin. The cells are resuspended in about 3 volumes of hypotonic buffer, placed on ice for about 20 min and lysed by about 20 strokes in a Dounce homogenizer. Disruption of about 90 to about 95 % of the cells is obtained in a 100 or 300 ml tight filling Dounce homogenizer using about 25 or about 15 strokes respectively. Nitrogen pressure disruption also takes place in a hypotonic buffer. Resuspended cells are placed in a nitrogen pressure cell at 400 psi of nitrogen for about 30 min at about 4o C with agitation. Disruption is accomplished by releasing the pressure and evacuating the cells from the pressure cell. The cell lysate is clarified by successive centrifugation steps; at about 400 to about 1000 x g (supernatant S1), at about 30,000 x g (supernatant S2) and at about 300,000 x g (supernatant S3). The cell lysate may also be clarified by the following procedure. Unbroken cells and nuclei are removed by centrifugation at about 3000 rpm, for about 10 minutes, at about 5° C in a Beckman GPR centrifuge. The post nuclear supernatant fluid is centrifuged for about 20 minutes at about 16,000 rpm in a Sorval centrifuge with a SS34 rotor. The supernatant fluid is further clarified by centrifugation for about 60 minutes at about 50,000 rpm in a Beckman centrifuge (50.2Ti rotor) or 45,000 rpm (45Ti rotor). The resultant supernatant fluid is stored at about -80o C following the addition of about 2 mM DTT and 0.1% CHAPS.

Purification of ICE is monitored by an in vitro cleavage assay utilizing radiolabeled pre-IL-1β as a substrate. An approximately 1.5 kilobase (kb) cDNA clone containing the entire coding sequence of pre-IL-1β is inserted into EcoRI-PstI cleaved pGEM-3 plasmid DNA (Promega-Biotec) and propagated in E. coli according to standard methods (Maniatis et al., Molecular Cloning, A Laboratory Manual, Cold Spring Harbor, Cold Spring Harbor, NY [1982]). Purified plasmid is linearized with PstI and then transcribed using a T7 RNA polymerase in vitro transcription system (Promega-Biotec) and then the mRNA processed according to the manufacturers' instructions. Translations are preformed by programing micrococcal nuclease-treated rabbit reticulocyte extracts (Promega Biotec) with the in vitro synthesized mRNA in the presence of 25 µCi of 35S-Methionine (Amersham) according to the manufacturers instructions. This yielded labeled pre-IL-1β which migrated as a doublet on sodium dodecyl sulfate polyacrylamide gel electrophoresis (SDS-PAGE) with an apparent molecular mass of about 34 and about 31kilodalton (kDa). The cleavage of pre-IL-1β is preformed by incubating 1 µl of rabbit reticulocyte extract containing radiolabeled precursor with about 10 to about 20 µl of the sample containing IL-1β converting enzyme. Cleavage of pre-IL-1β to yield 17.5 kDa mature Il-1β is assayed by SDS-PAGE according to the method of Laemmli (Nature 227: 680-685 [1970]), followed by fluorography using procedures known in the art. Specificity of enzymatic cleavage and characterization of cleavage products are determined with native pre-IL-1β and mutant pre-IL-1β. Construction of a mutant pre-IL-1β is preformed by site directed oligonucleotide mutagenesis which is well known in the art. A synthetic double-stranded 27 nucleotide (27-mer) long oligodeoxyribonucleotide, corresponding to amino acids 115-126, with ApaLI-HpaII ends is synthesized on an Applied Biosystems DNA 380A synthesizer according to established manufacturer's protocols. The 27-mer encodes an Asp116 → Ala116 amino acid substitution at the -1 position adjacent to the processing site of pre-IL-1β. The oligonucleotide is ligated by procedures well known in the art to EcoRI-ApaLI and HpaII-PstI fragments obtained from cleavage of full length pre-IL-1β cDNA. The human nucleotide and predicted amino acid sequence of the pre-IL-1β translation product is disclosed by March et al., Nature 315: 641-647 (1985). The ligated fragments are added to a ligation reaction containing EcoRI-PstI cleaved pGEM-3. Clones containing the pGEM/IL-1β mutant are identified by hybridization with the mutant oligonucleotide sequence. Clones are mapped by restriction endonuclease cleavage and the DNA sequenced to verify the authenticity of the mutation. Transcription of the vector bearing the mutant or native constructs produced a 1.5 kilobase (Kb) mRNA and translation results in a doublet of 34 and 31 kDa proteins.

When THP-1 purified pre-IL-1β converting enzyme is combined with the mutated Asp116 to Ala116 pre-IL-1β, little or no cleavage of the mutant precursor was observed. The normal cleavage product from the interaction of pre-IL-1β with pre-IL-1β converting enzyme is a 17.5 kD polypeptide with the N-terminal amino acid sequence of mature IL-1β. The Asp116 residue of pre-IL-1β is therefore important to the processing of mature IL-1β by IL-β converting enzyme.

The bioactivity of the cleavage products is quantitated by determining the amount of radiolabeled, processed IL-1β which binds to IL-1 membrane receptors. The assay utilizes the techniques of Chin et al., J. Exp. Med. 165: 70-86 (1987) and Tocci et al., J. Immunol. 138: 1109-1114 (1987). The cleavage product generated from wild type or native pre-IL-1β by pre-IL-1β converting enzyme is biologically active as determined by its ability to bind IL-1β receptor in a competitive receptor-binding assay as disclosed above.

These purification procedures yield substantially pure, biologically active ICE.

To understand the cellular location of pre-IL-1β converting enzyme activity, cleavage studies are carried out using mononuclear cell fractions prepared by Percoll gradient fractionation. Cellular homogenate from the first clarification step is layered over preformed 0-100 % Percoll gradients (Pharmacia) prepared with about 0.25 M sucrose, about 10 mM HEPES, pH 7.4, 10 mM KCl, 1.5 mM MgC12 and 0.1 mM EDTA. The loaded gradients are centrifuged at about 48,000 x g for about 25 min with 1.0 ml fractions being collected from the top. Enzymatic marker assays associated with the various subcellular compartments are carried out: cytosol, lactate dehydrogenase (Morgorstern et al., Anal. Biochem. 13: 149-161 [1965]); lysosomes, N-Acetyl β-D-glusosaminidase (Wollen et al., Biochem. 78: 111-121 [1961]); plasma membrane, 5'-nucleotidase (Rome et al., Cell 17: 143-153 [1979]) and microsomes, sulfatase C (Canonico et al., J. Reticulo. Soc. 24: 115-135 [1978]). The cytosolic fraction was the only fraction capable of cleaving pre-IL-1β into a product similar in size to mature IL-1β.

Since the converting enzyme activity was present in the cytosolic fraction, further purification steps were carried out on supernatant S3 to obtain a substantially pure IL-1β converting enzyme. The supernatant is sequentially precipitated by the addition of granular ammonium sulfate to achieve about 45% of saturation at about 4o C. The precipitated protein is pelleted at about 30,000 x g then brought from about 75% to about 80% of saturation with ammonium sulfate. This precipitate is pelleted, resuspended in Buffer A (about 20 mM KCl, about 25 mM HEPES, about pH 7.4, about 5.0 mM EDTA, about 2 mM DTT, about 1 mM PMSF, about 0.01% NP-40 and about 10% glycerol) and dialyzed for about 16 hr. The dialyzed solution is centrifuged at about 30,000 x g to remove particulate material.

A sample of the ammonium sulfate precipitated material is applied to a diethylaminoethyl (DEAE) anion exchange column equilibrated with Buffer A. The flow through fraction is retained and loaded onto a sulfyl propyl (SP) cation exchange column equilibrated with the same buffer. Pre-IL-1β converting enzyme is eluted with a linear gradient of about 30-500 mM KCl in Buffer A. The active fractions are dialyzed against Buffer A for about 16 hr. Pre-IL-1β converting enzyme eluted as a discreet peak with 50% recovery of activity (Table 1).

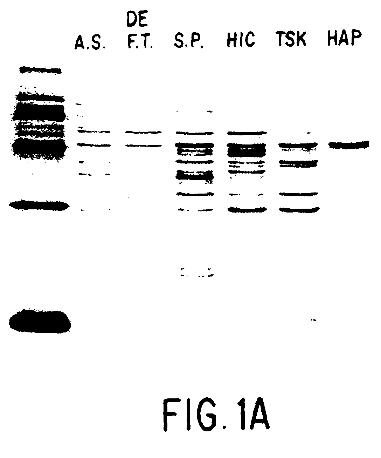

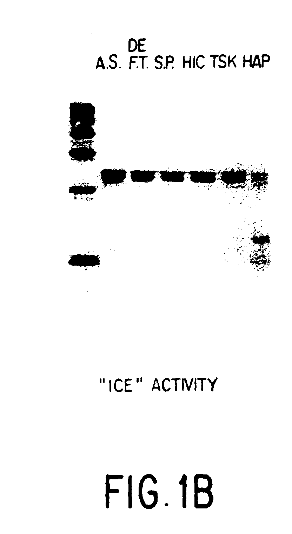

To analyze the protein components of biologically active ICE, sodium dodecyl sulfate polyacrylamide gel electophoresis was carried out on samples from the above fractionation steps essentially according to the method of Laemmli (Laemmli, Nature 227: 680-685 [1970]). After electrphoresis, separated proteins are visualized by staining with silver using a modification of the method developed by Oakley et al. (Analytical Biochemistry, 105:361-363 [1980]). The electrophetic patterns from the stepwise purification of ICE (Figure 1) demonstrate the appearance of a 22 kDa and 10 kDa protein in the final TSK and HAP steps that correlates with the ICE activity. In addition, the presence of a 24 kDa protein in column fractions of highly purified ICE is sometimes observed.

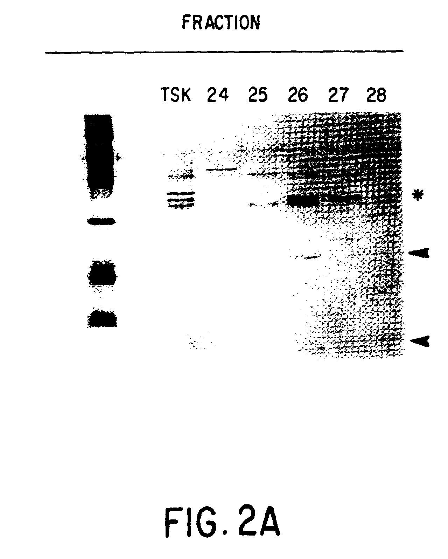

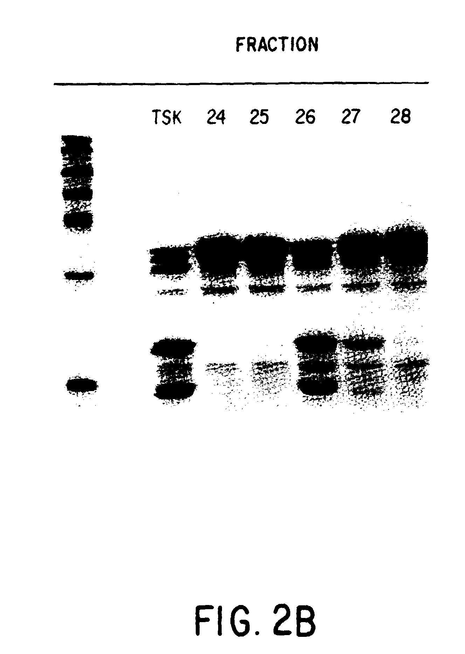

The 22 kDa and 10 kDa proteins can be individually isolated using norrowbore C4 reverse phase HPLC. A 200 ml aliquot of SP purified ICE can be applied to an Applied Biosystems C4 (2.1 mm X 100 mm, 300 A pore size) reverse phase column equilibrated in 0.1% trifluroacetic acid (TFA) in deionized water. Protein is eluted using a linear gradient of 15-90% acetonitrile in 0.1% aqueous TFA in 26 minutes. The 22 kDa and 10 kDa components can be isolated in pure form from the column (Figure 2).

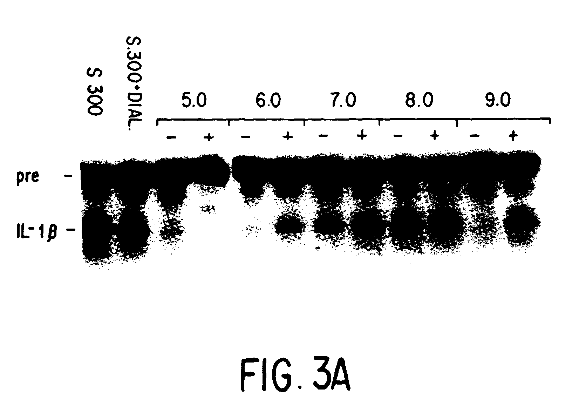

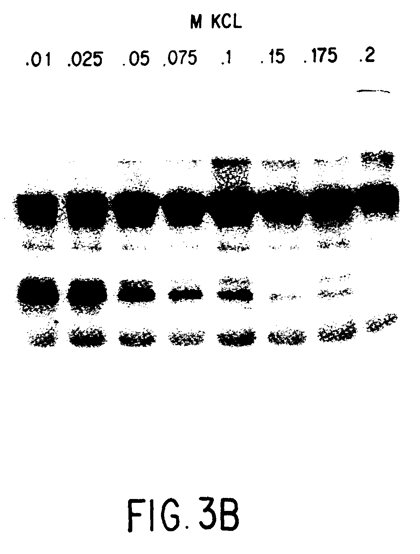

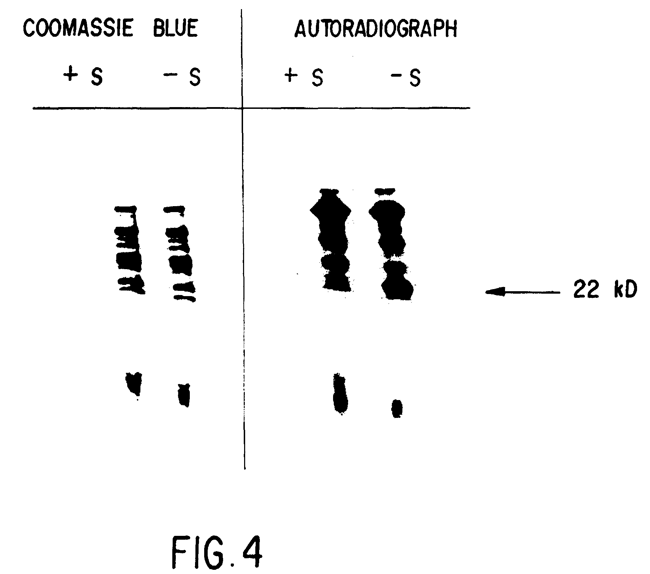

In order to obtain a highly accurate determination of the molecular mass of the 22 kDa and 10 kDa ICE subunits, five picomoles of each protein was purified to homogeneity by the above method and was subjected to capillary liquid chromatography on line to a Finnigan Triple-Sector Quadropole Model 700 electropsray mass spectrometer. The standard error of mass determination was found to be 0.01-0.02% using a standard protein, bovine cytochrome c, to determine instrument accuracy. Following biomass deconvolution of the electrospray ionization primary spectrum, the molecular weight of the isolated "10 kDa" component was found to have an average mass of 10,248 atomic mass units (Figure 4) and the "22 kDa" component was found to have an average mass of 19,866 atomic mass units (Figure 3). Herein, the "22 kDa" ICE subunit will be referred to as the 20 kDa subunit while the "10 kDa" ICE subunit will be referred to as the 10 kDa ICE subunit. These two proteins are associated with the fully active form of ICE. The "24 kDa" protein that occasionally co-purifies with the 20 and 10 kDa ICE subunits will be referred to as the 22 kDa protein, as it is approximately 2000 daltons larger than the 20 kDa ICE subunit when analyzed by SDS-PAGE.

Any of a variety of procedures may be used to molecularly clone ICE cDNA. These methods include, but are not limited to, direct functional expression of the ICE gene following the construction of an ICE-containing cDNA library in an appropriate expression vector system. Another method is to screen an ICE-containing cDNA library constructed in a bacteriophage or plasmid shuttle vector with a labelled oligonucleotide probe designed from the amino acid sequence of the ICE subunits. The preferred method consists of screening an ICE-containing cDNA library constructed in a bacteriophage or plasmid shuttle vector with a partial cDNA encoding the ICE subunits. This partial cDNA is obtained by the specific PCR amplification of ICE DNA fragments through the design of degenerate oligonucleotide primers from the amino acid sequence of the purified ICE subunits.

It is readily apparent to those skilled in the art that other types of libraries, as well as libraries constructed from other cells or cell types, may be useful for isolating ICE-encoding DNA. Other types of libraries include, but are not limited to, cDNA libraries derived from other cells or cell lines other than THP.1 cells, and genomic DNA libraries.

It is readily apparent to those skilled in the art that suitable cDNA libraries may be prepared from cells or cell lines which have ICE activity. The selection of cells or cell lines for use in preparing a cDNA library to isolate ICE cDNA may be done by first measuring cell associated ICE activity using the precursor IL-1β cleavage assay described fully above.

Preparation of cDNA libraries can be performed by standard techniques well known in the art. Well known cDNA library construction techniques can be found for example, in Maniatis, T., Fritsch, E.F., Sambrook, J., Molecular Cloning: A Laboratory Manual (Cold Spring Harbor Laboratory, Cold Spring Harbor, New York, 1982).

It is also readily apparent to those skilled in the art that DNA encoding ICE may also be isolated from a suitable genomic DNA library.

Construction of genomic DNA libraries can be performed by standard techniques well known in the art. Well known genomic DNA library construction techiques can be found in Maniatis, T., Fritsch, E.F., Sambrook, J. in Molecular Cloning: A Laboratory Manuel (Cold Spring Harbor Laboratory, Cold Spring Harbor, New York, 1982).

In order to clone the ICE gene by the preferred method, the amino acid sequence of ICE is necessary. To accomplish this, ICE protein may be purified and partial amino acid sequence determined by automated sequenators. It is not necessary to determine the entire amino acid sequence, but the linear sequence of two regions of 6 to 8 amino acids from both the 20 kDa and 10 kDa subunits is determined for the PCR amplification of a partial ICE DNA fragment.

Once suitable amino acid sequences have been identified, the DNA sequences capable of encoding them are synthesized. Because the genetic code is degenerate, more than one codon may be used to encode a particular amino acid, and therefore, the amino acid sequence can be encoded by any of a set of similar DNA oligonucleotides. Only one member of the set will be identical to the ICE sequence but will be capable of hybridizing to ICE DNA even in the presence of DNA oligonucleotides with mismatches. The mismatched DNA oligonucleotides may still sufficiently hybridize to the ICE DNA to permit identification and isolation of ICE encoding DNA.

Using the preferred method, cDNA clones encoding ICE are isolated in a two-stage approach employing polymerase chain reaction (PCR) based technology and cDNA library screening. In the first stage, NH₂-terminal and internal amino acid sequence information from the purified 20 kDa and 10 kDa ICE subunits is used to design degenerate oligonucleotide primers for the amplification of ICE-specific DNA fragments. In the second stage, these fragments are cloned to serve as probes for the isolation of full length cDNA from a commercially available lambda gt10 cDNA library (Clontech) derived from THP.1 cells (ATCC #TIB 202).

Amino acid sequence information from the purified 20 kDa and 10 kDa ICE subunits is obtained by automated amino acid sequencing using Edman chemistry of both the intact 20 kDa and 10 kDa subunits and the peptide fragments of the 20 kDa and 10 kDa subunits generated by specific proteolytic cleavage. Enzymatic fragmentation of the 20 kDa and 10 kDa ICE subunits was performed using either trypsin (Promega) or endoproteinase Asp.N (Boehringer Mannheim). Prior to digestion, the individual ICE subunits were reductively alkylated at cysteine residues by 4-vinylpyridine. 40 to 50 pmoles of the 20 kDa or 10 kDa ICE subunits is dissolved in 50 ul of a solution containing 6M guanidine-HCl, 1 mM EDTA, 0.25 M Tris-HCl, pH 8.5. 2.5 ul of 71 mM β-mercaptoethanol is added and the solution is then incubated for two hours under argon in the dark at room temperature. 2 ul of a 370 mM 4-vinylpyridine solution is then added and the resulting solution is incubated for an additional two hours under argon in the dark at room temperature. The alkylated ICE subunits were subsequently desalted by C-4 reverse phase chromotography (Applied Biosystem butyl, 7 micron column) employing a linear gradient of 15% to 90% B in 26 minutes (buffer A= 0.06% aqueous TFA; buffer B=89.99% acetonitrile, 10 % H₂O, 0.055% TFA). Tryptic digests were conducted in 50 ul of 50 mM ammonium bicarbonate buffer, pH 9.0 for a period of 16 hours at 37° C utilizing a trypsin to ICE subunit ratio (w/w) of 1:100 (10 ng trypsin to 1000 ng (50 pmol) of the 20 kDa subunit; 5 ng trypsin to 500 ng (50 pmol) of the 10 kDa subunit). Endoproteinase Asp.N digests were conducted in 50 ul of 50 mM ammonium bicarbonate buffer pH 9.0 for a period of 36 hours at 25° C utilizing an endoproteinase Asp.N to ICE subunit ratio (w/w) of 1:20 (40 ng Asp.N protease to 800 ng (40 pmol) of the 20 kDa subunit, 20 ng Asp.N protease to 400 ng (40 pmol) of the 10 kDa subunit).

Following incubation for the prescribed periods, digestion is terminated by the addition of 5 ul of 10% TFA and resulting peptide fragments are fractionated by C-18 reverse-phase HPLC on an Applied Biosystems 130A Separation System. The reverse phase chromotography conditions include the use of a Vydac C-18 column (2.1 x 100 mm) operated at a flow rate of 200 ul/min and a temperature of 52 C. Buffer A is 0.06% aqueous TFA while buffer B is 89.99% acetonitrile/10% H₂0/0.055% TFA. Peptides are eluted with a gradient of 2% B to 75% B in 60 minutes followed by 75% B to 95% B in 10 minutes, and detected at 214 nm (0.25 AUFS).

Automated amino acid sequencing using Edman chemistry was performed on an Applied Biosystem 477A instrument coupled to a 120A analyzer for on-line PTH-amino acid identification. For sequencing of the intact 20 kDa and 10 kDa subunits, 15 pmols of protein was routinely applied to the sequenator while for enzymatically derived peptide fragments 5-20 pmols was used.

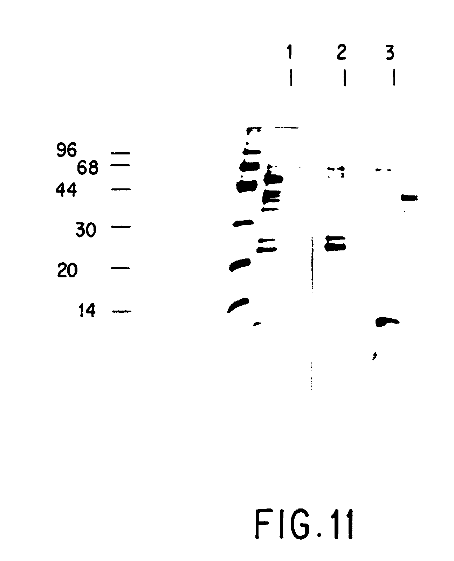

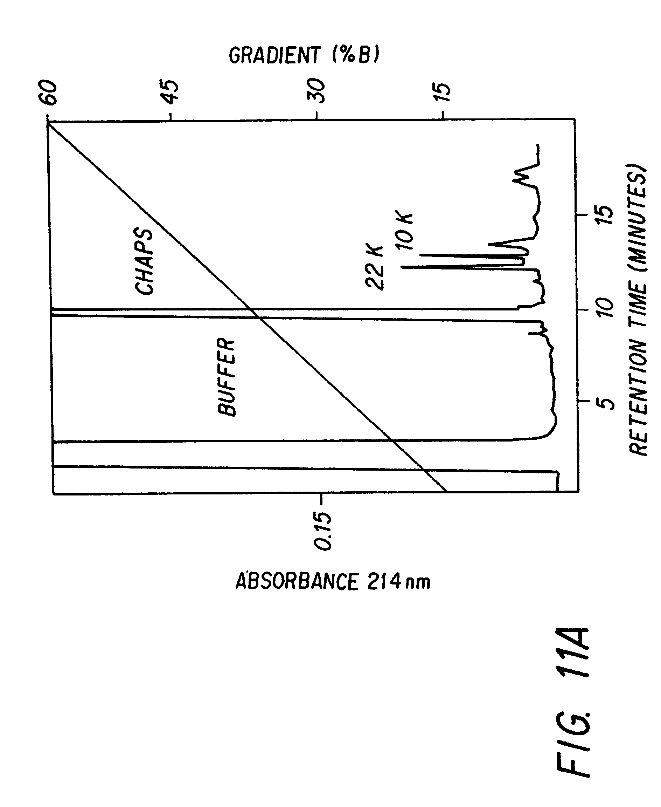

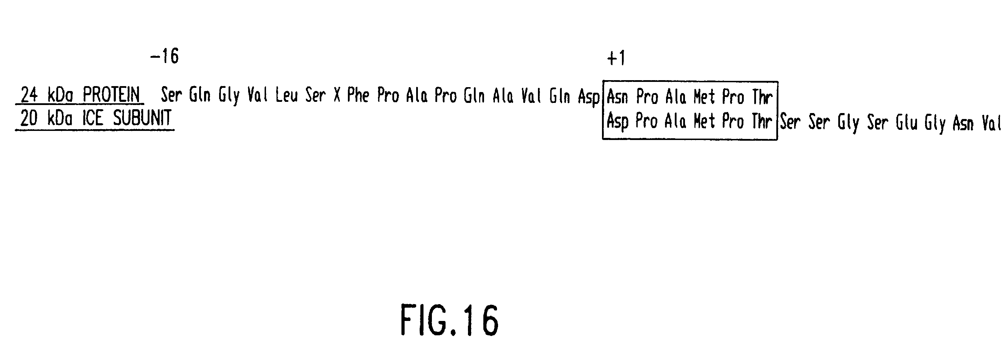

Table 3 details the amino acid sequence obtained from the 20 kDa and 10 kDa ICE subunits, respectively, as determined by sequence analysis of the intact ICE subunits. When the intact 10 kDa protein was subjected to sequencing, a single sequence was always observed consistent with a single, free amino terminus of a homogeneous protein. For the most part, a single, free amino terminus was also observed when the intact 20 kDa protein was sequenced. However, on occasion, a secondary sequence was detected in highly purified preparations containing the intact 20 kDa protein. The observation of the secondary sequence was coincident with the co-purification of a 24 kDa protein with the 20 kDa ICE subunit (Figure 11). The protein from which the secondary sequence was obtained was less prevalent in the mixture than the 20 kDa protein from which the primary sequence was obtained, allowing for the assignment of the secondary sequence to the 22 kDa protein (Table 3). A comparison of the amino terminal sequences of the 22 kDa protein and the 20 kDa ICE subunit reveals that the 22 kDa protein contains amino acid sequence corresponding directly to the NH₂ terminus of the 20 kDa ICE subunit with an NH₂ terminal extention of 16 amino acids (Figure 16). The only difference in the overlapping sequence of the 24 kDa and 20 kDa proteins is the substitution of an Asn for Asp at position +1 in the 24 kDa protein.

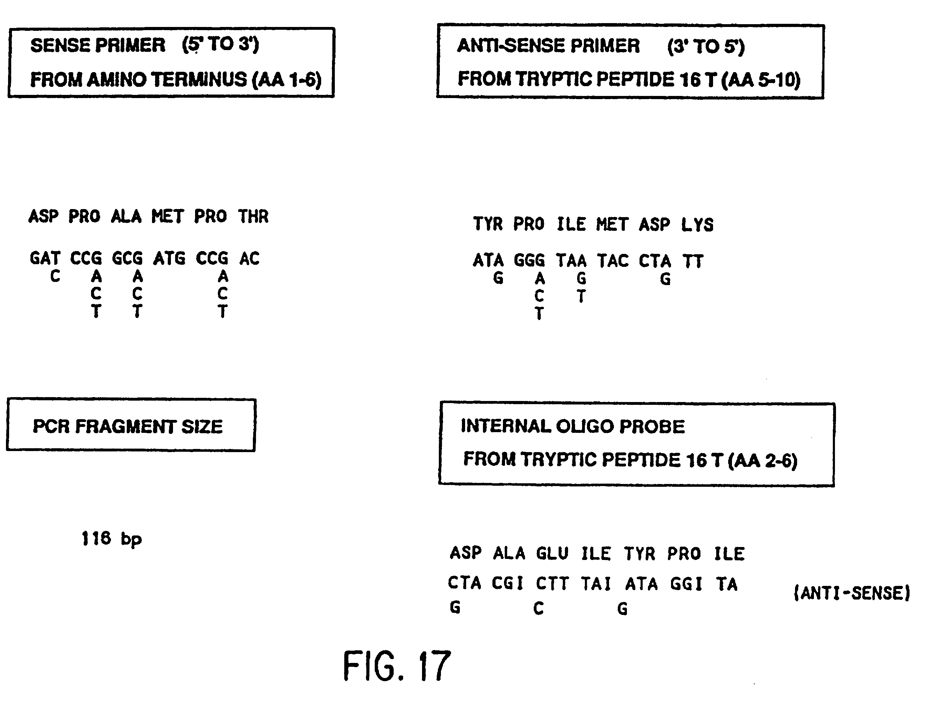

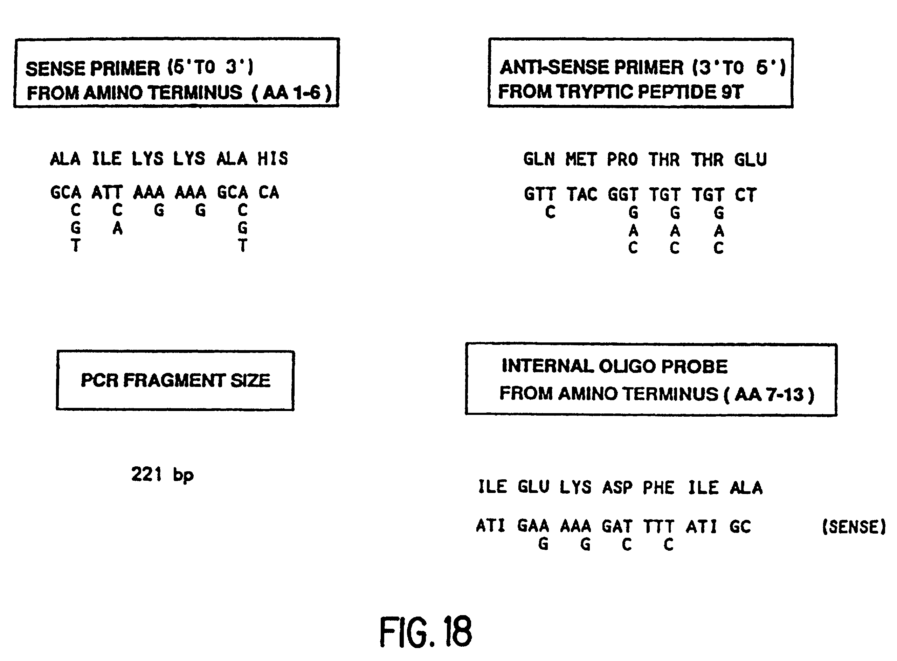

Degenerate oligonucleotides were designed based on the amino acid sequence from the amino terminus and internal regions of both the 20 kDa and 10 kDa proteins (Example 23). For the 20 kDa protein, the amino terminal primer, GAYCCNGCNATGCCNAC (SEQ.ID.NO.:3), was 128 fold degenerate while the internal primer, 3'-ATRGGNTADTACCTRTT-5' (SEQ.ID.NO.:4), was 48 fold degenerate (Figure 17). For the 10 kDa protein, the amino terminal primer, GCNATHAARAARGCNCA (SEQ.ID.NO.:5), was 192 fold degenerate while the internal primer, GTYTACGGNTGNTGNCT (SEQ.ID.NO.:6), was 128 fold degenerate (Figure 18).

Single-stranded THP.1 cDNA was synthesized from THP.1 cellular poly A+ mRNA and used as a PCR template. PCR was conducted essentially as described by a modification of the MOPAC procedure (Lee, et al., Science 239, 1288 (1988)). For each PCR, 10 pmol of each primer was added to that quantity of cDNA synthesized for 0.4µg of poly A+ mRNA in a reaction buffer consisting of 50 mM KCl, 10 mM TRIS-HCl (pH 8.3), 1.5 mM MgCl₂, 0.01% w/v gelatin, and 20 uM of each dNTP in a final volume of 10µl. The PCR program consisted of one cycle of denaturation for 100°C or 10 minutes, the addition of 2 units of Taq polymerase, followed by 30 cycles of the following steps: 95°C for 30 seconds, 48°C for 30 seconds, and 70°C for 1 minutes. For the 20 kDa protein, a PCR product of 116 bp was synthesized and its identity was verified by hybridization with an internal inosine-substituted oligonucleotide [ATIGGRTAIATYTCIGCR](SEQ.ID.NO.:7), while for the 10 kDa protein, a PCR product of 221 bp was synthesized and verified with a similar type of probe [ATIGARAARGAYTTYATIGC](SEQ.ID.NO.:8). Both PCR products were subcloned into Bluescript vectors (Stratagene), and sequenced by the chain termination method (Sanger, et al., PNAS 74, 5463 (1977)).

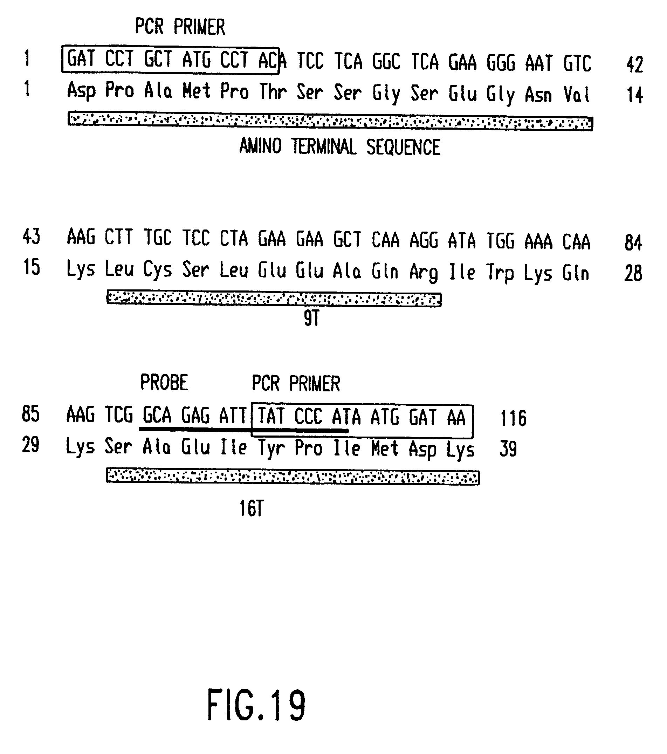

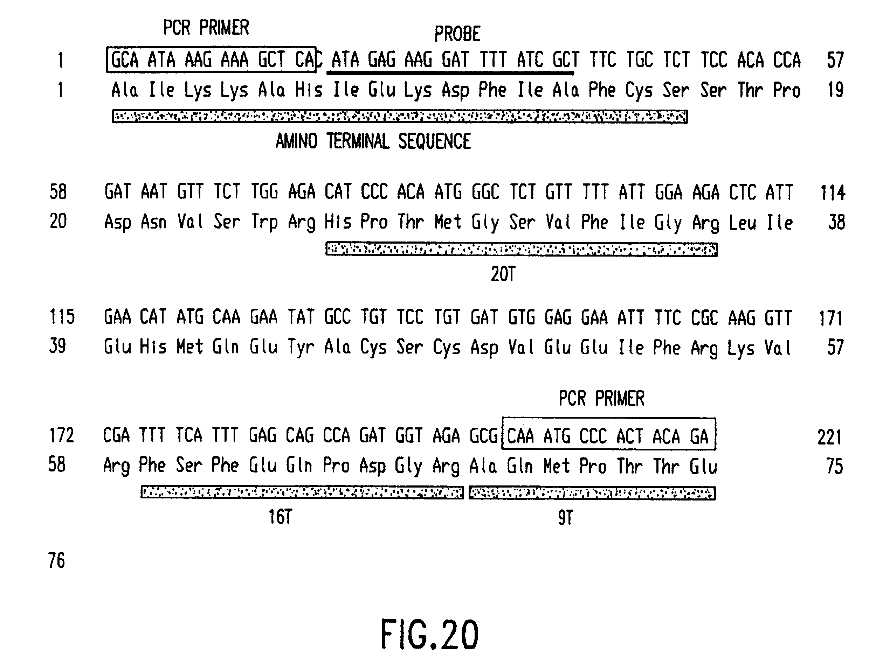

The deduced nucleic acid sequence of the PCR products derived from the 20 kDa and 10 kDa ICE subunits (Figure 19 and 20 respectively, Example 23) reveals complete identity with several of the previously sequenced tryptic peptides (Figures 19 and 20) not utilized in the design of primers or probes for PCR amplification.

These PCR derived products from Example 23 were used as hybridization probes for screening a lambda gt10 cDNA library derived from THP.1 cells (Clontech). Plating and plaque lifts of the library were performed by standard methods (T. Maniatis, E.F. Fritsch, J. Sambrook, Molecular Cloning: A Laboratory Manual (Cold Spring Harbor Laboratory, Cold Spring Harbor, New York, 1982). The probes were random-primed labelled with ³²P-dCTP to high specific activity and a separate screening of the library (600,000 plaques per screen) was conducted with each probe. The probes were added to hybridization buffer (50% formamide, 5X Denhardts, 6X SSC (1X SSC = 0.15 M), 0.5% SDS, 100 µg/ml salmon sperm DNA) at 1 x 10⁶ cpm/ml.

Eleven positively hybridizing phage were detected using the 10 kDa specific probe while seven positively hybridizing phage were observed using the 20 kDa probe.

Several cDNA clones ranging in size from 1.0 kb to 1.6 kb in length and containing a single open reading frame of 404 amino acids were subcloned into pGEM vectors (Promega) and bi-directionally sequenced in their entirety by the chain termination method (Sanger et al., P.N.A.S. USA, 74, pp 5463, 1977).

The sequence for the full-length cDNA encoding ICE is shown in Table 5, and was designated clone OCP9. The deduced amino acid sequence of ICE from the cloned cDNA is shown in Table 6. Inspection of the deduced amino acid sequence reveals the presence of a single, large open reading frame of 404 amino acids. By comparison with amino acid sequences derived from the purified native ICE 20 kDa and 10 kDa subunits, an additional in frame coding sequence of 119 amino acids is located amino terminal to the 20 kDa subunit. In addition to the sequence of the 20 kDa and 10 kDa ICE subunits, the entire NH₂-terminus of the 24 kDa protein is encoded in this open reading frame.

A precise correlation in molecular mass exists for both the 20 kDa and 10 kDa ICE subunits when a comparison is made between the molecular mass determined by mass spectrometry and that computed by the deduced amino acid sequence. For the 10 kDa subunit, the molecular mass determined from the deduced sequence is 10,242 (amino acid 317 to 404) which perfectly agrees with the mass spectrometry determination. For the 20 kDa subunit, a difference of no more than 20 amu is observed between the deduced sequence (19,844, amino acid 120 to 297) and the mass spectrometry determination.

Complete sequence identity is observed between the amino acid sequence determined by direct Edman sequencing of the individual ICE subunits and the deduced amino acid sequence from the cDNA, except for one amino acid found at position 120 of the full length nascent ICE protein. According to the cDNA sequence, Asn is encoded at position 120 of the full-length protein. This amino acid position corresponds to the NH₂-terminus of the 20 kDa subunit. The amino acid sequence derived from the purified native 20 kDa subunit determined the NH₂-terminal amino acid to be Asp. This difference between the deduced amino acid sequence and the known amino acid sequence, the only difference found, may be attributable to deamidation of the NH₂-terminal Asn of the 20 kDa subunit to form Asp.

Whether Asn or Asp is found at the NH₂-terminus of the 20 kDa subunit, or whether Asn or Asp is found within the ICE polypeptide at position 120 of the full length protein, or in a polypeptide fragment, is not expected to affect ICE activity significantly, if at all.

Further inspection of the ICE deduced amino acid sequence reveals several ICE-like cleavage sites between amino acid positions 103-104, 119-120, 297-298, and 316-317. Evidence for such a mechanism is given by the finding that when radiolabeled, in vitro translated p45 is incubated in the presence of affinity purified ICE, labeled cleavage products are generated which are congruent in size with the known purified-ICE forms-or predicted intermediates which could-result from single cleavages of p45. Processing was not due to a contaminating protease since a tetrapeptide aldehyde ICE inhibitor specifically blocked the cleavage of p45. Mutation of the Asp to an Ala at each of these sites prevents formation of the cleavage product predicted from processing at that particular site. Each of these potential cleavage sites could serve to generate the 20 kDa and 10 kDa subunits from the full length ICE protein, and may be involved in an autocatalytic mechanism for the activation and processing of ICE.

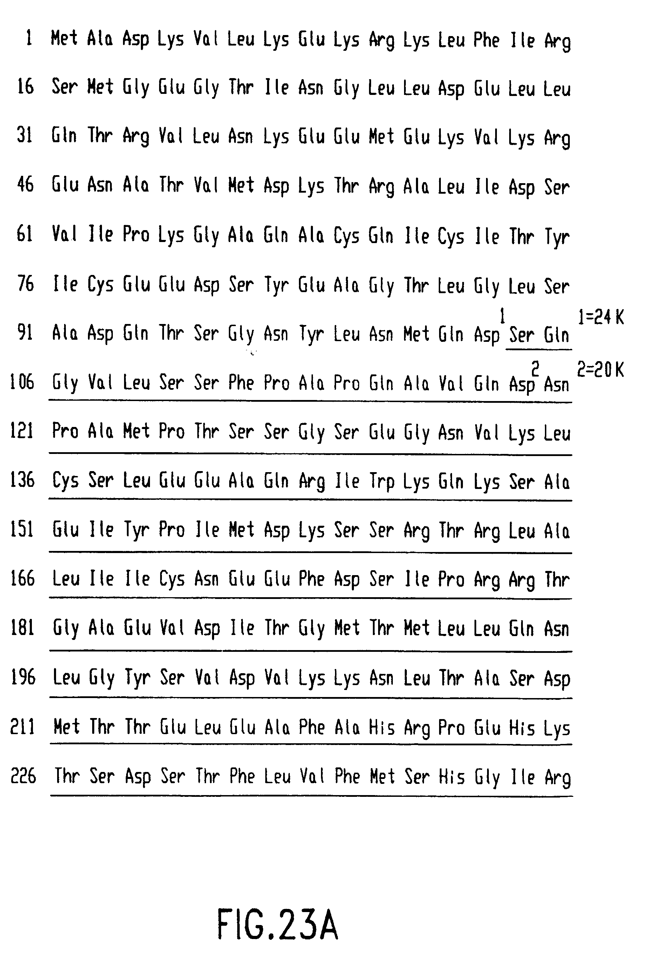

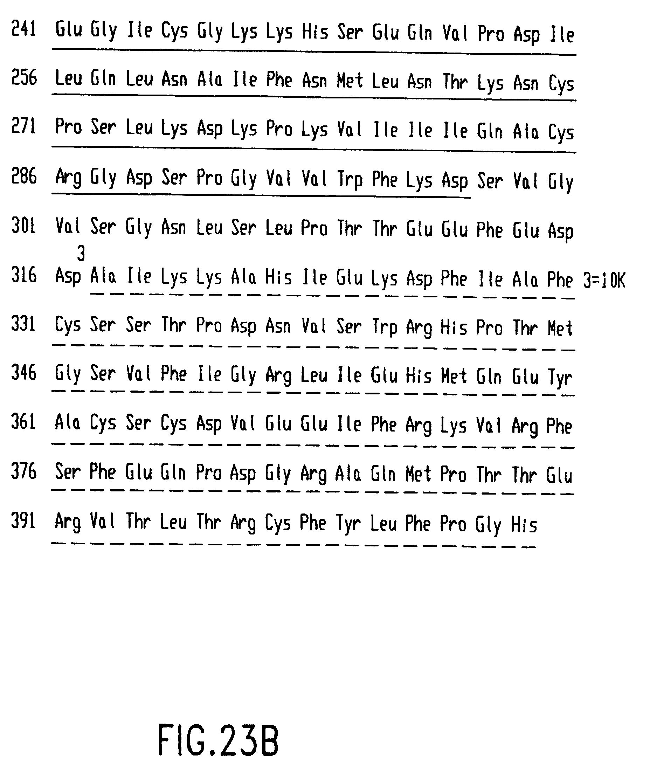

Purified biologically active ICE may have several different physical forms. ICE may exist as a full-length nascent or unprocessed polypeptide, or as partially processed polypeptides or combinations of processed polypeptides, as evidenced by the synthesis of a 45 kDa polypeptide by programming of a cell-free in vitro translation system with in vitro transcribed mRNA corresponding to the full length cDNA (Figure 23). The observed ICE translation product is coincident with the predicted size of the 404 amino acid-encoding ORF of the ICE cDNA. The full-length nascent ICE polypeptide is postranslationally modified by specific proteolytic cleavage events which result in the formation of fragments of the full length nascent polypeptide. A fragment, or physical association of fragments may have the full biological activity associated with ICE (cleavage of precursor IL-1β into mature IL-1β) however, the degree of ICE activity may vary between individual ICE fragments and physically associated ICE polypeptide fragments.

ICE in substantially pure form derived from natural sources according to the purification processes described herein, is found to be an association of two ICE polypeptide fragments encoded by a single mRNA. The two ICE polypeptide fragments are found to have an apparent molecular weight of 20 kDa and 10 kDa.

The cloned ICE cDNA obtained through the methods described above may be recombinantly expressed by molecular cloning into an expression vector containing a suitable promoter and other appropriate transcription regulatory elements, and transferred into prokaryotic or eukaryotic host cells to produce recombinant ICE. Techniques for such manipulations are fully described in Maniatis, T, et al., supra, and are well known in the art.

Expression vectors are defined herein as DNA sequences that are required for the transcription of cloned copies of genes and the translation of their mRNAs in an appropriate host. Such vectors can be used to express eukaryotic genes in a variety of hosts such as bacteria, bluegreen algae, plant cells, insect cells and animal cells.

Specifically designed vectors allow the shuttling of DNA between hosts such as bacteria-yeast or bacteria-animal cells. An appropriately constructed expression vector should contain: an origin of replication for autonomous replication in host cells, selectable markers, a limited number of useful restriction enzyme sites, a potential for high copy number, and active promoters. A promoter is defined as a DNA sequence that directs RNA polymerase to bind to DNA and initiate RNA synthesis. A strong promoter is one which causes mRNAs to be initiated at high frequency. Expression vectors may include, but are not limited to, cloning vectors, modified cloning vectors, specifically designed plasmids or viruses.

A variety of mammalian expression vectors may be used to express recombinant ICE in mammalian cells. Commercially available mammalian expression vectors which may be suitable for recombinant ICE expression, include but are not limited to, pMClneo (Stratagene), pXT1 (Stratagene), pSG5 (Stratagene), EBO-pSV2-neo (ATCC 37593) pBPV-1(8-2) (ATCC 37110), pdBPV-MMTneo(342-12) (ATCC 37224), pRSVgpt (ATCC 37199), pRSVneo (ATCC 37198), pSV2-dhfr (ATCC 37146), pUCTag (ATCC 37460), and λZD35 (ATCC 37565).

DNA encoding ICE may also be cloned into an expression vector for expression in a recombinant host cell. Recombinant host cells may be prokaryotic or eukaryotic, including but not limited to bacteria, yeast, mammalian cells including but not limited to cell lines of human, bovine, porcine, monkey and rodent origin, and insect cells including but not limited to drosophila derived cell lines. Cell lines derived from mammalian species which may be suitable and which are commercially available, include but are not limited to, CV-1 (ATCC CCL 70), COS-1 (ATCC CRL 1650), COS-7 (ATCC CRL 1651), CHO-K1 (ATCC CCL 61), 3T3 (ATCC CCL 92), NIH/3T3 (ATCC CRL 1658), HeLa (ATCC CCL 2), C127I (ATCC CRL 1616), BS-C-1 (ATCC CCL 26) and MRC-5 (ATCC CCL 171).

The expression vector may be introduced into host cells via any one of a number of techinques including but not limited to transformation, transfection, protoplast fusion, and electroporation. The expression vector-containing cells are clonally propagated and individually analyzed to determine whether they produce ICE protein. Identification of ICE expressing host cell clones may be done by several means, including but not limited to immunological reactivity with anti-ICE antibodies, and the presence of host cell-associated ICE activity.

Expression of ICE DNA may also be performed using in vitro produced synthetic mRNA. Synthetic mRNA can be efficiently translated in various cell-free systems, including but not limited to wheat germ extracts and reticulocyte extracts, as well as efficiently translated in cell based systems, including but not limited to microinjection into frog oocytes, with microinjection into frog oocytes being preferred.

To determine the ICE cDNA sequence(s) that yields optimal levels of enzymatic activity and/or ICE protein, ICE cDNA molecules including but not limited to the following can be constructed: the full-length open reading frame of the ICE cDNA (45 kDa = base 4- base 1215) and several constructs containing portions of the cDNA encoding both the 20 kDa and 10 kDa subunits. All constructs can be designed to contain none, all or portions of the 3' untranslated region of ICE cDNA (base 1216-1488). ICE activity and levels of protein expression can be determined following the introduction, both singly and in combination, of these constructs into appropriate host cells. Following determination of the ICE cDNA cassette yielding optimal expression in transient assays, this ICE cDNA construct is transferred to a variety of expression vectors, including but not limited to mammalian cells, baculovirus-infected insect cells,
E. Coli, and the yeast S. cerevisiae.

Mammalian cell transfectants and microinjected oocytes are assayed for both the levels of ICE enzymatic activity and levels of ICE protein by the following methods. The first method for assessing ICE enzymatic activity involves the direct introduction of the native substrate for ICE, the 31.5 K IL-1β precursor, simultaneously with ICE. To assess the substate specificity of expressed ICE, IL-1β precursor substrates with altered amino acids in the ICE cleavage sites will be tested. In the case of mammalian cells, this involves the co-transfection of two plasmids, one containing the ICE cDNA and the other containing the preIL-1β cDNA. In the case of oocytes, this involves the co-injection of synthetic RNAs for both ICE and the IL-1β precursor. Following an appropriate period of time to allow for expression, cellular protein is metabolically labelled with ³⁵S-methionine for 24 hours, after which cell lysates and cell culture supernatants is harvested and subjected to immunprecipitation with polyclonal antibodies directed against the IL-1β protein. Cleavage of the wild-type precursor to the 28 K and 17 K forms, and cleavage of the precursor containing an altered downstream processing site (Asp₁₁₆ to Ala₁₁₆) to the 28 K form is assessed by an SDS-PAGE gel based assay.

The second method for detecting ICE activity involves the direct measurement of ICE activity in cellular lysates prepared from mammalian cells transfected with ICE cDNA or oocytes injected with ICE mRNA. This assay can be performed using IL-1β precursor protein or synthetic peptides spanning the IL-1β cleavage sites. Cleavage products of the precursor is analyzed by standard gel based assay and cleavage products of the peptides are analyzed by HPLC.

Levels of ICE protein in host cells is quantitated by immunoaffinity and/or ligand affinity techniques. ICE-specific affinity beads or ICE-specific antibodies are used to isolate ³⁵S-methionine labelled or unlabelled ICE protein. Labelled ICE protein is analyzed by SDS-PAGE. Unlabelled ICE protein is detected by Western blotting, ELISA or RIA assays employing ICE specific antibodies.

Following expression of ICE in a recombinant host cell, ICE protein may be recovered to provide ICE in active form, capable of cleaving precusor IL-1β into mature IL-1β. Several ICE purification procedures are available and suitable for use. As described above for purification of ICE from natural sources, recombinant ICE may be purified from cell lysates and extracts, or from conditioned culture medium, by various combinations of, or individual application of salt fractionation, ion exchange chromatography, size exclusion chromatography, hydroxylapatite adsorption chromatography and hydrophobic interaction chromatography.

In addition, recombinant ICE can be separated from other cellular proteins by use of an immuno-affinity column made with monoclonal or polyclonal antibodies specific for full length nascent ICE, polypeptide fragments of ICE or ICE 20 kDa and 10 kDa subunits.

Monospecific antibodies to ICE are purified from mammalian antisera containing antibodies reactive against ICE or are prepared as monoclonal antibodies reactive with ICE using the technique of Kohler and Milstein, Nature 256: 495-497 (1975). Monospecific antibody as used herein is defined as a single antibody species or multiple antibody species with homogenous binding characteristics for ICE. Homogenous binding as used herein refers to the ability of the antibody species to bind to a specific antigen or epitope, such as those associated with the ICE, as described above. Enzyme specific antibodies are raised by immunizing animals such as mice, rats, guinea pigs, rabbits, goats, horses and the like, with rabbits being preferred, with an appropriate concentration of ICE either with or without an immune adjuvant.

Preimmune serum is collected prior to the first immunization. Each animal receives between about 0.1 µg and about 1000 µg of ICE associated with an acceptable immune adjuvant. Such acceptable adjuvants include, but are not limited to, Freund's complete, Freund's incomplete, alum-precipitate, water in oil emulsion containing Corynebacterium parvum and tRNA. The initial immunization consisted of the enzyme in, preferably, Freund's complete adjuvant at multiple sites either subcutaneously (SC), intraperitoneally (IP) or both. Each animal is bled at regular intervals, preferably weekly, to determine antibody titer. The animals may or may not receive booster injections following the initial immunizaiton. Those animals receiving booster injections are generally given an equal amount of the enzyme in Freund's incomplete adjuvant by the same route. Booster injections are given at about three week intervals until maximal titers are obtained. At about 7 days after each booster immunization or about weekly after a single immunization, the animals are bled, the serum collected, and aliquots are stored at about -20°C.

Monoclonal antibodies (mAb) reactive with ICE are prepared by immunizing inbred mice, preferably Balb/c, with ICE. The mice are immunized by the IP or SC route with about 0.1 µg to about 10 µg, preferably about 1 µg, of ICE in about 0.5 ml buffer or saline incorporated in an equal volume of an acceptable adjuvant, as discussed above. Freund's complete adjuvant is preferred. The mice receive an initial immunization on day 0 and are rested for about 3 to about 30 weeks. Immunized mice are given one or more booster immunizations of about 0.1 to about 10 µg of ICE in a buffer solution such as phosphate buffered saline by the intravenous (IV) route. Lymphocytes, from antibody positive mice, preferably splenic lymphocytes, are obtained by removing spleens from immunized mice by standard procedures known in the art. Hybridoma cells are produced by mixing the splenic lymphocytes with an appropriate fusion partner, preferably myeloma cells, under conditions which will allow the formation of stable hybridomas. Fusion partners may include, but are not limited to: mouse myelomas P3/NS1/Ag 4-1; MPC-11; S-194 and Sp 2/0, with Sp 2/0 being preferred. The antibody producing cells and myeloma cells are fused in polyethylene glycol, about 1000 mol. wt., at concentrations from about 30% to about 50%. Fused hybridoma cells are selected by growth in hypoxanthine, thymidine and aminopterin supplemented Dulbecco's Modified Eagles Medium (DMEM) by procedures known in the art. Supernatant fluids are collected form growth positive wells on about days 14, 18, and 21 and are screened for antibody produciton by an immunoassay such as solid phase immunoradioassay (SPIRA) using ICE as the antigen. The culture fluids are also tested in the Ouchterlony precipitation assay to determine the isotype of the mAb. Hybridoma cells from antibody positive wells are cloned by a technique such as the soft agar technique of MacPherson, Soft Agar Techniques, in Tissue Culture Methods and Applications, Kruse and Paterson, Eds., Academic Press, 1973.

Monoclonal antibodies are produced in vivo by injection of pristane primed Balb/c mice, approximately 0.5 ml per mouse, with about 2 x 10⁶ to about 6 x 10⁶ hybridoma cells about 4 days after priming. Ascites fluid is collected at approximately 8-12 days after cell transfer and the monoclonal antibodies are purified by techniques known in the art.

In vitro production of anti-pre-IL-1β mAb is carried out by growing the hydridoma in DMEM containing about 2% fetal calf serum to obtain sufficient quantities of the specific mAb. The mAb are purified by techniques known in the art.

Antibody titers of ascites or hybridoma culture fluids are determined by various serological or immunological assays which include, but are not limited to, precipitation, passive agglutination, enzyme-linked immunosorbent antibody (ELISA) technique and radioimmunoassay (RIA) techniques. Similar assays are used to detect the presence of ICE in body fluids or tissue and cell extracts.

It is readily apparent to those skilled in the art that the above described methods for producing monospecific antibodies may be utilized to produce antibodies specific for ICE polypeptide fragments, or full-length nascent ICE polypeptide, or the individual 20 kDa and 10 kDa subunits. Specifically, it is readily apparent to those skilled in the art that monospecific antibodies may be generated which are specific for only the 20 kDa ICE subunit or only the 10 kDa ICE subunit, or only the full-length nascent ICE molecule.

ICE antibody affinity columns are made by adding the antibodies to Affigel-10 (Biorad), a gel support which is pre-activated with N-hydroxysuccinimide esters such that the antibodies form covalent linkages with the agarose gel bead support. The antibodies are then coupled to the gel via amide bonds with the spacer arm. The remaining activated esters are then quenched with 1M ethanolamine HCl (pH 8). The column is washed with water followed by 0.23 M glycine HCl (pH 2.6) to remove any non-conjugated antibody or extraneous protein. The column is then equilibrated in phosphate buffered saline (pH 7.3) and the cell culture supernatants or cell extracts containing ICE or ICE subunits are slowly passed through the column. The column is then washed with phosphate buffered saline until the optical density (A₂₈₀) falls to background, then the protein is eluted with 0.23 M glycine-HCl (pH 2.6). The purified ICE protein is then dialyzed against phosphate buffered saline.

The full length ICE-encoding cDNA-containing in plasmid pGEM-Zf(-) was designated pOCP9. A sample of pOCP9 in E. coli strain HB101, was deposited under the terms of the Budapest Treaty, on or before August 15, 1991, in the permanent culture collection of the American Type Culture Collection, at 12301 Parklawn Drive, Rockville, MD., 20852, and has been assigned the accession number ATCC 68655.

The following examples illustrate the present invention without, however, limiting the same thereto.

### EXAMPLE 1

### Preparation of Cell-Free Extracts

Human peripheral blood monocytes were obtained from healthy donors by leukophoresis and purified by sedimentation through Lymphocyte Separation Media (Organon Teknika) followed by elutration on a Beckman counterflow centrifuge as described by Wicker et al., Cell. Immunol. 106: 318-329 (1987). Monocytes were identified by labeling with anti-MACl antibody followed by FACS analysis. Human THP-1 cells (American Type Culture Collection) were grown in suspension at 37o C in Dulbecco's Modified Minimal Essential Media (Hazelton) with 10% fetal calf serum (Hyclone Laboratories, defined sera with no detectable endotoxin) to a concentration of 1 x 10⁶ cells/ml. THP-1 cells were also grown in either 75 or 200 Liter fermenters in Iscoves media with a supplement of 10% horse serum containing low endotoxin under controlled conditions of pH 7.2 , 20 to 50% dissolved O2, 2 to 5% dissolved CO₂, 37° C temperature and 90 rpm agitation speed using a marine propeller blade. Cells were harvested using a Millipore Prostak cross-flow membrane filtration system run with a pressure of less than 2 psi across 50 sq ft of 0.6 uM membrane. Harvested cells were centrifuged to a pellet, washed with Dulbecco's PBS and lysed with hypotonic buffer containing 25 mM HEPES, pH 7.5, 5 mM MgC12, 1 mM EGTA, and protease inhibitors including 1 mM PMSF, 10 µg/ml leupeptin, 10 µg/ml pepstatin A. After preparation of the S-3, DTT and CHAPS is added to final concentrations of 2 mm and 0.1% respectively. Media used in suspension flasks or fermenters also contained 0.1-0.3% F68 pluronic to reduce shear force on the cells. Cells were typically grown for no more than 3-4 months following initial reculturing from preserved cell samples.

Cell-free extracts were prepared from human peripheral blood monocytes and THP-1 cells by nitrogen cavitation or by homogenization in hypotonic lysis buffer. Cells were collected by centrifugation at 1,000 x g, (for example 15 L of THP-1 cells) washed 3 times with Dulbecco's phosphate buffered saline containing no magnesium or calcium chloride and were pelleted at 1,000 x g for 10 minutes. The resulting cell pellets were resuspended in 3 volumes of hypotonic buffer (20 mM KCl, 25 mM HEPES, pH 7.4, 1.5 mM MgC12, 0.1 mM EDTA, 1 mM DTT), and placed in an ice bath for 20 minutes. The cells were lysed in a Dounce homogenizer and homogenized with 20 strokes. For gentler lysis, the cell pellets were resuspended in hypotonic buffer, placed in a stainless steel nitrogen pressure cell and pressurized to 400 psi with nitrogen gas and held for 30 minutes at 4o C with agitation. The cells were lysed by simultaneously releasing the pressure and evacuating the cells from the container. At this point the cell membranes were effectively broken by rapid decompression and shear flow. Cellular homogenates were clarified by centrifugation at either 400 or 1,000 x g for 20 min, the supernatant fluid, designated S-1, being saved and stored at -80° C. Freshly prepared S-1 supernatant fluid was further centrifuged at 30,000 x g for 10 min and designated S-2. The S-2 supernatant fluid was further centrifuged at 300,000 x g for 1.5 hr and the supernatant fluid, designated S-3, collected.

Cell-free extracts of THP-1 cell were also prepared with the following technique. Cells were washed 3 times in PBS and suspended for 20 min at 0° C at 10⁸ cells/ml in a hypotonic buffer containing 25 mM HEPES, pH 7.5, 5 mM MgC12, and 1 mM EGTA. Protease inhibitors were added (1 mM PMSF and 10 mg/ml of pepstatin and leupeptin), and the cells were broken in 100 or 300 ml tight fitting Dounce homogenizers using 25 or 15 strokes respectively to yield 90-95% breakage. The broken cells were centrifuged at 3000 rpm for 10 min at 5° C in a Beckman GPR centrifuge to remove nuclei and unbroken cells. The postnuclear supernatant was centrifuged for 20 min at 16,000 rpm in a Sorval centrifuge with an SS34 rotor followed by a second centrifugation for 60 min at 50,000 rpm in a Beckman centrifuge (50.2Ti rotor) or 45,000 rpm (45Ti rotor). After addition of 2 mM DTT and 0.1 % CHAPS, the resultant supernatant was stored at -80° C.

### EXAMPLE 2

### Salt Fractionation

The S-3 supernatant fluid, from Example 1, was sequentially precipitated by the addition of ammonium sulfate in order to both concentrate and partially purify pre-IL-1β converting enzyme activity. Granular ammonium sulfate was added to 50 ml of S-3 supernatant fluid to reach 45% saturation at 4° C. The fluid was allowed to equilibrate on ice with stirring for 15 minutes. The turbid precipitate was clarified by centrifugation at 10,000 x g with the resulting pellet being discarded. The supernatant fluid was then brought to 80% saturation of ammonium sulfate using the above protocol. The precipitate is then pelleted, resuspended in Buffer A (20 mM KCl, 25 mM HEPES, pH 7.4, 5.0 mM EDTA, 2 mM DTT, 1 mM PMSF, 0.1% NP-40 (Nonident detergent P-40), 10% glycerol) and dialyzed overnight against the same buffer. The dialyzed precipitate was centrifuged at 30,000 x g to remove particulate material and then stored at -80° C.

### EXAMPLE 3

### Ion Exchange Chromatography

Ten ml of the ammonium sulfate precipitated protein from Example 2, 110 mg total protein, was applied to a Bio-Rad DEAE-5-PW HPLC anion exchange column equilibrated in Buffer A. Fractions containing protein were detected by absorbance at 280 nm. The flow through fraction was retained and then loaded onto a Bio-Rad SP-5PW sulfopropyl cation exchange column equilibrated with Buffer A. A linear gradient of from 30-500 mM KCl in Buffer A was then used to elute converting enzyme activity. After elution the column fractions containing protein, as determined by absorbance at 280 nm, were dialyzed (Spectrum Laboratory products, Spectra/Por membrane, 8,000 molecular weight cutoff) against Buffer A for 16 hr. Individual fractions were assayed for converting enzyme activity using the protocol described in Example 4. ICE activity cleaves pre-IL-1β and generates a 17.5 kDa product which is the biologically active lymphokine. The converting enzyme activity, using these salt and pH conditions will not bind to DEAE anion exchange resins but will bind to sulfopropyl cation exchange resins. ICE was eluted as a discrete peak with recovery of at least 50% of the starting activity.

### EXAMPLE 4

### In Vitro Assays For Detection Of ICE Activity

Cleavage of pre-IL-1β with fractions from Examples 1-3 and 5-7 was performed by incubating 1 µl of rabbit reticulocyte extract containing radiolabeled precursor with 10-20 µl of the specific fractions. The radiolabeled precursor IL-1β was prepared in the following manner. A 1.5 kilobase (kb) cDNA clone containing the entire coding sequence of pre-IL-1β was inserted into EcoRI/Pst I-cleaved pGEM-3 plasmid DNA (Promega Biotec) and propagated in Escherichia coli according to standard methods know in the art, for example, see generally, Maniatis et al., Molecular Cloning: A Laboratory Manual ,Cold Spring Harbor Lab., Cold Spring Harbor, NY, (1982). Purified plasmid was linearized with Pst I and then transcribed by using a T7 RNA polymerase in vitro transcription system (Promega Biotec) and then the mRNA was processed according to the manufacturer's instructions. Translations were performed by programing micrococcal nuclease-treated rabbit reticulocyte extracts (Promega Biotec) with the in vitro synthesized mRNA in the presence of 25 uCi of [35S] methionine (1 Ci = 37 GBq; Amersham) according to the manufacturer's instructions. This yielded labeled pre-IL-1β which migrated as a doublet on SDS-PAGE with an apparent molecular mass of 34 and 31 kDa. Both bands can be immunoprecipitated with antisera directed to the carboxyl terminus of IL-1β. Interleukin-1β converting enzyme activity, cleavage of radiolabeled pre-IL-1β to yield 17.5 kDa mature Il-1β ,was monitored by SDS-PAGE.

Proteolytic cleavage of peptide substrates from Example 12 was carried out in the following manner. Samples of peptides were prepared in dimethyl sulfoxide at a concentration of 10 mM. The enzyme used in these studies was purified through sulfopropyl cation exhange chromatography. Reaction mixtures contained approximately 0.2 mg/ml enzyme, 0.2 mM peptide, 10% sucrose, 0.1% CHAPS (3-[(3-cholamidopropyl)dimethyl-ammonio]-1-propansulfon at), 10 mM HEPES (N-2-hydroxyethylpiperazine-N'-2-ethanesulfonic Acid), pH 7.5 in a 50 ul reaction volume. After incubation at 25 degrees for variable lengths of time, reactions were quenched with the addition of 450 ul 0.1% trifluoroacetic acid. The samples were analyzed by reverse-phase high performance liquid chromatography using a Vydac C-18 column (4.6 mm x 25 cm, 5 um particle size, 300 pore size) equilibrated with 5% acetonitrile, 0.1% trifluoroacetic acid at a flow rate of 1 ml/min. Peptides were eluted using a 10 min linear gradient of 5 to 30% acetonitrile and quantitated by monitoring the column effluent at 280 nm. Under these conditions substrate and tyrosine-containing product were separated with baseline resolution in every case. The identity of cleavage products was confirmed using peptide standards.

### EXAMPLE 5

### Size Exclusion Chromatography

ICE from Example 3 was further purified by size exclusion chromatography. The protein, 0.5 ml, was loaded onto a 7.5 x 600 mm TSK G3000SW gel exclusion column which had been equilibrated in Buffer A. The column was eluted with the same buffer and the respective fractions, monitored by absorbance at 280 nm, were assayed for converting enzyme activity. Interleukin-1β converting enzyme activity eluted from the column with an apparent molecular weight of 30,000. Similar experiments using TSK G2000 column resulted in an apparent molecular mass of 23,000. Thus the apparent molecular weight of active native ICE is between Mr 23,000 and 30,000.

The unique separation and purification processes described above have been summarized in Table 2.

**Table 2**

| Purification of ICE | | | |
|---|---|---|---|
| Purification Step | Total Protein (mg) | Activity Recovered (%)¹ | Fold Purity |
| Salt Fractionation | 110.0 | 100¹ | 1 |
| | | | |
| DEAE-5-PW | 52.0 | 100 | 2 |
| | | | |
| SP-5-PW | 1.5 | 50 | 50 |
| | | | |
| TSK G3000SW | 0.05 | 25 | 550 |

| | | | |
|---|---|---|---|
| 1¹ The yield of activity of each fraction was estimated by limit dilution of that fraction. | | | |

### EXAMPLE 6

### Hydrophobic Interaction Chromatography

The ICE from Example 3 or 5 was further purified by hydrophobic interaction chromatography. After the addition of solid ammonium sulfate to a final concentration of 1.5 M, the protein (1.5 ml) was loaded onto a silica based hydrophobic interaction column (Synchrom Synchropak propyl, 4.6 x 250 mm) which had been equilibrated in a buffer containing 20 mM HEPES, pH 7.4, 1.5 M (NH4)2SO4, 10 % glycerol, 5 mM EDTA, 1 mM PMSF and 2 mM DDT. The column was eluted with a continuous, linear, descending salt gradient form 1.5 to 0.0 M (NH4)2SO4. Column fractions were dialyzed against Buffer B and assayed for converting enzyme activity.

The unique separation and purification processes described above have been summarized in the following table.

**TABLE 2**

| Fraction | Protein mg/ml | Total Protein | Spec. Act. Units/mg | Total Units | Yield % |
|---|---|---|---|---|---|
| AS¹ | 138.0 | 20,700.0 | 76.8 | 1,589,760 | 100,0 |
| DEAE-FT | 44.2 | 13,260.0 | 113.1 | 1,499,760 | 94.0 |
| SP | 15.2 | 167.0 | 3,289.0 | 549,263 | 34.0 |
| HIC-3 | 11.3 | 33.9 | 4,424.0 | 149,937 | 9.4 |
| TSK | 0.38 | 2.85 | 26,315.0 | 74,997 | 4.7 |
| HAP | 0.006 | 0.036 | 1,666,666.0 | 60,000 | 3.7 |

| | | | | | |
|---|---|---|---|---|---|
| 1 AS = ammonium sulfate fraction; DEAE-FT = DEAE anion exchange chormatography flow through; SP = sulfopropyl cation exchange chromatography; HIC propyl hydrophobic interaction chromatography; TSK = TSK size exclusion chromatography; HAP = hydroxyapatite adsorption chromatography. | | | | | |

### EXAMPLE 7

### Hydroxylapatite Adsorption Chromatopraphy

The ICE from Example 5 or 6 was further purified by hydroxylapatite adsorption chromatography on a Bio-Rad HPHT analytical cartridge. The sample must be free of EDTA, which is generally removed by dialysis. The column was equilibrated in Buffer B (20 mM HEPES, pH 7.4, 5% glycerol and 2 mM DTT). Following application of 1.5 ml of ICE, the column was eluted with a continuous and linear gradiennt of 0-500 mM potassium phosphate in buffer B at room temperature. Fractions were collected, immediately placed on ice, and samples of each were dialyzed against Buffer B and measured for ICE activity.

### EXAMPLE 8

### Purification Process For Amino Acid Sequencing Mass Spectrophotometric Analysis Of Interleukin-1β Converting Enzyme

The supernatant fluid S-3 from Example 1 were clarified by 0.22 m hollow fiber filtration and concentrated 10-20 fold with an Amicon YM3 spiral cartridge and dialyzed overnight (8000 molecular weight cutoff dialysis membrane) against a buffer of 20 mM Tris, pH 7.8, 10% sucrose, 0.1% CHAPS, and 2 mM DTT. The dialyzed supernatant (about 3-5 g total protein, corresponding to 1000 ml of cytosolic extract) was adjusted to less than 500 micro Siemans conductivity with water and applied to a 475 ml bed volume DEAE-5PW HPLC (Biorad) column. ICE activity (Example 4) was eluted at about 40 mM NaCl in a gradient with the same buffer and increasing proportions of 0.5 M NaCl and 220 mM Tris HCl.

The ICE active fractions from the DEAE column were pooled, diluted with an equal volume of 75 mM HEPES, 10% (v/v) sucrose, 0.1% (v/v) CHAPS, 2 mM DTT buffer, adjusted to pH 7.0, and applied to a 150 ml bed volume SP-5PW HPLC (Biorad) column and eluted with a KCl gradient (0-0.5 M) in the same buffer. The ICE active fractions (about 75 mM salt) were chromatographed by SDS-PAGE (10-20%, 17-27%, 16%, or 18% gels) and silver stained to determine the bands that tracked with activity, see Example 9. These SP-HPLC fractions were also further chromatographed on a C4-narrowbore (ABI) HPLC column eluted with an acetonitrile gradient in 0.1% TFA. The various peaks (214 nm) were dried, chromatographed on SDS-PAGE, and silver stained to confirm the relative migration rates of the 20-22 and 10 kDa proteins as shown in Figure 1 (Example 8).

The NH₂-terminal amino acid sequence for the 20 kDa subunit and the 10 kDa subunit is shown in Table 3a and 3b.

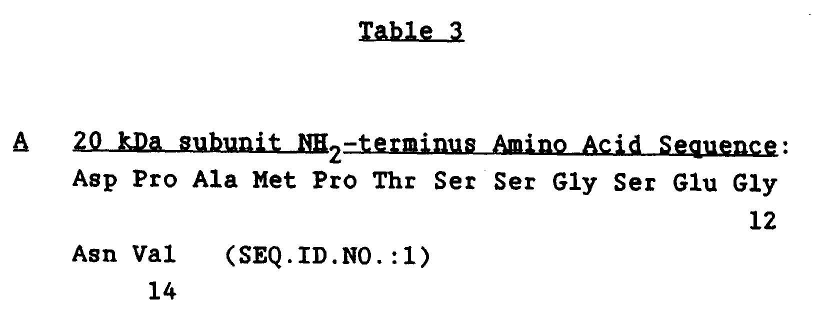

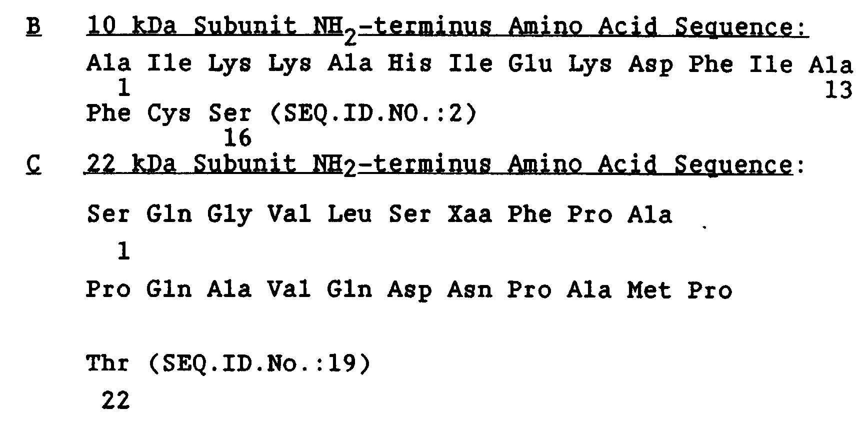

### EXAMPLE 9

### Sodium Dodecyl Sulfate Polyacrylamide Gel Electrophoresis And Staining of Interleukin-1β Converting Enzyme

Sodium dodecyl sulfate polyacrylamide gel electrophoresis of purified ICE from Examples 2, 3, 5, 6, 7,8, 10 was carried out essentially according to the method of Laemmli, Nature 227: 680-685 (1970). A 0.15 cm x 10 cm x 10 cm Bio-Rad mini-gel is used to cast a 15 % acrylamide 0.4% Bis-acrylamide SDS-PAGE resolving gel. A second discontinuous stacking layer consisting of 5% acrylamide - 0.14% bis-acrylamide is then cast on top of the resolving gel. After polymerization, the gels are loaded with no more than 50 µg of protein dissolved in Laemmlis buffer containing bromphenol blue as a tracking dye. The gels are then subjected to electrophoresis at 50 V for 1/2 to 1 hour then at 150 V until the bromphenol blue dye begins to elute from the gel. At this point electrophoresis is stopped and the gel is processed for silver staining.

After electrophoresis, separated proteins can be visualized by staining with silver using a modification of the method developed by Oakley et al. (Analytical Biochemistry, 105: 361-363. 1980) and now sold in kit form by Daiichi. Briefly, the gel is first soaked 30 minutes in 200 ml of a 50% methanol:water mixture followed by three washes of 10 minutes each in 200 ml of deionized water. After the initial washes the gel is then soaked in a solution of 40% methanol; 10% ethanol; 0.5% glutaraldehyde; 49.5% water for 15 minutes then washed thoroughly with 4 changes of 200 ml aliquots of deionized water each lasting 10 minutes. Staining and visualization of proteins is accomplished using the protocol of Oakley.

The electrophoretic patterns from the stepwise purification of ICE are shown in Figure 1. Silver stains are depicted in panel A and can be compared with pre-IL-1β cleavage activity in panel B. Separation was carried out on 200 nanogram aliquots from the above described converting enzyme purification protocol. Each purification step is represented: A.S; ammonium sulfate fraction. DE-FT; DEAE flow through; S.P.: SP cation exchange step. HIC; Propyl hydrophobic interaction chromatography. TSK; TSK-125 size exclusion chromatography. HAP; hydroxyapatite column chromatography. Note the appearance of a 22 and 10 KD protein in the final TSK and HAP steps correlates with the appearance of ICE activity. ICE activity which had been purified through an alternate purification scheme (A.S., DEAE, SP, HAP, TSK) was applied to a Propyl hydrophobic interaction column as described in Example 7. Protein was eluted with a reverse salt gradient as described in the methods Example 7. Eluted proteins were dialysed and analyzed by SDS-PAGE and silver staining (Panel A) as well as ICE activity (panel B). Note the correlation between the elution of the 22 and 10 KD proteins (arrows) and ICE activity.

### Example 11

### Protease Inhibitor Sensitivity of ICE

The ability to inhibit ICE convertase activity by various inhibitors was determined by adding the inhibitor to THP.1 S-300 and measuring IL-β cleavage as described in Example 4. Table 4a lists the inhibitors tested and whether the inhibitor had inhibitory activity.

**Table 4a**

| Protease Inhibitor Sensitivity of ICE | | |
|---|---|---|
| Inhibitor and class | Conc. | Inhibition |
| Serine | | |
| PMSF | 1.0 mM | - |
| DFP | 10 mM | - |
| a₁PI | 50 µg/ml | - |
| Leupeptin | 100 µM | - |
| | | |

| Elastase | | |
|---|---|---|
| L-659,166 | 1 mM | - |
| | | |

| Serine/thiol Chloromethyl ketone | | |
|---|---|---|
| TPCK | 300 µM | + |
| TLCK | 300 µM | + |
| PheCK | 1 mM | + |
| LeuCK | 1 mM | + |
| PheALaCK | 1 mM | + |

| Thiol proteases Diazomethyl ketones | | |
|---|---|---|
| A-PhePheDK | 1mM | - |
| PheALaDK | 1mM | - |
| E-64 | 1mM | - |
| Cystamine | 1mM | + |
| N-ethyl maleimide | 1mM | + |
| N-phenyl maleimide | 1mM | + |
| Methyl methanethiosulfonate | 1mM | + |
| | | |
| Iodoacetamide | 0.2mM | + |
| Iodoacetic acid | 0.2mM | + |
| | | |

| Metalloprotease | | |
|---|---|---|
| EDTA | 50 mM | - |
| EGTA | 10 mM | - |
| 1,10-phenanthroline | 10 mM | - |
| | | |

| Aspartyl | | |
|---|---|---|
| Chloromethyl ketonePepstatin | 100 µM | - |

ICE activity was inhibited only by sulfhydryl alkylating reagents. The inhibition may or may not be mechanistically related to ICE activity. Further testing on later purification stages in all cases yielded similar results. In all cases inhibition was measured as either complete (+) or no observable inhibition (-).

### EXAMPLE 12

### Synthesis Of Peptide Substrates

Peptides were synthesized via the Merrifield solid-phase technique using phenylacetamidomethyl resins and tBoc amino acids. Synthesis was performed on an Applied Biosystems 430A peptide synthesizer according to the manufacturers suggested protocols. Peptides were simultaneously deprotected and cleaved from the resin with 90% anhydrous HF, 10% anisole at 0° C for 1 h and then extracted from the resin with 10% acetic acid and lyophilized. The resulting crude peptides were purified by reversed phase HPLC on Waters C18 DeltaPak columns with a gradient of 5 to 70% acetonitrile in aqueous 0.2% trifluoroacetic acid (TFA). The structure of purified peptides was confirmed by mass spectral analysis.

Peptides of the general sequence
Asn-Glu-Ala-Tyr-Val-His-Asp-Ala-Pro-Val-Arg-Ser-Leu-Asn (hereafter referred to as P14mer) were also used for ICE characterization and were synthesized, purified and characterized as described above. Peptides of the sequence Ac-Tyr-Val-Ala-Asp-7-aminomethylcoumarin (Ac-Tyr-Val-Ala-Asp-AMC), N-(N-Acetyl-tyrosinyl-valinyl-alaninyl)-aspartic acid a-7-amino-4 methylcoumarin amide were synthesized by the following process.

### Step A:

N-Allyloxycarbonyl aspartic acid b-t-butyl ester a-7-amino-4-methylcoumarin amide. 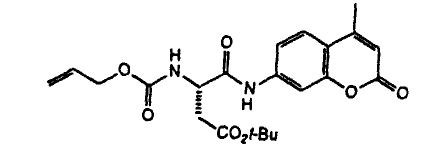 To a solution of N-alylloxycarbonyl aspartic acid b-t-butyl ester (3.44g, (12.6 mmol) and 7-amino-4-methylcoumarin (2.00 g, 11.42 mmol) in 15 mL of anhydrous dioxane was added ethyl dimethylaminopropyl carbodiimide (2.66 g, 13.86 mmol). After 75 min at reflux, the mixtuire was diluted with ethyl acetate and washed three times with 1 N hydrochloric acid and three times with saturated sodium bicarbonate. The solution was dried over sodium sulfate and concentrated in vacuo. The mixture was purified by HPLC on silica-gel (35x300 mm column, 10 % ethyl acetate in dichloromethane as eluent) to give the title compound as a colorless foam: 1H NMR (200 MHz, CD3OD) d 7.77 (d, 1H, J = 2.39 Hz), 7.68 (d, 1H, J = 9.06 Hz), 7.49 (dd, 1H, J = 2.36, 9.10 Hz), 6.21 (q, 1H, J = 1.30 Hz), 5.95 (m, 1H), 5.4-5.15 (m, 2H), 4.72-4.58 (m, 3H), 2.85 (dd, 1H, J = 6.17, 15.73 Hz), 2.65 (dd, 1H, J = 7.62, 16.37 Hz), 2.43 (d, 3H, J = 1.44 Hz), 1.43 (s, 9H).

### Step B:

Aspartic acid b-t-butyl ester a-7-amino-4-methylcoumarin amide. 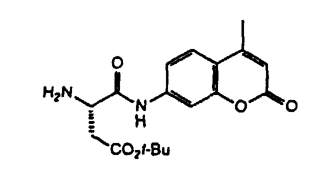 To a solution of N-Allyloxycarbonyl aspartic acid b-t-butyl ester a-7-amino-4-methylcoumarin amide (435 mg, 1.01 mmol) and Dimedone (1.13 g, 8.08 mmol) in 10 mL of anhydrous tetrahydrofuran was added tetrakis triphenylphosphine palladium (117 mg, 0.1 mmol). After 45 min, the mixture was diluted with ethyl acetate and washed five times with saturated sodium bicarbonate, dried over sodium sulfate and concentrated in vacuo. The mixture was disolved in a small amount of a solution of 1% ammonia and 10% methanol in dichloromethane and filtered through a 0.22 mm filter. The mixture was then purified by HPLC on silica-gel (22x300 mm column, eluting with a gradient of dichloromethane to 0.25% ammonia and 2.5 % methanol in dichloromethane) to give the title compound as a colorless foam: 1H NMR (200 MHz, CD3OD) d 7.93 (d, 1H, J = 1.76 Hz), 7.82 (d, 1H, J = 8.50 Hz), 7.63 (dd, 1H, J = 2.40, 9.10 Hz), 6.34 (q, 1H, J = 1.31 Hz), 3.89 (t, 1H, J = 6.35 Hz), 2.88 (dd, 1H, J = 6.03, 16.72 Hz), 2.75 (dd, 1H, J = 6.77, 16.75 Hz), 2.56 (d, 3H, J = 1.37 Hz), 1.54 (s, 9H).

### Step C:

N-(N-Acetyl-tyrosinyl-valinyl-alaninyl)-aspartic acid b-t-butyl ester a-7-amino-4-methylcoumarin. 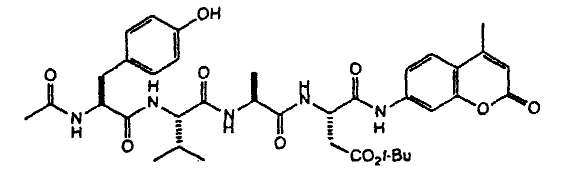 To a solution of N-(N-Acetyl-tyrosinyl-valinyl-alanine (288 mg, 0.733 mmol), aspartic acid b-t-butyl ester a-7-amino-4-methylcoumarin (242 mg, 0.698 mmol) and hydroxybenzotriazole (149 mg, 1.10 mmol) in 2 mL of dimethyl formamide at 0 °C was added dicyclohexylcarbodiimide (151 mg, 0.733). After 16 h at ambient temperature, the mixture was filtered and purified by Sephadex" LH-20 chromatography (1M x 50 mm column, methanol eluent). The resulting product was triturated with methanol to give the title compound as a colorless solid: 1H NMR (200 MHz, DMF-d7) d 8.3-7.5 (m, 7H), 7.09 (br d, 2H, J = 8.61 Hz), 6.72 (br d, 2H, J = 8.64 Hz), 6.27 (q, 1H, J = 1.31 Hz), 4.84 (m, 1H), 4.62 (m, 1H), 4.44-4.14 (m, 2H), 3.15-2.7 (m, 4H), 2.45 (d, 3H, J = 1.37 Hz), 2.13 (m, 1H), 1.87 (s, 3H), 1.41 (s, 9H), 1.37 (d, 3H. J = 7.38 Hz), 0.94 (d, 3H, J = 7.12 Hz), 0.93 (d, 3H, J = 7.12 Hz).

### Step D:

N-(N-Acetyl-tyrosinyl-valinyl-alaninyl)-aspartic acid a-7-amino-4-methylcoumarin amide. 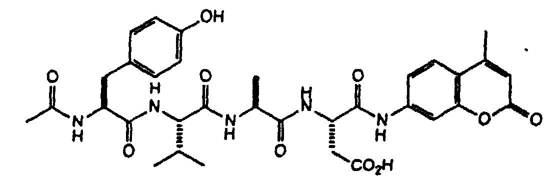 N-(N-Acetyl-tyrosinyl-valinyl-alaninyl)-aspartic acid b-t-butyl ester a-7-amino-4-methylcoumarin amide was disolved in trifluoroacetic acid. After 15 min the mixture was concentrated in vacuo to give the title compound as a colorless solid: 1H NMR (200 MHz, DMF-d7) d 8.3-7.5 (m, 7H), 7.09 (br d, 2H, J = 8.61 Hz), 6.72 (br d, 2H, J = 8.64 Hz), 6.27 (q, 1H, J = 1.31 Hz), 4.84 (m, 1H), 4.62 (m, 1H), 4.44-4.14 (m, 2H), 3.15-2.7 (m, 4H), 2.45 (d, 3H, J = 1.37 Hz), 2.13 (m, 1H), 1.87 (s, 3H), 1.41 (s, 9H), 1.37 (d, 3H. J = 7.38 Hz), 0.94 (d, 3H, .J = 7.12 Hz), 0.93 (d, 3H, J = 7.12 Hz). Microanalysis calculated for C33H39N5O10?1.65 H2O: C, 57.00, H, 6.13, N, 10.07; found: C, 56.97, H, 5.84, N 10.16.

### EXAMPLE 13

### Salt And pH Optimum Of Interleukin-1β Converting Enzyme

The pH optimum of THP-1 S-3 (Example 1) converting enzyme activity was determined. Samples of S-3 extracts were dialyzed against an assortment of buffers at pH values ranging form 5 to 9. Aliquots were removed from the dialyzed samples with the remainder being redialyzed against pH 7.4 HEPES buffer. The dialyzed samples were then tested again for converting enzyme activity. Pre-IL-1β converting enzyme has a narrow pH optimum situated between pH 7.0 and 8.0 and is not tolerant to exposure to acidic pH. Ion requirements of Pre-IL-1β converting enzyme were determined by salt titration. Pre-IL-1β converting enzyme (THP-1 derived) was incubated with increasing concentrations of KCl in the reaction. The results indicated that converting enzyme activity was optimal at low (<50 mM) concentrations. The extent of cleavage was assayed by SDS/PAGE according to Laemmli, Nature 227: 680-685 (1970), (see Example 9) followed by fluorography.

Using either crude THP-1 S-3 or partially purified enzyme as sources of ICE activity, salt and pH titrations were performed to determine optimum assay conditions as measured the by the gel based or P14mer cleavage assays, as described above. An example of both a salt and pH titration are included using crude THP-1 S-3. The data (Figure 3A) indicate that ICE activity is inhibited in a dose dependant fashion by the inclusion of KCl in the gel based cleavage assay. The pH optimum for ICE is approximately 7.4 in the gel based cleavage assay. The same experiment indicates that ICE activity is labile to acid pH but is stable in a basic pH environment. Cleavage of P14mer and Ac-YVAD-AMC exhibits a similar pH optimum and stability profile. The effects of salt, however, are somewhat different since the P14mer assay exhibits a sensitivity similar to the gel-based assay but the Ac-YVAD-AMC assay is essentially insensitive to salt. The data indicate that ICE is a neutral proteinase and exhibits no requirement for monovalent ions.

Figure 3A is a determination of the pH optimum of THP-1 S-3 converting enzyme activity. S-3 extracts were dialyzed against buffers at various pH values ranging from 5 to 9. Aliquots were removed from the dialysates and the remainder redialyzed against pH 7.4 HEPES buffer. All dialysates were then tested for converting enzyme activity. (-), S-300 at stated pH; (+), S-300 redialyzed against pH 7.4 HEPES buffer. The results demonstrate that ICE has a narrow pH optimum situated between pH 7.0 and 8.0 and is not tolerant to exposure to acid pH.

Figure 3B is a salt titration of converting enzyme activity. THP-1 S-300 extract was incubated with increasing concentrations of KCl in the gel-based cleavage assay. The results indicate that ICE activity is optimal at low (<50 mM) concentrations of KCl.

### EXAMPLE 14

### Characterization Of Interleukin-1β As A Thiol Protease

As shown in Example 11, Table 4a ICE is only inhibited by reagents capable of modifying thiol residues. The data in Table 3 was generated using partially purified enzyme or THP-1 S-3 and the gel-based cleavage assay as an indicator of ICE activity. This has been verified and extended using the P14mer assay as well as Ac-Tyr-Val-Ala-Asp-AMC as substrates. The only exception to the results of this table came when 1,10 phenanthroline (OP) was tested as an inhibitor. Originally it was shown that this compound did not inhibit ICE activity in the gel based cleavage assay. Using a discontinuous kinetic assay with P14mer as substrate (Example 12) revealed that OP can inhibit ICE activity in a time and concentration dependant manner. The rate constant (8 x 10-2 s-1 M-1) for inactivation is slow and it is not possible to reconstitute ICE activity by addition of ZnC12 following treatment with the chelator. Additional tests proved that, in fact, OP was functioning as an inhibitor by oxidizing ICE via a phenanthroline-metal complex. This was confirmed by: 1) demonstrating that the presence of high concentrations of the reducing agent DTT (10 mM) could protect ICE from the effects of OPA, suggesting that oxidation of a necessary component is responsible for ICE inactivation by OP. 2) Co-treatment of ICE with two metal chelators, EDTA and OP, did not result in an inhibition of ICE, a result that is inconsistent with the hypothesis that chelation of protein-bound metal is responsible for the observed inhibition of ICE activity by OP. 3) Addition of 10 mM CuSO4 to an ICE assay containing 1.0 mM OP results in a 17-fold increase in the rate at which ICE is inhibited by the compound, suggesting that a metal-phenanthroline complex is responsible for inhibition of ICE by OP. These data are consistent with the hypothesis that ICE contains in its structure a reduced thiol residue that, once oxidized or alkylated by a variety of means, renders the enzyme inactive. The data is not consistent with the hypothesis that ICE is a metalloprotease.

### EXAMPLE 15

### Identification of the 22 kD protein as being a component of ICE by active site labeling.

Since thiol alkylating reagents and metal-phenthroline complexes are capable of inhibiting ICE, it is possible that a necessary thiol group is located at or near the active site although other explanations (e.g. allosteric effects through a distant thiol) can be proposed to account for the effect. A necessary experiment, then, is to document that the thiol group is at a position capable of competing with ICE substrate. One method for obtaining this result is to demonstrate that the presence of saturating levels of substrate can block the inactivation of the enzyme by a reagent capable of inhibiting ICE through a covalent modification of a thiol group. A model reagent, iodoacetate, was chosen since the rate constant for inactivation of ICE (24 M-1 s-1) is much faster than carboxymethylation of simple cysteine residues. It has been determined that 100 mM iodoacetate will give a half-time of inhibition of 9.13 minutes (1 ∗ Km). Including saturating levels of the substrate Ac-Tyr-Val-Ala-Asp-AMC (200 ∗ Km or 2.8 mM) in the reaction results in theoretical protection of the enzyme to inactivation. Using 14C-iodoacetate as the alkylating reagent a similar protocol was used to identify proteins that could be labeled in the presence or absence of 200 x Km substrate. At a concentration of 100 mM radiolabeled iodoacetate a significant number of proteins are labeled during the course of the reaction. When 2.8 mM
Ac-Tyr-Val-Ala-Asp-AMC is included in the reaction < 2% of ICE is inactivated, a value close to that which is predicted. When the number and extent of labeled proteins is assessed by SDS-PAGE and fluorography it is clear that the labeling of a single protein of 22 kDa molecular weight was significantly reduced (Figure 4). The labeling of other proteins in the mixture was not affected, thus suggesting that the 22 kDa protein contains a thiol group capable of being alkylated with iodoacetate and that this thiol group is in a position capable of competing with the ICE substrate. As alkylation of this thiol correlates with the presence or absence of ICE activity, it can be stated that the 22 kDa protein comprises at least part of the ICE enzyme. ICE and 100 mM 14-C-iodoacetate were incubated in the presence (+S) or absence (-S) of 2.8 mM Ac-Tyr-Val-Ala-Asp-AMC and incubated for 1 hour. At 1 hour the reaction is quenched by the addition of 10 mM unlabeled iodoacetate and 10 mM DTT. Aliquots of the reaction were analyzed by SDS-PAGE followed by coomassie blue staining or autoradiography. The data indicate that no observable changes in protein composition occurred during the course of the reaction as based on coomassie blue staining. Autoradiography, however, indicates that a 22 kDa protein is labeled in the absence, but not the presence of ICE substrate.

### EXAMPLE 16

### Characterization of ICE as a thiol protease:

Characterization of ICE in terms of protease class was ultimately accomplished by the synthesis of two types of mechanism based thiol protease inhibitors, a diazomethyl ketone and a peptide aldehyde, both constructed from the Ac-Tyr-Val-Ala-Asp peptide. The peptidyl diazomethyl ketone (Ac-Tyr-Val-Ala-Asp-CHN2) and the peptide aldehyde (Ac-Tyr-Val-Ala-Asp-CHO) were both synthesized as described in an accompanying patent. It can be shown that Ac-Tyr-Val-Ala-Asp-CHN2 inhibits ICE activity irreversibly in a time and dose dependant manner, consistent with an irreversible alkylation of an active site thiol. Addition of excess amounts of substrate effectively compete against this inactivation, again indicating that an active site thiol is being modified.
Ac-Tyr-Val-Ala-Asp-CHO reversibly inhibits ICE in a dose dependant fashion and in a manner competitive with substrate. However, the specificity of action on ICE has been determined by synthesis of a chemically similar peptide aldehyde, Ac-Tyr(dAla)-Val-Ala-Asp-CHO and tested for its ability to inhibit ICE in a competitive and reversible manner. This compound was found to be 300-fold less potent at inhibiting ICE activity thus indicating that inhibition is dependant on recognition of a particular peptide structure represented by Ac-Tyr-Val-Ala-Asp-CHO and is not simply due to the presence of an aldheyde group on the C-terminus of a peptide.

### EXAMPLE 17

### Molecular Mass Determination Of Interleukin-1β Converting Enzyme

Five picomoles of the 22 and 10 kD proteins purified to homogeneity by method in Example 8 were subjected to capillary liquid chromatography, electrospray ionization, mass spectrometry. Mass determination was performed on a Finnigan Triple-Sector Quadropole Model 700 mass spectrometer. The standard error of mass determination was found to be 0.01 - .02% using a standard protein, bovine cytochrome c, to determine machine accuracy. Following biomass deconvolution of the electrospray ionization mass spectrum, the molecular weight of the isolated "10 kDa" component was found to have an average mass of 10,248 atomic mass units and the "22 kDa" protein was found to have an average mass of 19,866 atomic mass units.

### EXAMPLE 18

### Isoelectric Focusing Of Interleukin-1β Converting Enzyme

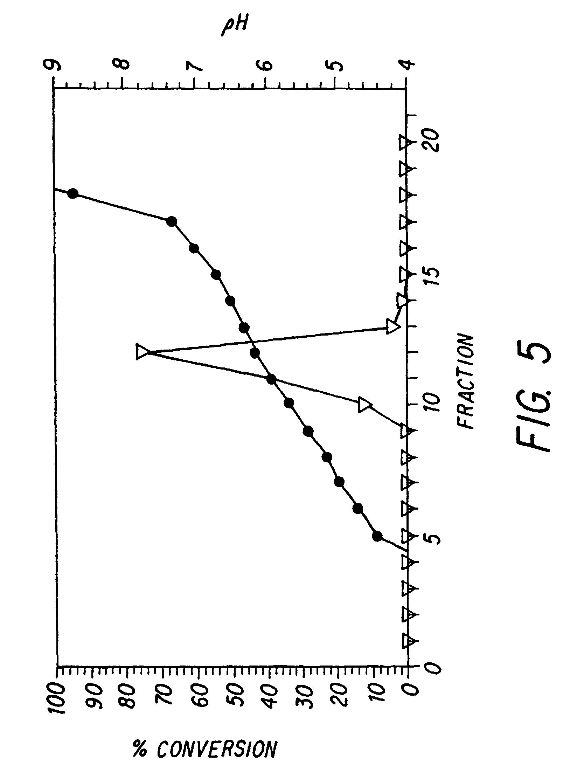

The isolelectric point of ICE was estimated using a Bio-Rad Rotofor solution phase isoelectric focusing apparatus. Ten ml of partially purified ICE enzyme containing approximately 1,260 units of ICE (a unit being defined as that amount of enzyme required to result in the cleavage of 40 pmoles of P14mer substrate per minute in a buffer containing 10% sucrose, 0.1% CHAPS, 20 mM HEPES, pH 7.4 200 mM P14mer substrate incubated at 25o C for 2 hours.) was diluted into 40 ml of a 10% Sucrose, 0.1% CHAPS, 0.2% Ampholytes (Bio-Lyte, pH 5 - 8 range) buffer and then subjected to isoelectric focusing using a Bio-Rad Rotofor electrofocusing apparatus. Electrophoresis was performed according to the manufacturers instructions. The electrofocusing gradient was eluted and fractions tested for ICE activity as described above using the P14mer peptide based assay and authentic pre-IL-1β as substrate in the gel-based cleavage assay . In both assays the peak of ICE activity focused to a portion of the gradient with a measured pH of 6.3 units, Figure 5.

### EXAMPLE 19

### Kinetic Evidence For Oligomeric Structure Of Interleukin-1β Converting Enzyme

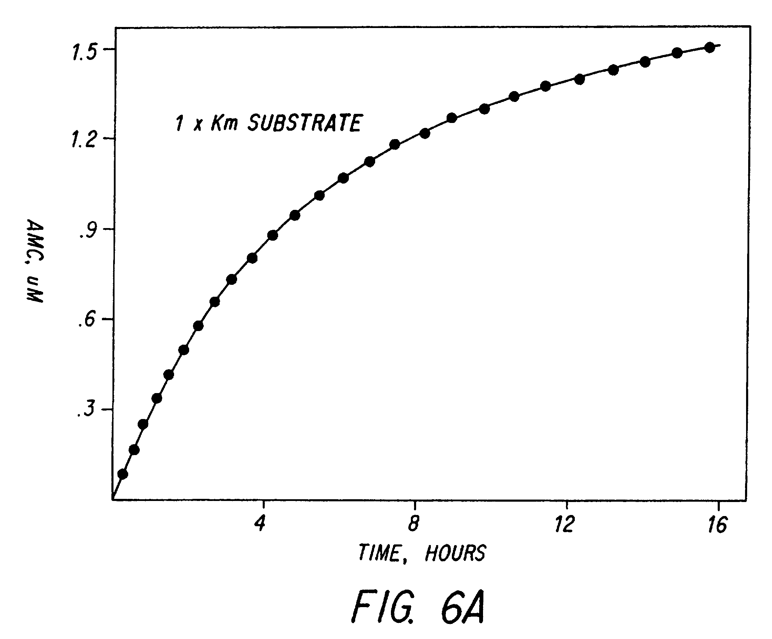

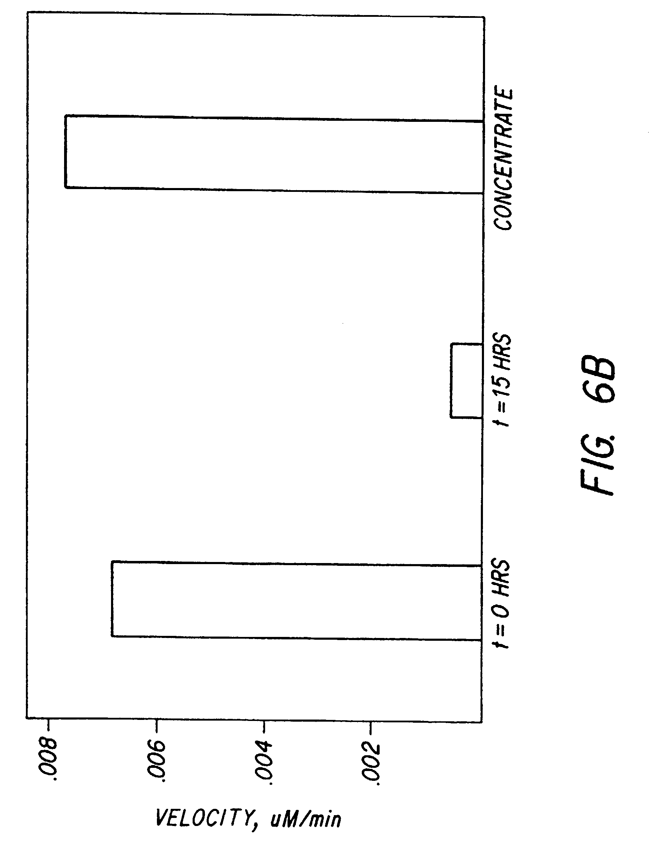

Original titration studies with partially purified ICE suggested that ICE cleavage activity was not strictly linear with enzyme concentration at low enzyme levels, a finding consistent with an oligomeric enzyme structure. Similarly, ICE activity is known to decrease following dilution into buffer in a time- dependant manner and will ultimately achieve a stable steady- state enzyme velocity. After this steady is achieved, the enzyme can then be reconcentrated to its original volume and, after a 24 hour incubation period, can be demonstrated to have the identical starting enzyme activity (Figure 6). This curve was obtained by diluting a standard stock solution of partially purified ICE (4 units/ul), 1000-fold into a standard assay mixture (50 uM substrate (1∗Km), 100 mM HEPES, 10% sucrose, 0.1% CHAPS, 10 mM DTT, 1 mg/ml BSA). The solid line is theoretical for a kinetic model that describes a first-order loss of enzyme activity with a rate constant,kobs = 0.004 min-1, corresponding to a half-life of 2.8 hours at 25°. The model also determines values for the initial reaction velocity (vo = 0.0068 uM AMC/min) and the velocity obtained at infinite time (vs = 0.00048 uM AMC/min). These parameters are also represented by bars in the insert of Fig. 6. If the experiment is repeated using saturating levels of substrate (1 mM, 20∗Km), no time-dependant inactivation is observed, indicating that the enzyme-substrate complex is kinetically stabilized. This dissociation is 100% reversable and complete reassociation of the complex is achieved if the diluted enzyme is reconcentrated 1000-fold to the original volume. These data suggest that loss of ICE activity is completely reversible and the sensitivity of ICE activity to dilution is due to the establishment of a new equilibrium between two dissociable species, this association being stabilized by saturating levels of substrate.

### EXAMPLE 20

### Stabilization Or Destabilization of Interleukin-1β Converting Enzyme Oligomer Following Treatment With Mixed Disulfides

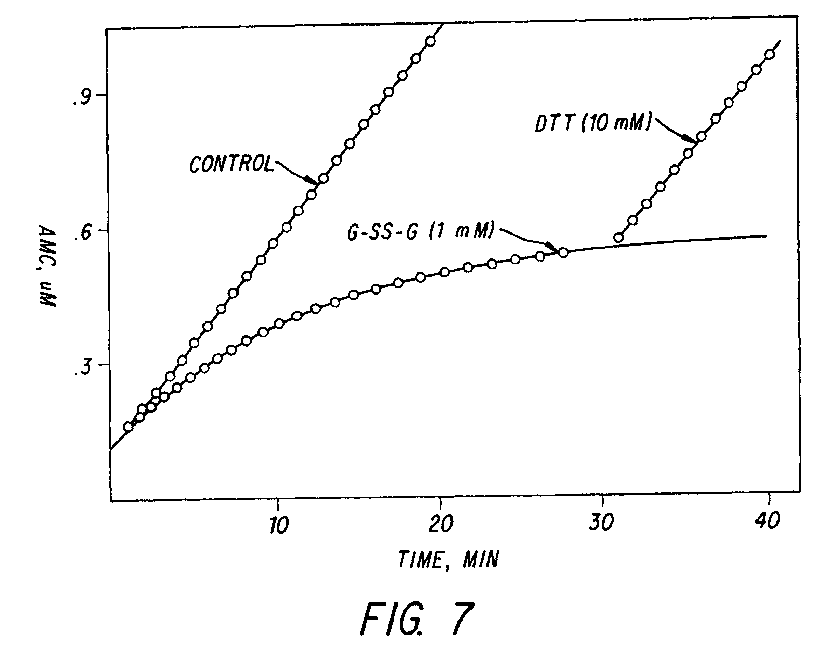

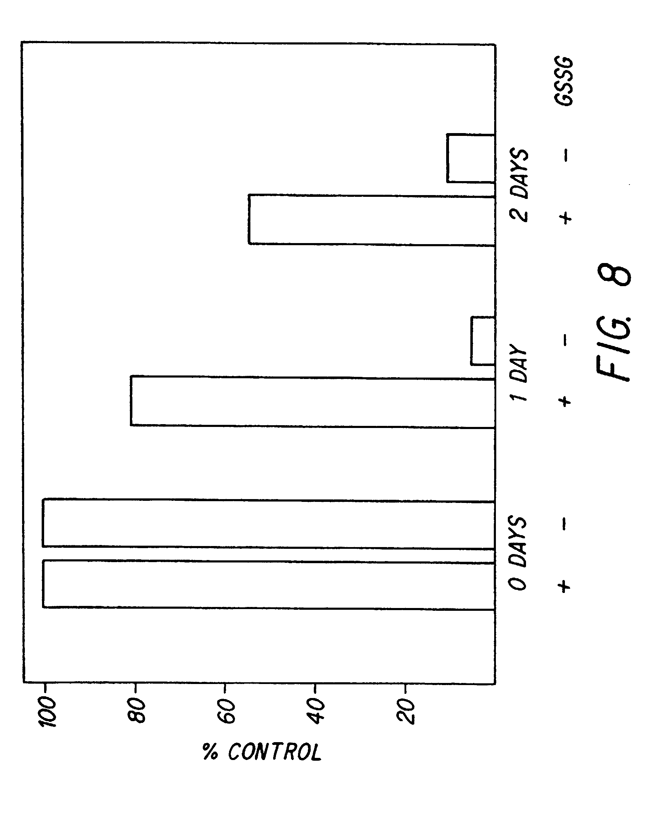

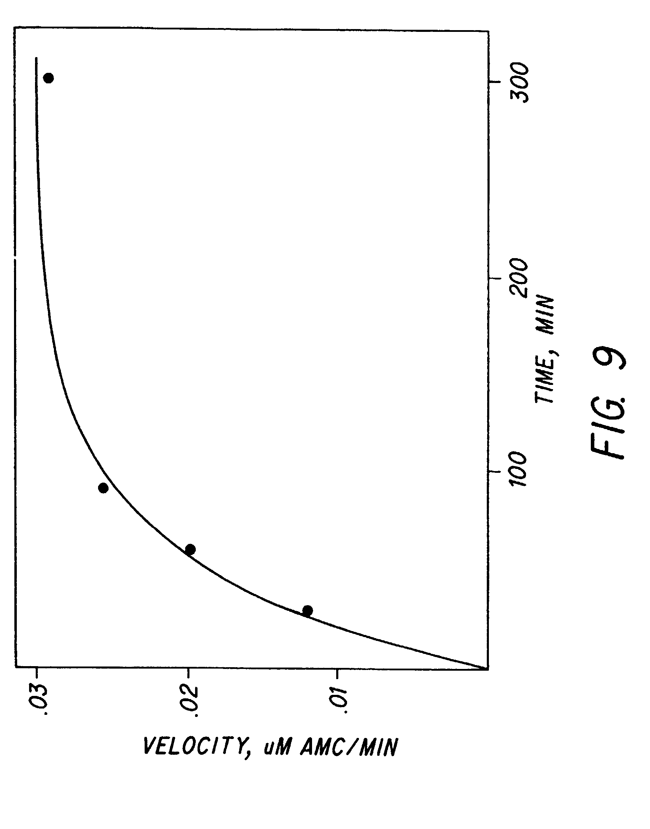

In an effort to generate reversible active site modifying reagents to facilitate purification and characterization of the enzyme, a variety of mixed disulfides were tested for their ability to reversibly inhibit ICE activity through generation of a mixed disulfide with the active site thiol. Two that were tested exhibited markedly dissimilar abilities to inhibit ICE in a DTT reversible manner. Oxidized glutathione (GSSG) and cystamine (2-aminoethanedithiol) were tested for their ability to inhibit ICE in a continuous fluorometric assay. ICE treated with 1 mM GSSG was inhibited with a t1/2 of 8 minutes. Addition of 10 mM DTT after the enzyme reaches its plateau of inactivation instantly reactivates the enzyme to 98% of starting activity, indicating a completely reversible process (Figure 7). The effects of GSSG treatment on the observed dissociation of ICE upon dilution was tested by treating concentrated ICE with GSSG, then diluting into substrate-free ICE assay buffer. The treated enzyme was allowed to equilibrate at room temperature then assayed for remaining ICE activity. Surprisingly, GSSG stabilized ICE activity, increasing the half-time of dissociation from 90 minutes to 2800 minutes, an increase of over 30 fold relative to untreated enzyme (Figure 8). When compared with GSSG, cystamine did not appear to stabilize ICE activity. This was demonstrated in experiments designed to assess the extent of reversibility of cystamine treatment. Following treatment of ICE with cystamine, the enzyme exhibits a time dependant decrease in activity with a half-time of 19 minutes at 5 mM cystamine. Upon addition of DTT after achievement of the inactivation plateau only 23% of enzyme activity could be recovered, suggesting the formation of irreversibly inactivated enzyme. That the apparently "irreversibly" inactivated enzyme was in fact dissociated monomer was shown by treating highly concentrated ICE (3 units/ml) with 5 mM cystamine followed by a 3 day equilibration at 4o C. Following addition of DTT to the concentrated enzyme, recovery of activity was monitored by removing aliquots, diluting them into ICE assay buffer and measuring the initial velocity of the reaction. Complete recovery of activity was observed and was achieved with a halflife of 36 minutes (Figure 9). This suggests that cystamine dissociates ICE into monomeric components which, if suitably concentrated, can reversibly associate following treatment with DTT.

### EXAMPLE 21

### Size Exclusion Chromatography Of Oxidized Glutathione Or Cystamine Modified Interleukin-1β

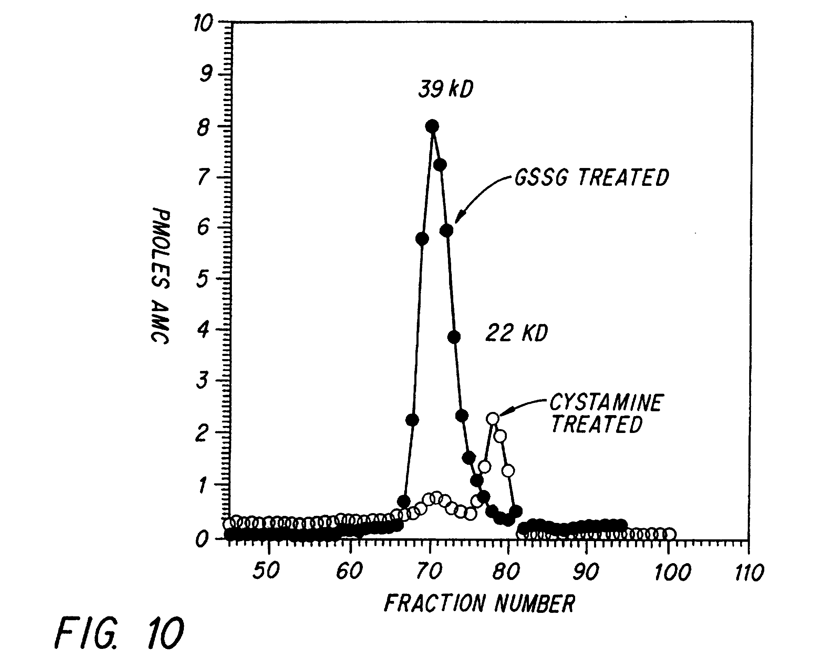

Since the data in Example 20 suggests that the association of ICE oligiomers can be influenced by pretreatment with either GSSG or cystamine, experiments were carried out to discern the molecular weight of the stabilized and destabilized enzyme. As previously noted, Example 5, ICE elutes with an apparent Mr of 23,000 on TSK G2000 or 30,000 on TSK G3000 columns in the presence of DTT. When concentrated ICE is first pretreated with GSSG or cystamine for 24 hrs then passaged over a TSK G2000 size exclusion column, the enzyme activity elutes from the column with two separable and distinct molecular weights. GSSG treated enzyme elutes from the column with an apparent Mr 39,000 while cystamine treated enzyme had an apparent Mr of 22,000 (Figure 10). When SDS-PAGE gels of column fractions are analyzed for protein content by silver staining, it can be observed that the 22 and 10 kD proteins coelute with ICE activity and the elution position of these two proteins changes with the change in molecular weight observed following GSSG or cystamine treatment. This data indicates that the 22 and 10 kD proteins comprise two subunits of the ICE molecule and that the apparently reversible association of these proteins can account for the oligomeric behavior of ICE in solution.

### EXAMPLE 22

### Affinity Chromatography of Interleukin-1B Converting Enzyme

### Preparation of a chromatographic matrix from Compound A

An affinity column for interleukin-1 converting enzyme was prepared from the potent peptide aldehyde inhibitor Acetyl-Tyr-Val-Lys-Asp-CHO (Compound A), coupled via a 12-atom bis-oxirane spacer to SEPHAROSE CL-4B through the lysine residue.

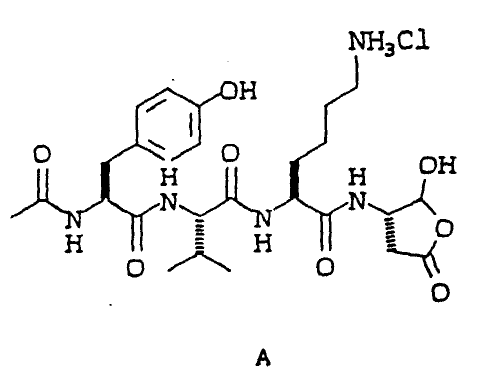

### Synthesis Of Affinity Matrix

### Step A: Epoxy-activated SEPHAROSE CL-4B:

Epoxy-activated SEPHAROSE CL-4B was prepared as described in the literature (Sundberg, L., and Porath, J. (1974) J. Chromatogr. 90, 87-98). Specifically, a slurry consisting of 100 gm suction-dried SEPHAROSE CL-4B, 100 ml of 1,4-butanediol diglycidyl ether (a nominal 70% solution), and 100 ml 0.6M NaOH containing 2 mg/ml NaBH4 was mixed with an overhead stirrer for 16 hours at ambient temperature. The resulting epoxy-activated SEPHAROSE CL-4B was washed exhaustively on a coarse sintered glass funnel with 10 liters of water, and stored in water at 4°C.

### Step B: Coupling of Peptide Aldehyde Dimethyl Acetal:

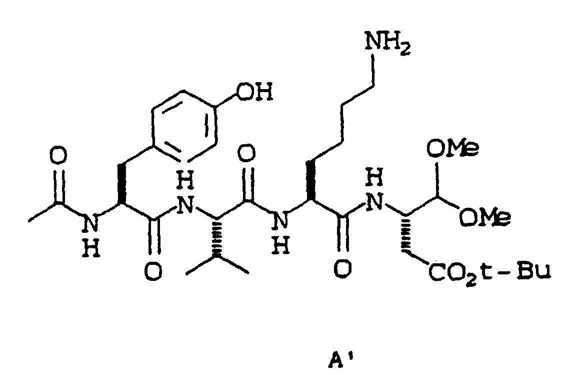

The blocked aspartyl-t-butyl ester, dimethyl acetal (Compound A') of the active aldehyde, Compound A, was dissolved as a 10 mM solution in methanol, and then combined with more methanol, water, and a 400 mM sodium carbonate solution adjusted to pH 11.00 with HCl, to give a 50% methanol solution containing 2 mM inhibitor and 200 mM carbonate buffer. This solution (10 ml) was mixed with the suction-dried cake (10 gm) of epoxy-activated SEPHAROSE CL-4B, and the slurry was stirred by rotation at 37°C for 3 days. The resulting affinity matrix was washed thoroughly with 1M KCl and water, and was stored as a slurry at 4°C. The incorporation, based on results with [14-C]-lisinopril (Bull, H.G., Thornberry, N.A., and Cordes, E.H. (1985) J. Biol. Chem. 260, 2963-2972), is estimated to be 1 umol/ml packed affinity matrix.

### Step C: Activation to Aldehyde:

The above procedure gave the dimethyl-acetal of Acetyl-Tyr-Val-Lys-Asp-CHO coupled to the spacer arm, the t-butyl protecting group on the aspartate residue being lost during the coupling conditions. Activation of this matrix to the aldehyde was carried out in the affinity column just prior to use, by equilibrating the matrix with 0.01N HCl and letting it stand for 2 hours at 25°C. A control matrix containing [14-C]glycine as a tracer gave no evidence (<1%) for loss of ligand under these conditions.

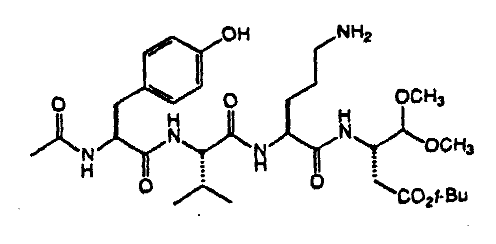

### Synthesis of N-(N-Acetyl-tyrosinyl-valinyl-lysinyl)-3-amino-4-oxobut anoic acid dimethyl acetal b-t-butyl ester.

### Step A:

### N-allyloxycarbonyl-3-amino-4-hyroxybutanoic acid tert-butyl ester.

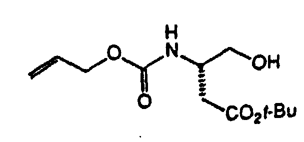

To a solution of N-allyloxycarbonyl (S)-aspartic acid b-tert-butyl ester (2.00 g, 7.32 mmol) in 50 mL of tetrahydrofuran (THF) at 0 °C, was added N-methyl morpholine (NMM, 885 mL, 8.05 mmol) followed by isobutyl chloroformate (IBCF, 997 mL, 7.68 mmol). After 15 min, this mixture was added to a suspension of sodium borohydride (550 mg, 14.55 mmol) in 50 mL of THF and 12.5 mL of methanol at -45 °C. After 30 min at -45 °C, the mixture was warmed to 0 °C and held at that temperature for 30 min. The reaction was quenched with acetic acid, diluted with 1:1 ethyl acetate:hexane, and washed 3 times with dilute sodium bicarbonate. The organics were dried over sodium sulfate, filtered, and concentrated. The residue was purified by MPLC on silica-gel (35x350 mm column, 30% ethyl acetate/hexane) to give the desired product: 1H NMR (200 MHz, CD3OD) d 5.9 (m, 1H), 5.28 (br d, 1H, J = 17 Hz), 5.15 (br d, 1H, J = 9 Hz), 4.52 (br d, 2H, J = 6 Hz), 3.98 (m, 1H), 3.48 (ABX, 2H, J = 5, 6, 11 Hz), 2.53 (dd, 1H, J = 5, 16 Hz), 2.32 (dd, 1H, J = 9, 16 Hz), 1.43 (s, 9H).

### Step B:

### N-allyloxycarbonyl-3-amino-4-oxobutanoic acid b-tert-butyl ester dimethyl acetal.

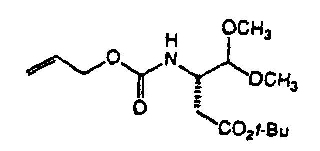

To a solution of dimethyl sulfoxide (757 mL, 10.67 mmol) in 10 mL of dichloromethnane at _45 °C was added oxalyl chloride (508 mL, 5.82 mmol). After 5 min, a solution of N-allyloxycarbonyl-3-amino-4-hyroxybutanoic acid tert-butyl ester (1.25 g, 4.85 mmol) in 10 mL of dichloromethane was added. After 15 min, triethyl amine (2.03 mL, 14.55 mmol) was added. After 30 min, the mixture was warmed to -23 °C and stirred for 30 min. The mixture was diluted with 1:1 ethyl acetate/hexane, washed with water, 1 N sodium hydrogensulfate, and twice with water. The organics were dried over sodium sulfate, filtered, and concentrated. The resultant oil was disolved in 200 mL of methanol and 20 mL of trimethyl orthoformate and 100 mg of p-toluene sulphonic acid were added. After 16 hours, the reaction was quenched with saturated sodium bicarbonate and concentrated in vacuo. The mixture was diluted with ether and washed 5 times with dilute sodium bicarbonate. The ether layer was dried over magnesium sulfate, filtered, and concentrated to afford the title compound as a colorless oil: 1H NMR (200 MHz, CD3OD) d 5.9 (m, 1H), 5.26 (br d, 1H, J = 17 Hz), 5.14 (br d, 1H, J = 10 Hz), 4.51 (br d, 2H, J = 5.33 Hz), 4.25 (d, 1H, J = 4.79 Hz), 4.11 (m, 1H), 3.40 (s, 3H), 3.39 (s, 3H), 2.52 (dd, 1H, J = 4.86, 15.27 Hz), 2.30 (dd, 1H, J = 9.00, 15.28 Hz), 1.43 (s, 9H).

### Step C:

### 3-Amino-4-oxobutanoic acid b-tert-butyl ester dimethyl acetal.

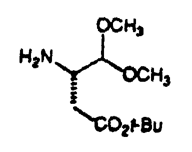

To a solution of N-allyloxycarbonyl-3-amino-4-oxobutanoic acid b-tert-butyl ester dimethyl acetal (312 mg, 1.03 mmol) in 10 mL of THF was added morpholine ( 897 mL, 10.3 mmol) and tetrakis triphenylphosphine palladium (100 mg). After 3 hours, the mixture was diluted with 1:1 ethyl acetate/hexane and washed 5 times with dilute sodium bicarbonate. The orgains were dried over sodium sulfate, filtered, and concentrated. The resulting oil was purified by MPLC on silica-gel (22x300 mm column, linear gradient of dichloromethane to 1% ammonia and 10 % methanol in dichloromethane) to afford the title compound as a pale-yellow oil: 1H NMR (200 MHz, CD3OD) d 4.15 (d, 1H, J = 5.67 Hz), 3.41 (s, 3H), 3.40 (s, 3H), 3.19 (m, 1H), 2.47 (dd, 1H, J = 4.88, 16.06 Hz), 2.22 (dd, 1H, J = 7.86, 16.16 Hz), 1.45 (s, 9H).

### Step D:

### N-(N-Acetyl-tyrosinyl-valinyl-(e-CBZ-lysinyl))-3-amino-4-oxobutanoic acid b-tert-butyl ester dimethyl acetal.

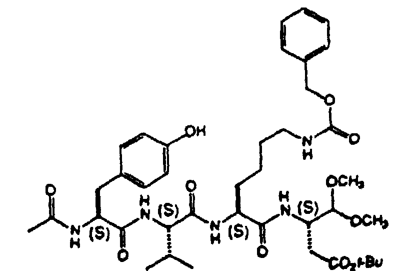

To a solution of 3-Amino-4-oxobutanoic acid b-tert-butyl ester dimethyl acetal (238 mg, 1.09 mmol) in 5 mL of DMF at 0 °C was added N-methyl morpholine (599 mL, 5.45 mmol) followed sequentially by N-Acetyl-tyrosinyl-valinyl-e-CBZ-lysine (735 mg, 1.09 mmol), hydroxybenzotriazole (221 mg, 1.64 mmol), and dicyclohexylcarbodiimide (225 mg, 1.09 mmol). After 16 hours at ambient temperature, the mixture was filtered and purified by Sephadex" LH-20 chromatography (1M x 50 mm column, methanol eluent). The resulting product was further purified by MPLC on silica-gel (22 x 300 mm column, eluting with a linear gradient of dichloromethane to 1% ammoinia and 10% methanol in dichloromethane) to give the title compound as a colorless solid: 1H NMR (200 MHz, CD3OD) d 7.31 (br s, 5H), 7.04 (br d, 2H, J = 8.35 Hz), 6.67 (br d, 2H, J = 8.45 Hz), 5.04 (s, 2H), 4.61 (m, 1H), 4.44-4.25 (m, 3H), 4.17 (d, 1H, J = 7.27 Hz), 3.39 (s, 3H), 3.38 (s, 3H), 3.1-2.9 (m, 3H), 2.75 (dd, 1H, J = 9.28, 14.12 Hz), 2.53 (dd, 1H, J = 5.47, 15.58 Hz), 2.33 (dd, 1H, J = 7.96, 15.53 Hz), 2.04 (m, 1H), 1.88 (s, 3H), 1.8-1.2 (m, 6H), 1.41 (s, 9H), 0.94 (d, 6H, J = 6.74 Hz).

### Step E:

### N-(N-Acetyl-tyrosinyl-valinyl-lysinyl)-3-amino-4-oxobutanoic acid dimethyl acetal b-t-butyl ester.

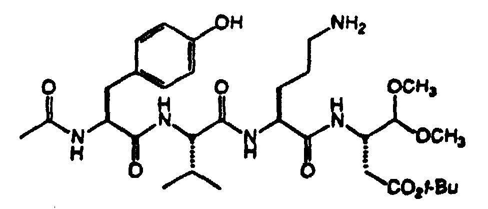

A solution of N-(N-Acetyl-tyrosinyl-valinyl-e-CBZ-lysinyl)-3-amino-4-oxobutanoic acid b-tert-butyl ester dimethyl acetal (15.6 mg) was disolved in 2 mL of methanol and 10 mg of Pearlman's catalyst (Pd(OH)2 on Carbon) was added. After 30 min under hydrogen, the mixture was filtered and concentrated to give the title compound: 1H NMR (200 MHz, CD3OD) d 7.04 (br d, 2H, J = 8.44 Hz), 6.67 (br d, 2H, J = 8.54 Hz), 4.57 (dd, 1H, J = 5.23, 9.04 Hz), 4.5-4.0 (m, 4H), 3.38 (s, 3H), 3.34 (s, 3H), 3.02 (dd, 1H, J = 5.17, 13.81 Hz), 2.75 (dd, 1H, J = 9.23, 14.06 Hz), 2.66 (t, 2H, J = 7.08 Hz), 2.53 (dd, 1H, J = 5.47, 15.58 Hz), 2.34 (dd, 1H, J = 7.91, 15.57 Hz), 2.03 (m, 1H), 1.88 (s, 3H), 1.9-1.2 (m, 6H), 1.41 (s, 9H), 0.94 (d, 6H, J = 6.69 Hz), 0.93 (d, 3H, J = 6.64 Hz).

### Affinity Chromatography Procedure

The starting enzyme preparation was purified about 100-fold from THP-1 cell lysate by anion exchange chromatography as described in Examples 2, 3, 5-8 and/or 10.

### Step A: Binding of ICE:

The activated affinity column (5 ml, 1 cm x 6.5 cm) and a guard column of native SEPHAROSE CL-4B of equal dimensions were equilibrated with 10 column volumes of the chromatography buffer (100 mM hepes, 10% sucrose, and 0.1% 3-[(3-cholamidopropyl)-dimethylammonio]-1-propanesulfonate (CHAPS) at pH 7.50) supplemented with 1 mM dithiothreitol. The enzyme solution (15 ml, 150,000 units, 150 mg protein) was applied through the guard column and run onto the affinity column at a flow rate of 0.022 ml/min at 4°C, and washed through with an additional 10 ml chromatography buffer at the same flow rate. During loading, 8% of the enzymatic activity was not retained, presumably due to the slow rate constant for binding. After loading, the guard column was removed and the affinity column was washed with 25 column volumes of buffer at a faster flow rate of 0.5 ml/min at 4°C. No enzymatic activity was detected in the wash fractions.

### Step B: Elution of Bound ICE:

To elute the enzyme, the column was then flooded with 1 column volume of buffer containing 200 mM Acetyl-Tyr-Val-Lys(CBZ)-Asp-CHO (Compound B), and left for 24 hours at room temperature to achieve dissociation of the matrix-bound enzyme. The free enzyme-inhibitor complex was then recovered from the affinity column by washing with 2 column volumes of buffer at a flow rate of 0.022 ml/min. Repeating the exchange with fresh inhibitor produced < 5% more enzyme, indicating that the first exchange had been adequate.

### Step C: Reactivation of ICE:

The eluted ICE was reactivated using two synergistic chemical approaches: conversion of the inhibitor to its oxime, and oxidation of the active site thiol to its mixed disulfide with glutathione by thiol-disulfide interchange.

The enzyme-inhibitor solution recovered from the affinity column was adjusted to contain 100 mM neutral hydroxylamine and 10 mM glutathione disulfide to effect reactivation. Under these conditions, after a short lag with a halflife of 100 sec for consumption of excess free inhibitor, the dissociation of E-I complex is entirely rate determining with a halflife of about 100 min at 25°C. After allowing 10 halflives for the exchange, the inhibitor oxime and excess reagents were removed by exhaustive desalting in an AMICON CENTRICON-10 ultrafiltration cell using the chromatography buffer at 4°C. When desired, the enzyme-glutathione conjugate was reduced with 10 mM dithiothreitol (halflife < 1 min) to give active enzyme. The purified enzyme is stable indefinitely at -80°C. The recovery of enzymatic activity by affinity chromatography was >90%, and the final purification achieved was about 75,000-fold, as measured by SDS-polyacrylamide gel electrophoresis. The results are summarized on Table 4b.

| TABLE 4b | | | | | | |
|---|---|---|---|---|---|---|
| AFFINITY PURIFICATION OF ICE | | | | | | |
| | vol. (ml) | units | units/ml | mg | mg/ml | units/ mg |
| DEAE sample | 15 | 150,000 | 10 | 150 | 10 | 3 10 |
| Affinity Eluate | 0.2 | 140,000 | 700 | 0.03* | 0.15 | 4.7 × 10⁶ |

| | | | | | | |
|---|---|---|---|---|---|---|
| * estimated from silver staining intensity on SDS-PAGE | | | | | | |

Recovery of ICE was 93% with a purification of 4700-fold.

### Synthesis of N-(N-Acetyl-tyrosinyl-valinyl-e-CBZ-lysinyl)-3-amino-4-oxobutanoic acid.

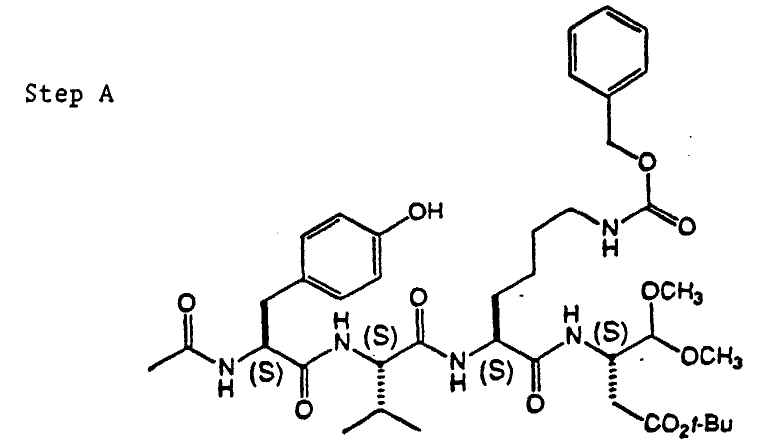

N-(N-Acetyl-tyrosinyl-valinyl-(e-CBZ-lysinyl))-3-amino-4-oxobutanoic acid β-tert-butyl ester dimethyl acetal. To a solution of 3-Amino-4-oxobutanoic acid β-tert-butyl ester dimethyl acetal (238 mg, 1.09 mmol) in 5 mL of DMF at 0°C was added N-methyl morpholine (599 mL, 5.45 mmol) followed sequentially by N-Acetyl-tyrosinyl-valinyl-e-CBZ-lysine (735 mg, 1.09 mmol), hydroxybenzotriazole (221 mg, 1.64 mmol), and dicyclohexylcarbodiimide (225 mg, 1.09 mmol). After 16 hours at ambient temperature, the mixture was filtered and purified by SEPHADEX LH-20 chromatography (1M x 50 mm column, methanol eluent). The resulting product was further purified by MPLC on silica-gel (22 x 300 mm column, eluting with a linear gradient of dichloromethane to 1% ammoinia and 10% methanol in dichloromethane) to give the title compound as a colorless solid: 1H NMR (200 MHz, CD3OD) d 7.31 (br s, 5H), 7.04 (br d, 2H, J = 8.35 Hz), 6.67 (br d, 2H, J = 8.45 Hz), 5.04 (s, 2H), 4.61 (m, 1H), 4.44-4.25 (m, 3H), 4.17 (d, 1H, J = 7.27 Hz), 3.39 (s, 3H), 3.38 (s, 3H), 3.1-2.9 (m, 3H), 2.75 (dd, 1H, J = 9.28, 14.12 Hz), 2.53 (dd, 1H, J = 5.47, 15.58 Hz), 2.33 (dd, 1H, J = 7.96, 15.53 Hz), 2.04 (m, 1H), 1.88 (s, 3H), 1.8-1.2 (m, 6H), 1.41 (s, 9H), 0.94 (d, 6H, J = 6.74 Hz). 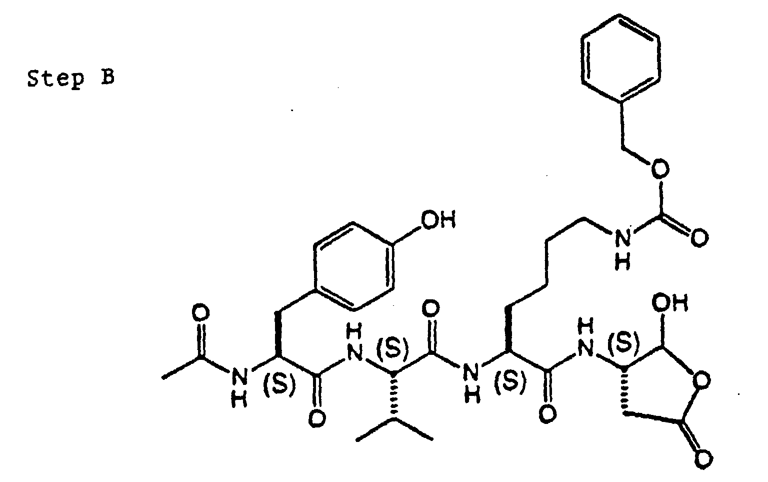 N-(N-Acetyl-tyrosinyl-valinyl-e-CBZ-lysinyl)-3-amino-4-oxobutanoic acid.

A solution of N-(N-Acetyl-tyrosinyl-valinyl-e-CBZ-lysinyl)-3-amino-4-oxobutanoic acid β-tert-butyl ester dimethyl acetal (14.9 mg) was treated with 1 mL of trifluoroacetic acid, aged for 15 minutes, and concentrated in vacuo. The residue was dissolved in 1.0 mL of methanol and 1.0 mL of water containing 20 uL of thionyl chloride was added. After 1 hour, the pH of the solution was adjusted to around 5 with sodium acetate to afford a solution of the title compound: compound: 1H NMR (200 MHz, CD3OD) d 7.33 (br s, 5H), 7.05 (br d, 2H, J = 8.35 Hz), 6.74 (br d, 2H, J = 8.35 Hz), 4.6-3.9 (m, 5H), 3.1-2.3 (m, 6H), 1.98 (m, 1H), 1.92 (s, 3H), 1.8-1.2 (m, 6H), 0.89 (d, 6H, J = 6.60 Hz).

### EXAMPLE 23

### Generation of ICE specific DNA products by PCR

Degenerate oligonucleotides were designed based on the amino acid sequence from the amino terminus and internal regions of both the 20 kDa and 10 kDa proteins (Figure 17 and 18). For the 20 kDa protein, the amino terminal primer, GAYCCNGCNATGCCNAC (SEQ.ID.NO.:3), was 128 fold degenerate while the internal primer, 3'ATRGGNTADTACCTRTT5' (SEQ.ID.NO.:4), was 48 fold degenerate. For the 10 kDa protein, the amino terminal primer, GCNATHAARAARGCNCA (SEQ.ID.NO.:5), was 192 fold degenerate while the internal primer, GTYTACGGNTGNTGNCT (SEQ.ID.NO.:6), was 128 fold degenerate.

Single-stranded THP.1 cDNA was synthesized from THP.1 cellular poly A+ mRNA and used as a PCR template. PCR was conducted essentially as described by a modification of the MOPAC procedure (Lee, et al., Science 239, 1288 (1988)). For each PCR, 10 pmol of each primer was added to that quantity of cDNA synthesized for 0.4µg of poly A+ mRNA in a reaction buffer consisting of 50 mM KCl, 10 mM TRIS-HCl (pH 8.3), 1.5 mM MgCl₂, 0.01% w/v gelatin, and 200 uM of each dNTP in a final volume of 10µl. The PCR program consisted of one cycle of denaturation for 100°C for 10 minutes, the addition of 2 units of Taq polymerase, followed by 30 cycles of the following steps: 95°C for 30 seconds, 48°C for 30 seconds, and 70°C for 1 minutes. For the 20 kDa protein, a PCR product of 116 bp was synthesized and its identity was verified by hybridization with an internal inosine-substituted oligonucleotide [ATIGGRTAIATYTCIGCR](SEQ.ID.NO.:7), while for the 10 kDa protein, a PCR product of 221 bp was synthesized and verified with a similar type of probe [ATIGARAARGAYTTYATIGC](SEQ.ID.NO.:8). Both PCR products were subcloned into Bluescript vectors (Stratagene), and sequenced by the chain termination method (Sanger, et al., PNAS 74, 5463 (1977)).

The deduced amino acid sequence for the 20 kDa and the 10 kDa subunit are shown in Table 7, panel A, and panel B, respectively.

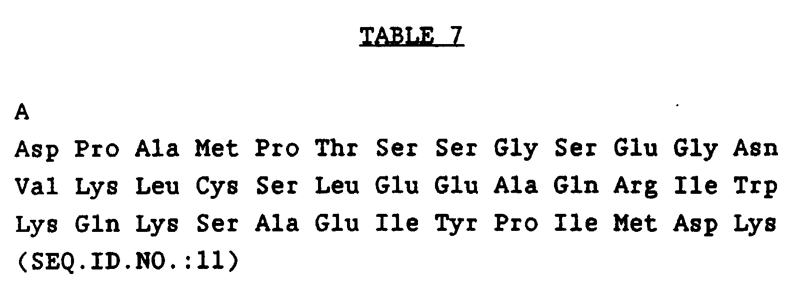

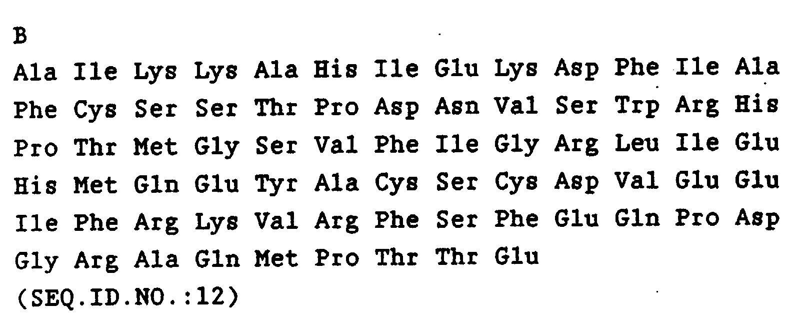

### EXAMPLE 24

### Isolation of ICE cDNA clones from lambda cDNA libraries

The PCR derived products for the 10kda and 20kda ICE subunits were used as hybridization probes for screening a lambda gt10 cDNA library from THP.1 cells (Clontech). Plating and plaque lifts of the library were performed by standard methods (T. Maniatis, E.F. Fritsch, J. Sambrook, Molecular Cloning: A Laboratory Manual (Cold Spring Harbor Laboratory, Cold Spring Harbor, New York, 1982). The probes were random-primed labelled with ³²P-dCTP to high specific activity and a separate screening of the library (600,000 plaques per screen) was conducted with each probe. The probes were added to hybridization buffer (50% formamide, 5X Denhardts, 6X SSC (1X SSC = 0.15 M), 0.5% SDS, 100 µg/ml salmon sperm DNA) at 1 x 10⁶ cpm/ml. Eleven positively hybridizing phage were detected using the 10 kDa specific probe while seven positively hybridizing phage were observed using the 20 kDa probe.

### EXAMPLE 25

### Subcloning and sequencing of ICE cDNA clones

Several cDNA clones ranging in size from 1.0 kb to 1.6 kb in length and containing a single open reading frame of 404 amino acids were subcloned into pGEM vectors (Promega) and bi-directionally sequenced in their entirety by the method of Sanger. The sequence for the full-length cDNA encoding ICE is shown in Table 5 (A), with the 24 kDa (B), 20 kDa (C), and 10 kDa (D) coding regions shown as well. The full length clone was designated clone OCP9. The deduced amino acid sequence of full length ICE from the cloned cDNA is shown in Table 6 (A), with the deduced amino acid sequence for the, 24 kDa (B), 20 kDa (C), and 10 kDa (D) subunits shown as well.

Inspection of the deduced amino acid sequence and comparison with the amino acid sequences derived from purified native ICE 20 kDa and 10 kDa subunits (Figure 19 and 20) reveals amino acid sequence identity except for one amino acid found at position 120 of the full length nascent ICE protein. According to the cDNA sequence, Asn is encoded at position 120 of the full-length protein. This amino acid position corresponds to the NH₂-terminus of the 20 kDa subunit. The amino acid sequence derived from the purified native 20 kDa subunit determined the NH₂-terminal amino acid to be Asp. This difference between the deduced amino acid sequence and the known amino acid sequence, the only difference found, may be attributable to deamidation of the NH₂-terminal Asn of the 20 kDa subunit to form Asp. This type of deamidation is known to occur and is common when Asn is found at the NH₂-terminus. Whether Asn or Asp is found at the NH₂-terminus of the 20 kDa subunit, or whether Asn or Asp is found within the ICE polypeptide at position 120 of the full length protein, or in a polypeptide fragment, is not expected to affect ICE activity significantly, if at all.

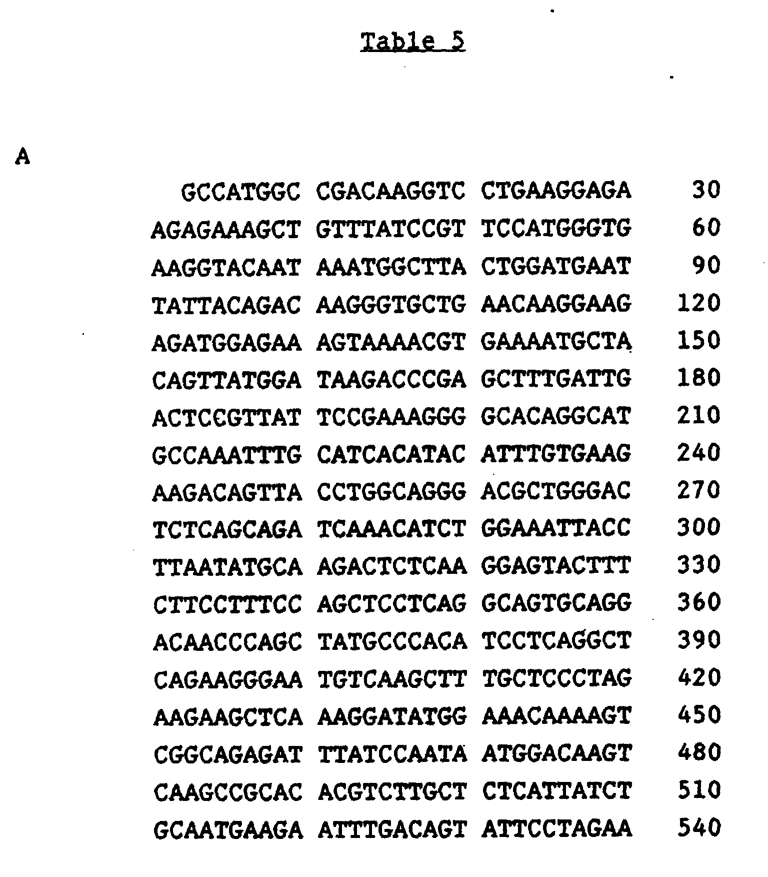

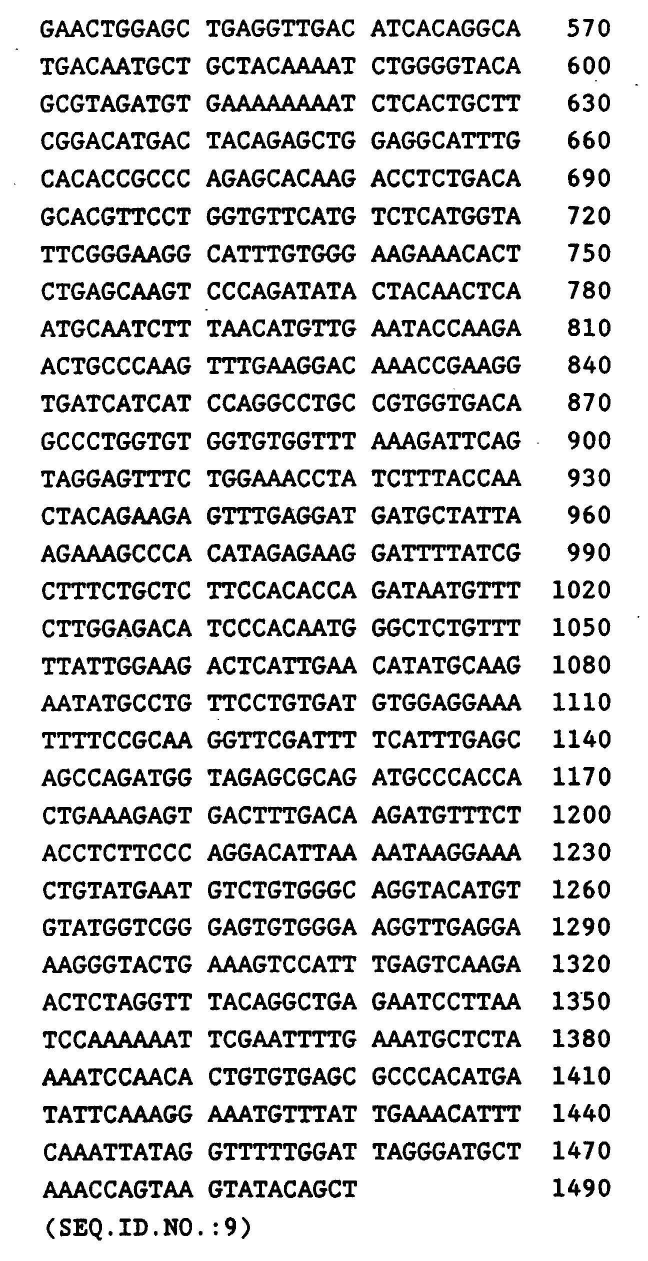

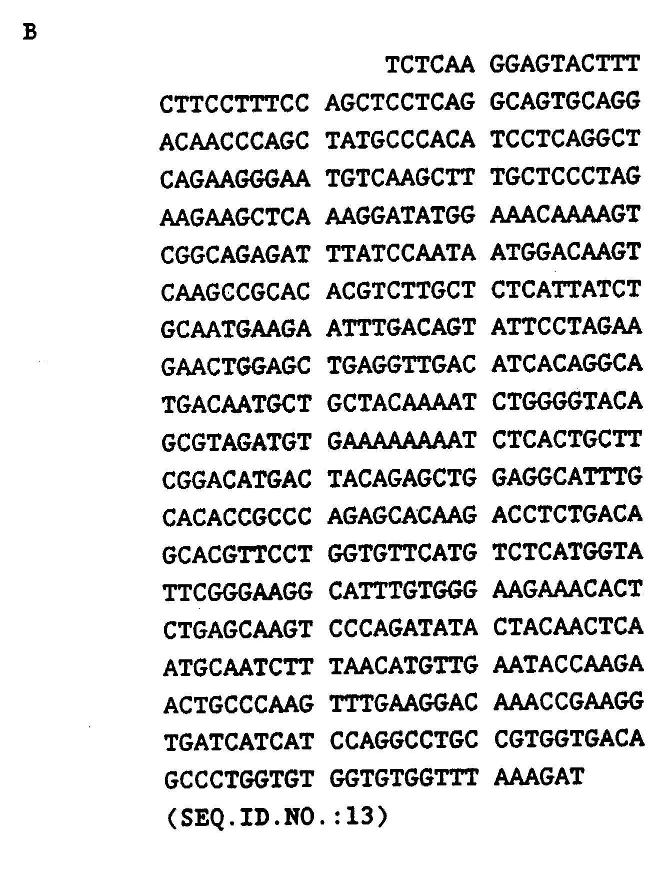

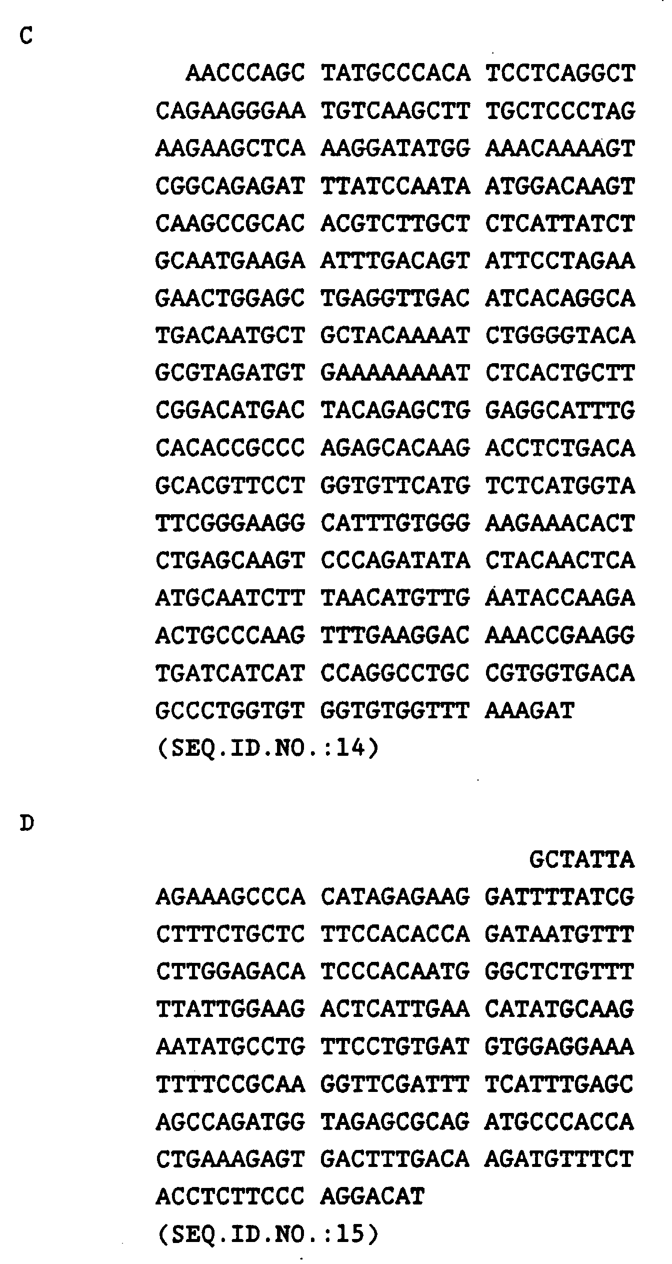

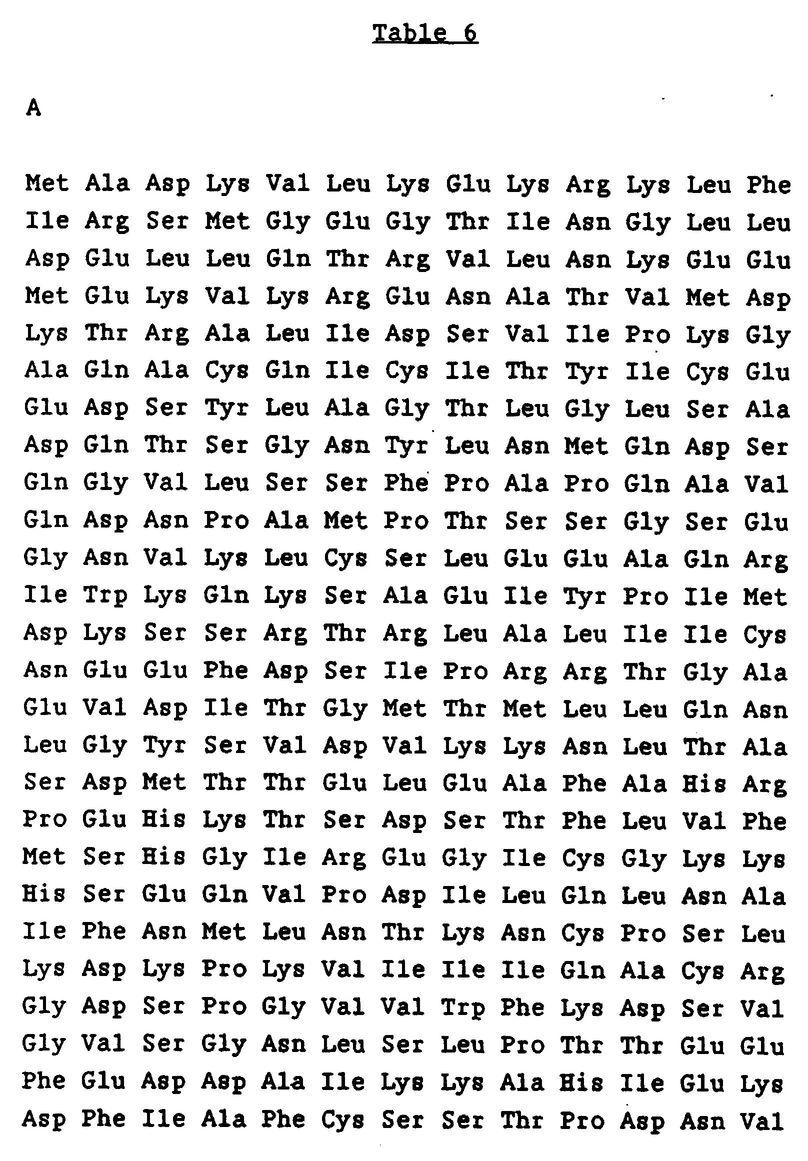

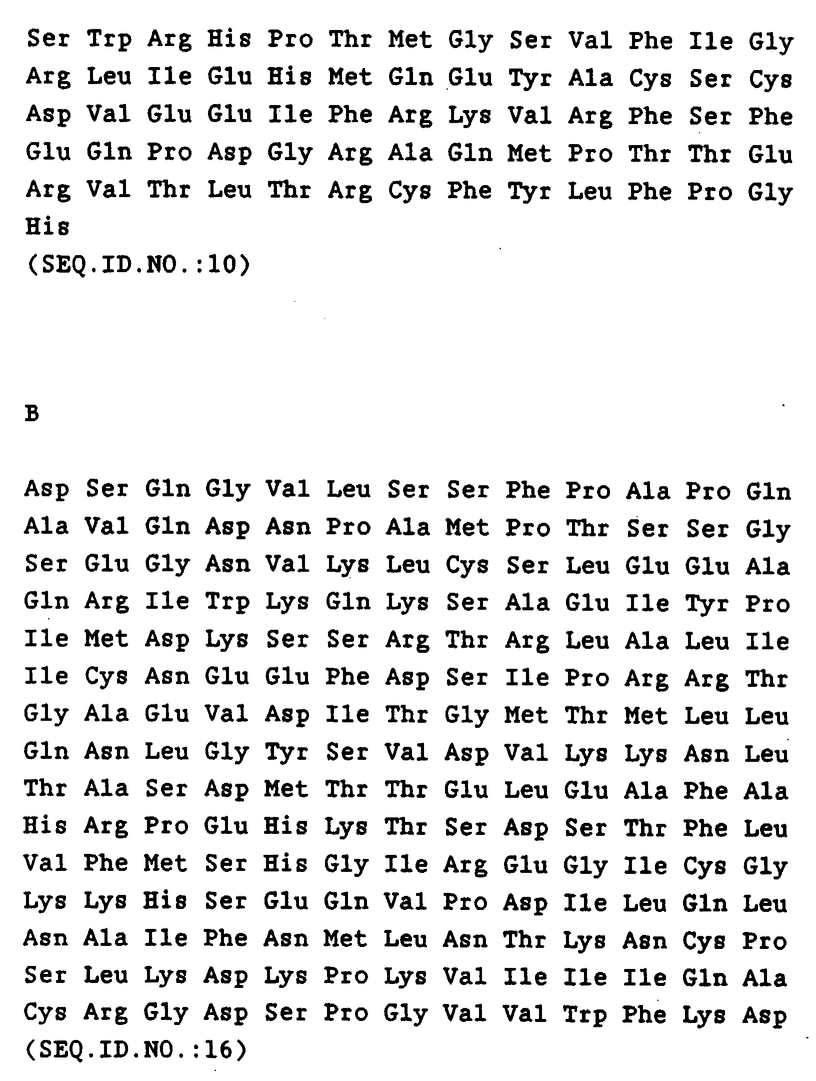

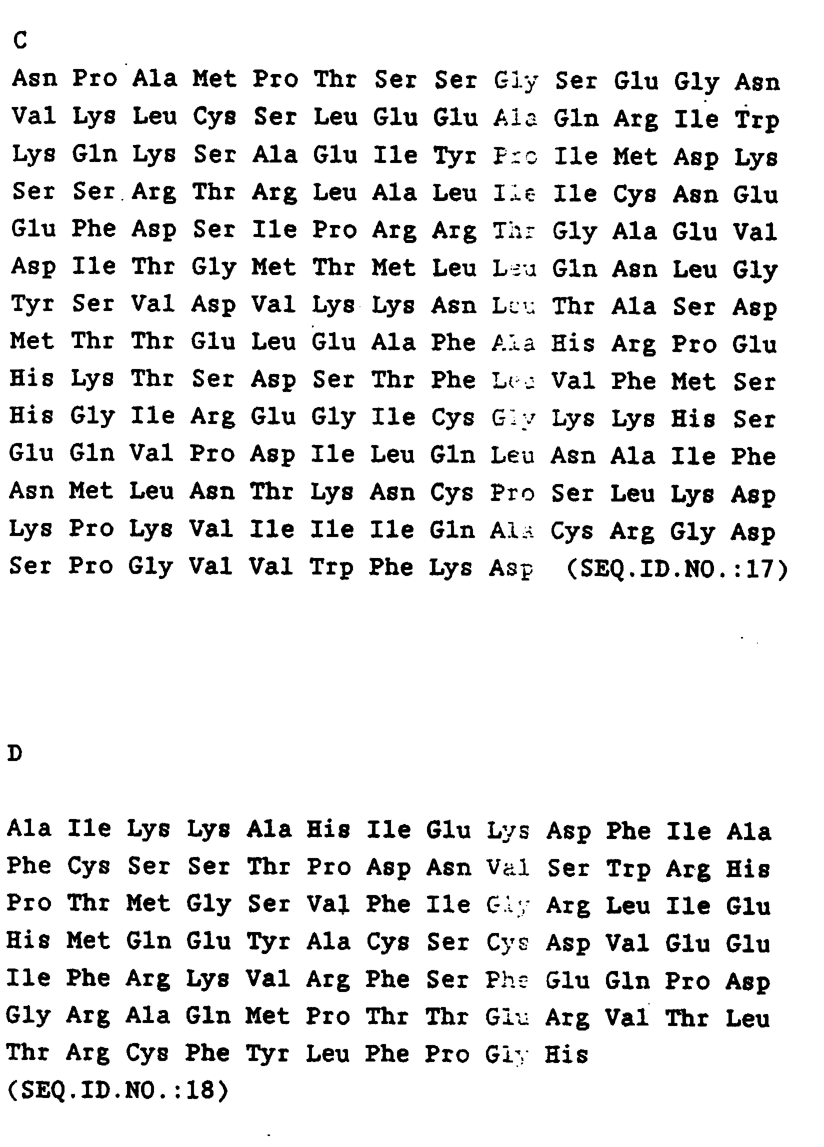

### EXAMPLE 26

### Cloning of the ICE cDNA into E. coli Expression Vectors

Recombinant ICE is produced in E. coli following the transfer of the ICE expression cassette into E. coli expression vectors, including but not limited to, the pET series (Novagen). The pET vectors place ICE expression under control of the tightly regulated bacteriophage T7 promoter. Following transfer of this construct into an E. coli host which contains a chromosomal copy of the T7 RNA polymerase gene driven by the inducible lac promoter, expression of ICE is induced when an approriate lac substrate (IPTG) is added to the culture. The levels of expressed ICE are determined by the assays described above.

The cDNA encoding the entire open reading frame for p45 was inserted into the NdeI site of pET 11a. Constructs in the positive orientation were identified by sequence analysis and used to transform the expression host strain BL21. Transformants were then used to inoculate cultures for the production of ICE protein. Cultures may be grown in M9 or ZB media, whose formulation is known to those skilled in the art. After growth to an OD₆₀₀= 1.5, expression of ICE was induced with 1 mM IPTG for 3 hours at 37°C. Authentic ICE enzymatic activity was found in the insoluble inclusion body fraction from these cells. Soluble ICE was extracted from the inclusion body fraction with 5 M guanidine-HCl in a buffer containing 50 mM Tris-HCl (pH 8) and 100 mM dithiothreitol. Active ICE was generated from this extract following dialysis against 100 volumes of 25 mM HEPES (pH 7.5), 5 mM dithiothreitol, 10% sucrose.

### EXAMPLE 27

### In Vitro Translation of ICE mRNA by Xenopus Oocyte Microinjection Vector and Expression in Mammalian Cells

ICE cDNA constructs are ligated into in vitro transcription vectors (the pGEM series, Promega) for the production of synthetic mRNAs.

Synthetic mRNA is produced in sufficient quantity in vitro by cloning double stranded DNA encoding ICE mRNA into a plasmid vector containing a bacteriophage promoter, linearizing the plasmid vector containing the cloned ICE-encoding DNA, and transcribing the cloned DNA in vitro using a DNA-dependent RNA polymerase from a bacteriophage that specifically recognizes the bacteriophage promoter on the plasmid vector.

Various plasmid vectors are available containing a bacteriophage promoter recognized by a bacteriophage DNA-dependent RNA polymerase, including but not limited to plasmids pSP64, pSP65, pSP70, pSP71, pSP72, pSP73, pGEM-3Z, pGEM-4Z, pGEM-3Zf, pGEM-5Zf, pGEM-7Zf, pGEM-9Zf, and pGEM-11Zf, the entire series of plasmids is commercially available from Promega.

The double stranded ICE-encoding DNA is cloned into the bacteriophage promoter containing vector in the proper orientation using one or more of the available restriction endonuclease cloning sites on the vector which are convenient and appropriate for cloning ICE DNA. The vector with the ligated ICE DNA is used to transform bacteria, and clonal isolates are analyzed for the presence of the vector with the ICE DNA in the proper orientation.

Once a vector containing the ICE-encoding DNA in the proper orientation is identified and isolated, it is linearized by cleavage with a restriction endonuclease at a site downstream from, and without disrupting, the ICE transcription unit. The linearized plasmid is isolated and purified, and used as a template for in vitro transcription of ICE mRNA.

The template DNA is then mixed with bacteriophage-specific DNA-dependent RNA polymerase in a reaction mixture which allows transcription of the DNA template forming ICE mRNA. Several bacteriophage-specific DNA-dependent RNA polymerases are available, including but not limited to T3, T7, and SP6 RNA polymerase. The synthetic ICE mRNA is then isolated and purified.

It may be advantageous to synthesize mRNA containing a 5' terminal cap structure and a 3' poly A tail to improve mRNA stability. A cap structure, or 7-methylguanosine, may be incorporated at the 5'terminus of the mRNA by simply adding 7-methylguanosine to the reaction mixture with the DNA template. The DNA-dependent RNA polymerase incorporates the cap structure at the 5' terminus as it synthesizes the mRNA. The poly A tail is found naturally occurring in many cDNA's but can be added to the 3' terminus of the mRNA by simply inserting a poly A tail-encoding DNA sequence at the 3' end of the DNA template.

The isolated and purified ICE mRNA is translated using either a cell-free system, including but not limited to rabbit reticulocyte lysate and wheat germ extracts (both commercially available from Promega and New England Nuclear) or in a cell based system, including but not limited to microinjection into Xenopus oocytes, with microinjection into Xenpus oocytes being preferred.

Xenopus oocytes are microinjected with a sufficient amount of synthetic ICE mRNA to produce ICE protein. The microinjected oocytes are incubated to allow translation of the ICE mRNA, forming ICE protein.

These synthetic mRNAs will be injected into Xenopus oocytes (stage 5 -6) by standard procedures [Gurdon, J.B. and Wickens, M.D. Methods in Enzymol. 101: 370-386, (1983)]. Oocytes will be harvested and analyzed for ICE expression as described below.

### EXAMPLE 28

### Cloning of ICE cDNA into a Mammalian Expression Vector

ICE cDNA expression cassettes are ligated at appropriate restriction endonuclease sites to the following vectors containing strong, universal mammalian promoters: pBC12BI [Cullen, B.R. Methods in Enzymol. 152: 684-704 1988], and pEE12 (CellTech EP 0 338,841) and its derivatives pSZ9016-1 and p9019. p9019 represents the construction of a mammalian expression vector containing the hCMVIE prm, polylinker and SV40 polyA element with a selectable marker/amplification system comprised of a mutant gene for dehydrofolate reductase (mDHFR) (Simonsen, C.C. and Levinson, A. D. Proc. Natl. Acad. Sci USA 80: 2495-2499 [1983]) driven by the SV40 early promoter. An SV40 polyadenylation sequence was generated by a PCR reaction defined by primers 13978-120 and 139778-121 using pD5 (Berker and Sharp, Nucl. Acid Res. 13: 841-857 [1985]) as template. The resulting 0.25 Kb PCR product was digested with ClaI and SpeI and ligated into the 6.7 Kb fragment of pEE12 which had been likewise digested. The resultant plasmid was designated p9018. p9018 was digested with BglII and SfiI to liberate the 3' portion of the SV40 early promoter and the GScDNA from the vector. A 0.73 Kb SfiI-XhoII fragment isolated from plasmid pFR400 (Simonsen, C.C. and Levinson, A. D. Proc. Natl. Acad. Sci USA 80: 2495-2499 [1983]) was ligated to the 5.6 Kb vector described above, reconstituting the SV40 early promoter, and inserting the mdHFR gene. This plasmid is designated p9019. pSZ9016-1 is identical to p9019 except for the substitution of the HIV LTR for the huCMVIE promoter. This vector was constructed by digesting p9019 with XbaI and MluI to remove the huCMVIE promoter. The HIV LTR promoter, from residue -117 to +80 (as found in the vector pCD23 containing the portion of the HIV-1 LTR (Cullen, Cell 46:973 [1986]) was PCR amplified from the plasmid pCD23 using oligonucleotide primers which appended to the ends of the product the MluI and SpeI restriction sites on the 5' side while Hind III and Xba I sites were appended on the 3' side. Following the digestion of the resulting 0.2 kb PCR product with the enzymes MluI and Xba I the fragment was agarose gel purified and ligated into the 4.3 Kb promoterless DNA fragment to generate the vector pSZ9016-1.

Cassettes containing the ICE cDNA in the positive orientation with respect to the promoter are ligated into appropriate restriction sites 3' of the promoter and identified by restriction site mapping and/or sequencing. These cDNA expression vectors are introduced into various fiboblastic host cells: [COS-7 (ATCC# CRL1651), CV-1 tat [Sackevitz et al., Science 238: 1575 (1987)], 293, L (ATCC#CRL6362)] by standard methods including but not limited to electroporation,or chemical procedures (cationic liposomes, DEAE dextran, calcium phosphate). Transfected cells and cell culture supernatants can be harvested and analyzed for ICE expression as described below.

All of the vectors used for mammalian transient expression can be used to establish stable cell lines expressing ICE. Unaltered ICE cDNA constructs cloned into expression vectors will be expected to program host cells to make intracellular ICE protein. In addition, ICE is expressed extracellularly as a secreted protein by ligating ICE cDNA constructs to DNA encoding the signal sequence of a secreted protein such as the human growth hormone or human lysozyme. The transfection host cells include, but are not limited to, CV-1-P [Sackevitz et al., Science 238: 1575 (1987)], tk-L [Wigler, et al. Cell 11: 223 (1977)], NS/0, and dHFr-CHO [Kaufman and Sharp, J. Mol. Biol. 159: 601, (1982)].

Co-transfection of any vector containing ICE cDNA with a drug selection plasmid (included, but not limited to G418, aminoglycoside phosphotransferase, pLNCX [Miller, A.D. and Rosman G. J. Biotech News 7: 980-990 (1989)]; hygromycin, hygromycin-B phospholransferase, pLG90 [Gritz. L. and Davies, J., GENE 25: 179 (1983)] ; APRT, xanthine-guanine phosphoribosyltransferase,pMAM (Clontech) [Murray, et al., Gene 31: 233 (1984)] will allow for the selection of stably transfected clones. Levels of ICE are quantitated by the assays described above.

ICE cDNA constructs are ligated into vectors containing amplifiable drug-resistance markers for the production of mammalian cell clones synthesizing the highest possible levels of ICE. Following introduction of these constructs into cells, clones containing the plasmid are selected with the appropriate agent, and isolation of an overexpressing clone with a high copy number of the plasmid is accomplished by selection in increasing doses of the agent. The following systems are utilized: the 9016 or the 9019 plasmid containing the mutant DHFR gene [Simonson, C. and Levinson, A., Proc. Natl. Acad. Sci. USA 80: 2495 (1983)], transfected into dHFR- CHO cells and selected in methotrexate; the pEE12 plasmid containing the glutamine synthetase gene, transfected into NS/O cells and selected in methionine sulfoximine (CellTech International Patent Application 2089/10404); and 9016 or other CMV prm vectors, co-transfected with pDLAT-3 containing the thymidine kinase gene [Colbere and Garopin, F., Proc. Natl. Acad. Sci. 76: 3755 (1979)] in APRT and TK deficient L cells, selected in APRT (0.05 mM azaserine, 0.1 mM adenine, 4 ug/ml adenosine) and amplified with HAT (100 uM hypoxanthine, 0.4 uM aminopterin, 16 uM thymidine).

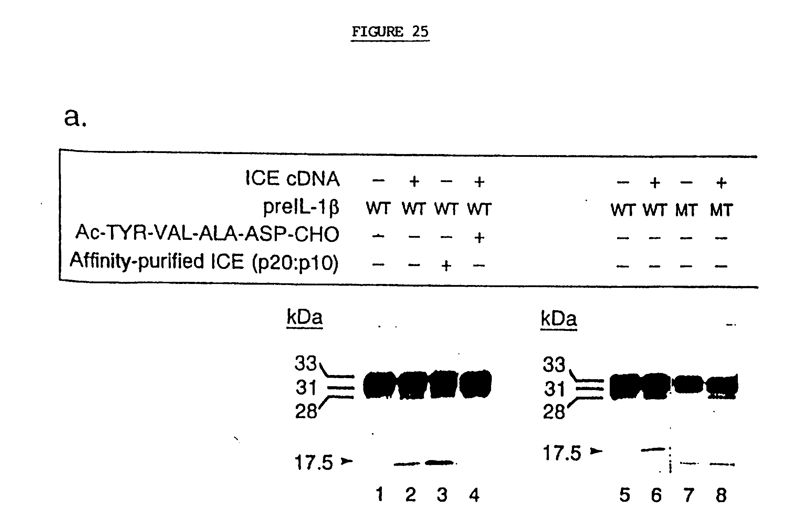

The expression of recombinant ICE was achieved by transfection of full-length ICE cDNA, including the complete ORF of the 45 kDa ICE preprotein (Fig. 23), into a mammalian host cell. The 1.6 kb EcoRI fragment containing the full length ICE cDNA was cloned into vector pSZ-9016. 6µg of this DNA along with 0.6 ug of pX8TAT, a mammalian expression vector which places the trans-activating protein (TAT) of HIV under the control of the SV40 early promoter, was transfected into COS-7 cells by the cationic-liposome method. Cells were harvested 48 hours later and lysed in detergent buffer (25 mM HEPES pH 7, 1% Triton-X-100, 1mM EDTA, 2mM DTT, 10 ug/ml aprotinin, 10 ug/ml leupeptin, 10 ug/ml pepstatin, and 2 mM PMSF). Cell lysates were incubated with radiolabeled IL-1β precursor to measure ICE activity. Cleavage products of IL-1β were analyzed by immunoprecipitation with IL-1B antibody and fractionation on SDS polyacrylamide gels. The IL-1β precursor was cleaved to the mature, 17 kDa form by cells transfected with ICE cDNA, but not by cells transfected with the expression plasmid alone. The cleavage product comigrated with mature IL-1β produced by incubation of substrate with affinity purified ICE and was completely inhibited by the specific ICE inhibitor (L-709,049). The substrate specificity of the expressed ICE activity was verified by incubating lysates with an IL-1β precursor cleavage site mutant (Ala¹¹⁶) which cannot be cleaved by ICE. As with native ICE, the activity from the transfectants cleaves the mutant protein to a 28 kDa product but not to the 17 kDa form (Fig. 25).

Recombinant ICE activity was not observed upon transient transfection of individual constructs expressing the p20 or p10 subunits, both singly or in combination. These results suggest that p20 and p10 when expressed in this manner may not be folded properly and thus the active conformation of ICE may only be generated upon proteolysis of the p45 proenzyme. In addition, mutation of the catalytic Cys (residue 285) to Ala abolishes ICE activity while deletion of the predomain (p14) demonstrates that it is not required for ICE activity.

### EXAMPLE 29

### Cloning of ICE cDNA into a Baculovirus Expression Vector for Expression in Insect Cells

Baculovirus vectors, which are derived from the genome of the AcNPV virus, are designed to provide high level expression of cDNA in the Sf9 line of insect cells (ATCC CRL#1711). Recombinant baculoviruses expressing ICE cDNA is produced by the following standard methods (InVitrogen Maxbac Manual): the ICE cDNA constructs are ligated into the polyhedrin gene in a variety of baculovirus transfer vectors, including the pAC360 and the BlueBac vector (InVitrogen). Recombinant baculoviruses are generated by homologous recombination following co-transfection of the baculovirus transfer vector and linearized AcNPV genomic DNA [Kitts, P.A., Nuc. Acid. Res. 18: 5667 (1990)] into Sf9 cells. Recombinant pAC360 viruses are identified by the absence of inclusion bodies in infected cells and recombinant pBlueBac viruses are identified on the basis of B-galactosidase expression (Summers, M. D. and Smith, G. E., Texas Agriculture Exp. Station Bulletin No. 1555)). Following plaque purification, ICE expression is measured by the assays described above.

The cDNA encoding the entire open reading frame for p45 was inserted into the BamHI site of pBlueBacII. Constructs in the positive orientation were identified by sequence analysis and used to transfect Sf9 cells in the presence of linear AcNPV wild type DNA.

Authentic, enzymatically-active ICE was found in the cytoplasm of infected cells. Active ICE was extracted from infected cells under native conditions by hypotonic or detergent lysis.

### EXAMPLE 30

### Cloning of ICE cDNA into a yeast expression vector

Recombinant ICE is produced in the yeast S. cerevisiae following the insertion of the optimal ICE cDNA cistron into expression vectors designed to direct the intracellular or extracellular expression of heterologous proteins. In the case of intracellular expression, vectors such as EmBLyex4 or the like are ligated to the ICE cistron [Rinas, U. et al., Biotechnology 8: 543-545 (1990); Horowitz B. et al., J. Biol. Chem. 265: 4189-4192 (1989)]. For extracellular expression, the ICE cistron is ligated into yeast expression vectors which fuse a secretion signal (a yeast or mammalian peptide) to the NH2 terminus of the ICE protein [Jacobson, M. A., Gene 85: 511-516 (1989); Riett L. and Bellon N. Biochem. 28: 2941-2949 (1989)].

These vectors include, but are not limited to pAVE1>6, which fuses the human serum albumin signal to the expressed cDNA[Steep 0. Biotechnology 8: 42-46 (1990)], and the vector pL8PL which fuses the human lysozyme signal to the expressed cDNA [Yamamoto, Y., Biochem. 28: 2728-2732)]. In addition, ICE is expressed in yeast as a fusion protein conjugated to ubiquitin utilizing the vector pVEP [Ecker, D. J., J. Biol. Chem. 264: 7715-7719 (1989), Sabin, E. A., Biotechnology 7: 705-709 (1989), McDonnell D. P., Mol. Cell Biol. 9: 5517-5523 (1989)]. The levels of expressed ICE are determined by the assays described above.

### EXAMPLE 31

### Purification of Recombinant ICE

Recombinantly produced ICE may be purified by any one or combination of purification procedures of Examples 1-3, and 5-7. In addition, recombinantly produced 20 kDa subunit, 10kDa subunit and nascent full-length ICE polypeptides may be individually purified using the appropriate monospecific antibodies.

## Claims

1. A DNA molecule which encodes the complete unmodified form of precursor interleukin 1 beta converting enzyme, having the nucleotide sequence:

2. A DNA molecule which encodes the 22 kDa protein of precursor interleukin 1 beta converting enzyme, having the nucleotide sequence:

3. A DNA molecule which encodes the 20 kDa subunit of precursor interleukin 1 beta converting enzyme, having the nucleotide sequence:

4. A DNA molecule which encodes the 10 kDa subunit of precursor interleukin 1 beta converting, having the nucleotide sequence:

5. An expression vector for the expression of cloned genes in a recombinant host, the expression vector containing one or more cloned genes with a nucleotide sequence selected from the group consisting of:
SEQ. ID. NO.: 9, or SEQ. ID. NO.:13, or SEQ.ID.NO.: 14, or SEQ. ID. NO.:15.

6. A recombinant host cell containing one or more cloned genes, the cloned genes having a nucleotide sequence selected from the group consisting of:
SEQ. ID. NO.: 9, or SEQ. ID. NO.:13, or SEQ.ID.NO.: 14, or SEQ. ID. NO.:15.

7. A recombinant host cell expressing recombinant interleukin 1 beta and containing one or more genes selected from the group consisting of: SEQ.ID.NO.: 9, or SEQ.ID.NO.:13, or SEQ.ID.NO.:14, or SEQ.ID.NO.:15.

8. A protein, in substantially pure form which can specifically cleave precursor IL-1 beta to form mature IL-1 beta, having an amino acid sequence:

9. A protein in substantially pure form which corresponds to the 22 kDa subunit of precursor interleukin 1 beta converting enzyme, having the amino acid sequence:

10. A protein, in substantially pure form which corresponds to the 20 kDa subunit of presursor interleukin 1 beta converting enzyme, having an amino acid sequence: wherein R is Asp or Asn.

11. A protein, in pure form, which corresponds to the 10 kDa subunit of precursor interleukin 1 beta converting enzyme, having an amino acid sequence:

12. A composite protein molecule, in pure form, having a specific activity of 4.7 x 10⁶ units/mg which can specifically cleave precursor interleukin 1 to form mature interleukin 1 beta, comprising:
(a) a polypeptide having the amino acid sequence SEQ.ID.NO.:17
and
(b) a polypeptide having the amino acid sequence SEQ. ID. NO.:18

13. A composite protein molecule, in pure form, having a specific activity of 1·67 x 10⁶ units/mg which can specifically cleave precursor interleukin 1 beta to form mature interleukin 1 beta, comprising:
(a) a polypeptide having the amino acid sequence SEQ.ID.NO.:16,
(b) a polypeptide having the amino acid sequence SEQ.ID.NO.:18.

14. A monospecific antibody immunologically reactive with monomeric precursor interleukin 1 beta converting enzyme, the enzyme having the amino acid sequence SEQ. ID. NO.: 10.

15. A monospecific antibody immunologically reactive with the 20 kDa subunit of precursor interleukin 1 beta converting enzyme, the 20 kDa subunit having the amino acid sequence SEQ.ID.NO.: 17

16. A monospecific antibody immunologically reactive with the 10 kDa subunit of precursor interleukin 1 beta converting enzyme, 10 kDa subunit having the amino acid sequence SEQ.ID.NO.:18.

17. A recombinant microorganism as deposited at the ATCC under the accession number ATCC 68655.

## Patentansprüche

1. DNA-Molekül, welches für die vollständige, unmodifizierte Form von Vorläufer-Interleukin-1β-konversionsenzym kodiert, mit der Nukleotidsequenz:

2. DNA-Molekül, welches für das 22-kD-Protein von Vorläufer-Interleukin-1β-Konversionsenzym kodiert, mit der Nukleotidsequenz:

3. DNA-Molekül, welches für die 20-kD-Untereinheit von Vorläufer-Interleukin-1β-Konversionsenzym kodiert, mit der Nukleotidsequenz:

4. DNA-Molekül, welches für die 10-kD-Untereinheit von Vorläufer-lnterleukin-1β-Konversionsenzym kodiert, mit der Nukleotidsequenz:

5. Expressionsvektor zur Expression klonierter Gene in einem rekombinanten Wirt, wobei der Expressionsvektor ein oder mehrere klonierte Gene mit einer Nukleotidsequenz, die aus der Gruppe, bestehend aus SEQ-ID-Nr. 9 oder SEQ-ID-Nr. 13 oder SEQ-ID-Nr. 14 oder SEQ-ID-Nr. 15, ausgewählt ist, enthält.

6. Rekombinante Wirtszelle, die ein oder mehrere klonierte Gene enthält, wobei die klonierten Gene eine Nukleotidsequenz aufweisen, die aus der Gruppe, bestehend aus SEQ-ID-Nr. 9 oder SEQ-ID-Nr. 13 oder SEQ-ID-Nr. 14 oder SEQ-ID-Nr. 15, ausgewählt ist.

7. Rekombinante Wirtszelle, die rekombinantes Interleukin-1β exprimiert und ein oder mehrere Gene enthält, das oder die aus der Gruppe, bestehend aus SEQ-ID-Nr. 9 oder SEQ-ID-Nr. 13 oder SEQ-ID-Nr. 14 oder SEQ-ID-Nr. 15, ausgewählt ist bzw. sind.

8. Protein in im wesentlichen reiner Form, welches Vorläufer-IL-1β spezifisch spalten kann, um reifes IL-1β zu bilden, mit einer Aminosäuresequenz:

9. Protein in im wesentlichen reiner Form, welches der 22-kD-Untereinheit von Vorläufer-Interleukin-1β-Konversionsenzym entspricht, mit der Aminosäuresequenz:

10. Protein in im wesentlichen reiner Form, welches der 20-kD-Untereinheit von Vorläufer-Interleukin-1β-Konversionsenzym entspricht, mit einer Aminosäuresequenz: worin R Asp oder Asn ist.

11. Protein in reiner Form, welches der 10-kD-Untereinheit von Vorläufer-lnterleukin-1β-Konversionsenzym entspricht, mit einer Aminosäuresequenz:

12. Zusammengesetztes Proteinmolekül in reiner Form mit einer spezifischen Aktivität von 4,7 x 10⁶ Einheiten/mg, welches Vorläufer-lnterleukin-1β spezifisch spalten kann, um reifes Interleukin-1β zu bilden, umfassend:
(a) ein Polypeptid mit der Aminosäuresequenz SEQ-ID-Nr. 17 und
(b) ein Polypeptid mit der Aminosäuresequenz SEQ-ID-Nr. 18.

13. Zusammengesetztes Proteinmolekül in reiner Form mit einer spezifischen Aktivität von 1,67 x 10⁶ Einheiten/mg, welches Vorläufer-lnterleukin-1β spezifisch spalten kann, um reifes Interleukin-1β zu bilden, umfassend:
(a) ein Polypeptid mit der Aminosäuresequenz SEQ-ID-Nr. 16
(b) ein Polypeptid mit der Aminosäuresequenz SEQ-ID-Nr. 18.

14. Monospezifischer Antikörper, der zu einer immunologischen Reaktion mit monomerem Vorläufer-Interleukin-1β-Konversionsenzym imstande ist, wobei das Enzym die Aminosäuresequenz SEQ-ID-Nr. 10 aufweist.

15. Monospezifischer Antikörper, der zu einer immunologischen Reaktion mit der 20-kD-Untereinheit von Vorläufer-Interleukin-1β-Konversionsenzym imstande ist, wobei die 20-kD-Untereinheit die Aminosäuresequenz SEQ-ID-Nr. 17 aufweist.

16. Monospezifischer Antikörper, der zu einer immunologischen Reaktion mit der 10-kD-Untereinheit von Vorläufer-Interleukin-1β-Konversionsenzym imstande ist, wobei die 10-kD-Untereinheit die Aminosäuresequenz SEQ-ID-Nr. 18 aufweist.

17. Rekombinanter Mikroorganismus wie bei der ATCC unter der Hinterlegungsnummer ATCC 68655 hinterlegt.

## Revendications

1. Molécule d'ADN qui code la forme non modifiée complète de l'enzyme de conversion de l'interleukine-1β précurseur, ayant la séquence nucléotidique suivante :

2. Molécule d'ADN qui code la protéine de 22 kDa de l'enzyme de conversion de l'interleukine-1β précurseur, ayant la séquence nucléotidique suivante :

3. Molécule d'ADN qui code la sous-unité de 20 kDa de l'enzyme de conversion de l'interleukine-1β précurseur, qui a la séquence nucléotidique suivante :

4. Molécule d'ADN qui code la sous-unité de 10 kDa de l'enzyme de conversion de l'interleukine-1β précurseur, qui a la séquence nucléotidique suivante :

5. Vecteur d'expression pour l'expression de gènes clonés dans un hôte recombinant, le vecteur d'expression contenant un ou plusieurs gènes clonés ayant un séquence nucléotidique choisie dans le groupe comprenant les séquences SEQ.ID.NO.: 9 ou SEQ.ID.NO.: 13 ou SEQ.ID.NO.: 14 ou SEQ.ID.NO.: 15.

6. Cellule hôte recombinante, contenant un ou plusieurs gènes clonés, les gènes clonés ayant une séquence nucléotidique choisie dans le groupe comprenant les séquences SEQ.ID.NO.: 9 ou SEQ.ID.NO.: 13 ou SEQ.ID.NO.: 14 ou SEQ.ID.NO.: 15.

7. Cellule hôte recombinante exprimant l'interleukine-1β recombinante et contenant un ou plusieurs gènes sélectionnés dans le groupe comprenant les séquences SEQ.ID.NO.: 9 ou SEQ.ID.NO.: 13 ou SEQ.ID.NO.: 14 ou SEQ.ID.NO.: 15.

8. Protéine, sous forme essentiellement pure, qui peut d'une manière spécifique cliver l'IL-1β précurseur pour former l'IL-1β mature, et qui a la séquence d'acides aminés suivante :

9. Protéine sous une forme essentiellement pure, qui correspond à la sous-unité de 22 kDa de l'enzyme de conversion de l'interleukine-1β précurseur, qui a la séquence d'acides aminés suivante :

10. Protéine, sous une forme essentiellement pure, qui correspond à la sous-unité de 20 kDa de l'enzyme de conversion de l'interleukine-1β précurseur, qui a la séquence d'acides aminés suivante : où R est Asp ou Asn.

11. Protéine, sous forme pure, qui correspond à la sous-unité de 10 kDa de l'enzyme de conversion de l'interleukine-1β précurseur, qui a la séquence d'acides aminés suivante :

12. Molécule de protéine composite, sous forme pure, ayant une activité spécifique de 4,7 x 10⁶ unités/mg, qui peut d'une manière spécifique cliver l'interleukine-1 précurseur pour former l'interleukine-1β mature, comprenant :
(a) un polypeptide ayant la séquence d'acides aminés SEQ.ID.NO.: 17, et
(b) un polypeptide ayant la séquence d'acides aminés SEQ.ID.NO.: 18.

13. Molécule de protéine composite, sous forme pure, ayant une activité spécifique de 1,67 x 10⁶ unités/mg, qui peut d'une manière spécifique cliver l'interleukine-1β précurseur pour former l'interleukine-1β mature, et comprenant :
(a) un polypeptide ayant la séquence d'acides aminés SEQ.ID.NO.: 16, et
(b) un polypeptide ayant la séquence d'acides aminés SEQ.ID.NO.: 18.

14. Anticorps monospécifique, qui réagit d'un point de vue immunologique avec l'enzyme de conversion de l'interleukine-1β précurseur monomère, l'enzyme ayant la séquence d'acides aminés SEQ.ID.NO.: 10.

15. Anticorps monospécifique, réactif d'un point de vue immunologique avec la sous-unité de 20 kDa de l'enzyme de conversion de l'interleukine-1β précurseur, la sous-unité de 20 kDa ayant la séquence d'acides aminés SEQ.ID.NO.: 17.

16. Anticorps monospécifique, réactif d'un point de vue immunologique avec la sous-unité de 10 kDa de l'enzyme de conversion de l'interleukine-1β précurseur, la sous-unité de 10 kDa ayant la séquence d'acides aminés SEQ.ID.NO.: 18.

17. Microorganisme recombinant, tel que déposé auprès de l'ATCC sous le numéro d'enregistrement ATCC 68655.


### SEQUENCE LISTING

### (1) GENERAL INFORMATION:

(i) APPLICANT: Howard, Andrew D
   Molineaux, Susan M
   Tocci, Michael J
   Calaycay, Jimmy
   Miller, Douglas K.
(ii) TITLE OF INVENTION: COMPLIMENTARY DNA ENCODING PRECURSOR INTERLEUKIN 1B CONVERTING ENZYME
(iii) NUMBER OF SEQUENCES: 19
(iv) CORRESPONDENCE ADDRESS:
   (A) ADDRESSEE: Merck & Co., Inc.
   (B) STREET: P.O. Box 2000, 126 E. Lincoln Avenue
   (C) CITY: Rahway
   (D) STATE: New Jersey
   (E) COUNTRY: USA
   (F) ZIP: 07065
(v) COMPUTER READABLE FORM:
   (A) MEDIUM TYPE: Floppy disk
   (B) COMPUTER: IBM PC compatible
   (C) OPERATING SYSTEM: PC-DOS/MS-DOS
   (D) SOFTWARE: PatentIn Release #1.0, Version #1.25
(vi) CURRENT APPLICATION DATA:
   (A) APPLICATION NUMBER:
   (B) FILING DATE:
   (C) CLASSIFICATION:
(vii) PRIOR APPLICATION DATA:
   (A) APPLICATION NUMBER: 746,454
   (B) FILING DATE: 16 Aug 1991
(viii) ATTORNEY/AGENT INFORMATION:
   (A) NAME: Wallen, John W. III
   (B) REGISTRATION NUMBER: 35403
   (C) REFERENCE/DOCKET NUMBER: 18498IC
(ix) TELECOMMUNICATION INFORMATION:
   (A) TELEPHONE: (908) 594-3905
   (C) TELEX: (908) 594-4720

### (2) INFORMATION FOR SEQ ID NO:1:

(i) SEQUENCE CHARACTERISTICS:
   (A) LENGTH: 14 amino acids
   (B) TYPE: amino acid
   (C) STRANDEDNESS: single
   (D) TOPOLOGY: linear
(ii) MOLECULE TYPE: peptide
   (xi) SEQUENCE DESCRIPTION: SEQ ID NO:1:
   Asp Pro Ala Met Pro Tyr Ser Ser Gly Ser Glu Gly Asn Val
   1               5                   10

### (2) INFORMATION FOR SEQ ID NO:2:

(i) SEQUENCE CHARACTERISTICS:
   (A) LENGTH: 17 amino acids
   (B) TYPE: amino acid
   (C) STRANDEDNESS: single
   (D) TOPOLOGY: linear
(ii) MOLECULE TYPE: peptide
   (xi) SEQUENCE DESCRIPTION: SEQ ID NO:2:
   Ala Ile Lys Lys Ala His Ile Glu Lys Asp Phe Ile Ala Phe Cys Ser
   1               5                   10                  15
   Ser

### (2) INFORMATION FOR SEQ ID NO:3:

(i) SEQUENCE CHARACTERISTICS:
   (A) LENGTH: 17 base pairs
   (B) TYPE: nucleic acid
   (C) STRANDEDNESS: single
   (D) TOPOLOGY: linear
(ii) MOLECULE TYPE: cDNA
(xi) SEQUENCE DESCRIPTION: SEQ ID NO:3:
   GAYCCNGCNA TGCCNAC                                                  17

### (2) INFORMATION FOR SEQ ID NO:4:

(i) SEQUENCE CHARACTERISTICS:
   (A) LENGTH: 17 base pairs
   (B) TYPE: nucleic acid
   (C) STRANDEDNESS: single
   (D) TOPOLOGY: linear
(ii) MOLECULE TYPE: CDNA
(xi) SEQUENCE DESCRIPTION: SEQ ID NO:4:
   ATRGGNTADT ACCTRTT 17

### (2) INFORMATION FOR SEQ ID NO:5:

(i) SEQUENCE CHARACTERISTICS:
   (A) LENGTH: 17 base pairs
   (B) TYPE: nucleic acid
   (C) STRANDEDNESS: single
   (D) TOPOLOGY: linear
(ii) MOLECULE TYPE: cDNA
(xi) SEQUENCE DESCRIPTION: SEQ ID NO:5:
   GCNATHAARA ARGCNCA 17

### (2) INFORMATION FOR SEQ ID NO:6:

(i) SEQUENCE CHARACTERISTICS:
   (A) LENGTH: 17 base pairs
   (B) TYPE: nucleic acid
   (C) STRANDEDNESS: single
   (D) TOPOLOGY: linear
(ii) MOLECULE TYPE: cDNA
(xi) SEQUENCE DESCRIPTION: SEQ ID NO:6:
   GTYTACGGNT GNTGNCT 17

### (2) INFORMATION FOR SEQ ID NO:7:

(i) SEQUENCE CHARACTERISTICS:
   (A) LENGTH: 18 base pairs
   (B) TYPE: nucleic acid
   (C) STRANDEDNESS: single
   (D) TOPOLOGY: linear
(ii) MOLECULE TYPE: cDNA
(xi) SEQUENCE DESCRIPTION: SEQ ID NO:7:
   ATIGGRTAIA TYTCIGCR 18

### (2) INFORMATION FOR SEQ ID NO:8:

(i) SEQUENCE CHARACTERISTICS:
   (A) LENGTH: 20 base pairs
   (B) TYPE: nucleic acid
   (C) STRANDEDNESS: single
   (D) TOPOLOGY: linear
(ii) MOLECULE TYPE: cDNA
(xi) SEQUENCE DESCRIPTION: SEQ ID NO:8:
   ATNGARAARG AYTTYATNGC 20

### (2) INFORMATION FOR SEQ ID NO:9:

(i) SEQUENCE CHARACTERISTICS:
   (A) LENGTH: 1490 base pairs
   (B) TYPE: nucleic acid
   (C) STRANDEDNESS: single
   (D) TOPOLOGY: linear
(ii) MOLECULE TYPE: cDNA
(xi) SEQUENCE DESCRIPTION: SEQ ID NO:9:
   CGGCCATGGC CGACAAGGTC CTGAAGGAGA AGAGAAAGCT GTTTATCCGT TCCATGGGTG
   AAGGTACAAT AAATGGCTTA CTGGATGAAT TATTACAGAC AAGGGTGCTG AACAAGGAAG
   AGATGGAGAA AGTAAAACGT GAAAATGCTA CAGTTATGGA TAAGACCCGA GCTTTGATTG
   ACTCCGTTAT TCCGAAAGGG GCACAGGCAT GCCAAATTTG CATCACATAC ATTTGTGAAG
   AAGACAGTTA CCTGGCAGGG ACGCTGGGAC TCTCAGCAGA TCAAACATCT GGAAATTACC
   TTAATATGCA AGACTCTCAA GGAGTACTTT CTTCCTTTCC AGCTCCTCAG GCAGTGCAGG
   ACAACCCAGC TATGCCCACA TCCTCAGGCT CAGAAGGGAA TGTCAAGCTT TGCTCCCTAG
   AAGAAGCTCA AAGGATATGG AAACAAAAGT CGGCAGAGAT TTATCCAATA ATGGACAAGT
   CAAGCCGCAC ACGTCTTGCT CTCATTATCT GCAATGAAGA ATTTGACAGT ATTCCTAGAA
   GAACTGGAGC TGAGGTTGAC ATCACAGGCA TGACAATGCT GCTACAAAAT CTGGGGTACA
   GCGTAGATGT GAAAAAAAAT CTCACTGCTT CGGACATGAC TACAGAGCTG GAGGCATTTG
   CACACCGCCC AGAGCACAAG ACCTCTGACA GCACGTTCCT GGTGTTCATG TCTCATGGTA
   TTCGGGAAGG CATTTGTGGG AAGAAACACT CTGAGCAAGT CCCAGATATA CTACAACTCA
   ATGCAATCTT TAACATGTTG AATACCAAGA ACTGCCCAAG TTTGAAGGAC AAACCGAAGG
   TGATCATCAT CCAGGCCTGC CGTGGTGACA GCCCTGGTGT GGTGTGGTTT AAAGATTCAG
   TAGGAGTTTC TGGAAACCTA TCTTTACCAA CTACAGAAGA GTTTGAGGAT GATGCTATTA
   AGAAAGCCCA CATAGAGAAG GATTTTATCG CTTTCTGCTC TTCCACACCA GATAATGTTT
   CTTGGAGACA TCCCACAATG GGCTCTGTTT TTATTGGAAG ACTCATTGAA CATATGCAAG
   AATATGCCTG TTCCTGTGAT GTGGAGGAAA TTTTCCGCAA GGTTCGATTT TCATTTGAGC
   AGCCAGATGG TAGAGCGCAG ATGCCCACCA CTGAAAGAGT GACTTTGACA AGATGTTTCT
   ACCTCTTCCC AGGACATTAA AATAAGGAAA CTGTATGAAT GTCTGTGGGC AGGTACATGT
   GTATGGTCGG GAGTGTGGGA AGGTTGAGGA AAGGGTACTG AAAGTCCATT TGAGTCAAGA
   ACTCTAGGTT TACAGGCTGA GAATCCTTAA TCCAAAAAAT TCGAATTTTG AAATGCTCTA
   AAATCCAACA CTGTGTGAGC GCCCACATGA TATTCAAAGG AAATGTTTAT TGAAACATTT
   CAAATTATAG GTTTTTGGAT TAGGGATGCT AAACCAGTAA GTATACAGCT

### (2) INFORMATION FOR SEQ ID NO:10:

(i) SEQUENCE CHARACTERISTICS:
   (A) LENGTH: 404 amino acids
   (B) TYPE: amino acid
   (C) STRANDEDNESS: single
   (D) TOPOLOGY: linear
(ii) MOLECULE TYPE: protein
(xi) SEQUENCE DESCRIPTION: SEQ ID NO:10:
   Met Ala Asp Lys Val Leu Lys Glu Lys Arg Lys Leu Phe Ile Arg Ser
   1               5                   10                  15
   Met Gly Glu Gly Thr Ile Asn Gly Leu Leu Asp Glu Leu Leu Gln Thr
               20                  25                  30
   Arg Val Leu Asn Lys Glu Glu Met Glu Lys Val Lys Arg Glu Asn Ala
           35                  40                  45
   Thr Val Met Asp Lys Thr Arg Ala Leu Ile Asp Ser Val Ile Pro Lys
       50                  55                  60
   Gly Ala Gln Ala Cys Gln Ile Cys Ile Thr Tyr Ile Cys Glu Glu Asp
   65                  70                  75                  80
   Ser Tyr Leu Ala Gly Thr Leu Gly Leu Ser Ala Asp Gln Thr Ser Gly
                   85                  90                  95
   Asn Tyr Leu Asn Met Gln Asp Ser Gln Gly Val Leu Ser Ser Phe Pro
               100                 105                 110
   Ala Pro Gln Ala Val Gln Asp Asn Pro Ala Met Pro Thr Ser Ser Gly
           115                 120                 125
   Ser Glu Gly Asn Val Lys Leu Cys Ser Leu Glu Glu Ala Gln Arg Ile
       130                 135                 140
   Trp Lys Gln Lys Ser Ala Glu Ile Tyr Pro Ile Met Asp Lys Ser Ser
   145                 150                 155                 160
   Arg Thr Arg Leu Ala Leu Ile Ile Cys Asn Glu Glu Phe Asp Ser Ile
   165 170 175
   Pro Arg Arg Thr Gly Ala Glu Val Asp Ile Thr Gly Met Thr Met Leu
   180 185 190
   Leu Gln Asn Leu Gly Tyr Ser Val Asp Val Lys Lys Asn Leu Thr Ala
   195 200 205
   Ser Asp Met Thr Thr Glu Leu Glu Ala Phe Ala His Arg Pro Glu His
   210 215 220
   Lys Thr Ser Asp Ser Thr Phe Leu Val Phe Met Ser His Gly Ile Arg
   225 230 235 240
   Glu Gly Ile Cys Gly Lys Lys His Ser Glu Gln Val Pro Asp Ile Leu
   245 250 255
   Gln Leu Asn Ala Ile Phe Asn Met Leu Asn Thr Lys Asn Cys Pro Ser
   260 265 270
   Leu Lys Asp Lys Pro Lys Val Ile Ile Ile Gln Ala Cys Arg Gly Asp
   275 280 285
   Ser Pro Gly Val Val Trp Phe Lys Asp Ser Val Gly Val Ser Gly Asn
   290 295 300
   Leu Ser Leu Pro Thr Thr Glu Glu Phe Glu Asp Asp Ala Ile Lys Lys
   305 310 315 320
   Ala His Ile Glu Lys Asp Phe Ile Ala Phe Cys Ser Ser Thr Pro Asp
   325 330 335
   Asn Val Ser Trp Arg His Pro Thr Met Gly Ser Val Phe Ile Gly Arg
   340 345 350
   Leu Ile Glu His Met Gln Glu Tyr Ala Cys Ser Cys Asp Val Glu Glu
   355 360 365
   Ile Phe Arg Lys Val Arg Phe Ser Phe Glu Gln Pro Asp Gly Arg Ala
   370 375 380
   Gln Met Pro Thr Thr Glu Arg Val Thr Leu Thr Arg Cys Phe Tyr Leu
   385 390 395 400
   Phe Pro Gly His

### (2) INFORMATION FOR SEQ ID NO:11:

(i) SEQUENCE CHARACTERISTICS:
   (A) LENGTH: 39 amino acids
   (B) TYPE: amino acid
   (C) STRANDEDNESS: single
   (D) TOPOLOGY: linear
(ii) MOLECULE TYPE: peptide
(xi) SEQUENCE DESCRIPTION: SEQ ID NO:11:
   Asp Pro Ala Met Pro Thr Ser Ser Gly Ser Glu Gly Asn Val Lys Leu
   1               5                   10                  15
   Cys Ser Leu Glu Glu Ala Gln Arg Ile Trp Lys Gln Lys Ser Ala Glu
               20                  25                  30
   Ile Tyr Pro Ile Met Asp Lys
           35

### (2) INFORMATION FOR SEQ ID NO:12:

(i) SEQUENCE CHARACTERISTICS:
   (A) LENGTH: 74 amino acids
   (B) TYPE: amino acid
   (C) STRANDEDNESS: single
   (D) TOPOLOGY: linear
(ii) MOLECULE TYPE: peptide
(xi) SEQUENCE DESCRIPTION: SEQ ID NO:12:
   Ala Ile Lys Lys Ala His Ile Glu Lys Asp Phe Ile Ala Phe Cys Ser
   1               5                   10                  15
   Ser Thr Pro Asp Asn Val Ser Trp Arg His Pro Thr Met Gly Ser Val
               20                  25                  30
   Phe Ile Gly Arg Leu Ile Glu His Met Gln Glu Tyr Ala Cys Ser Cys
           35                  40                  45
   Asp Val Glu Glu Ile Phe Arg Lys Val Arg Phe Ser Phe Glu Gln Pro
       50                  55                  60
   Asp Gly Arg Ala Gln Met Pro Thr Thr Glu
   65                  70

### (2) INFORMATION FOR SEQ ID NO:13:

(i) SEQUENCE CHARACTERISTICS:
   (A) LENGTH: 582 base pairs
   (B) TYPE: nucleic acid
   (C) STRANDEDNESS: single
   (D) TOPOLOGY: linear
(ii) MOLECULE TYPE: cDNA
(xi) SEQUENCE DESCRIPTION: SEQ ID NO:13:
   TCTCAAGGAG TACTTTCTTC CTTTCCAGCT CCTCAGGCAG TGCAGGACAA CCCAGCTATG
   CCCACATCCT CAGGCTCAGA AGGGAATGTC AAGCTTTGCT CCCTAGAAGA AGCTCAAAGG
   ATATGGAAAC AAAAGTCGGC AGAGATTTAT CCAATAATGG ACAAGTCAAG CCGCACACGT
   CTTGCTCTCA TTATCTGCAA TGAAGAATTT GACAGTATTC CTAGAAGAAC TGGAGCTGAG
   GTTGACATCA CAGGCATGAC AATGCTGCTA CAAAATCTGG GGTACAGCGT AGATGTGAAA
   AAAAATCTCA CTGCTTCGGA CATGACTACA GAGCTGGAGG CATTTGCACA CCGCCCAGAG
   CACAAGACCT CTGACAGCAC GTTCCTGGTG TTCATGTCTC ATGGTATTCG GGAAGGCATT
   TGTGGGAAGA AACACTCTGA GCAAGTCCCA GATATACTAC AACTCAATGC AATCTTTAAC
   ATGTTGAATA CCAAGAACTG CCCAAGTTTG AAGGACAAAC CGAAGGTGAT CATCATCCAG
   GCCTGCCGTG GTGACAGCCC TGGTGTGGTG TGGTTTAAAG AT

### (2) INFORMATION FOR SEQ ID NO:14:

(i) SEQUENCE CHARACTERISTICS:
   (A) LENGTH: 534 base pairs
   (B) TYPE: nucleic acid
   (C) STRANDEDNESS: single
   (D) TOPOLOGY: linear
(ii) MOLECULE TYPE: cDNA
(xi) SEQUENCE DESCRIPTION: SEQ ID NO:14:
   AACCCAGCTA TGCCCACATC CTCAGGCTCA GAAGGGAATG TCAAGCTTTG CTCCCTAGAA
   GAAGCTCAAA GGATATGGAA ACAAAAGTCG GCAGAGATTT ATCCAATAAT GGACAAGTCA
   AGCCGCACAC GTCTTGCTCT CATTATCTGC AATGAAGAAT TTGACAGTAT TCCTAGAAGA
   ACTGGAGCTG AGGTTGACAT CACAGGCATG ACAATGCTGC TACAAAATCT GGGGTACAGC
   GTAGATGTGA AAAAAAATCT CACTGCTTCG GACATGACTA CAGAGCTGGA GGCATTTGCA
   CACCGCCCAG AGCACAAGAC CTCTGACAGC ACGTTCCTGG TGTTCATGTC TCATGGTATT
   CGGGAAGGCA TTTGTGGGAA GAAACACTCT GAGCAAGTCC CAGATATACT ACAACTCAAT
   GCAATCTTTA ACATGTTGAA TACCAAGAAC TGCCCAAGTT TGAAGGACAA ACCGAAGGTG
   ATCATCATCC AGGCCTGCCG TGGTGACAGC CCTGGTGTGG TGTGGTTTAA AGAT

### (2) INFORMATION FOR SEQ ID NO:15:

(i) SEQUENCE CHARACTERISTICS:
   (A) LENGTH: 264 base pairs
   (B) TYPE: nucleic acid
   (C) STRANDEDNESS: single
   (D) TOPOLOGY: linear
(ii) MOLECULE TYPE: cDNA
(xi) SEQUENCE DESCRIPTION: SEQ ID NO:15:
   GCTATTAAGA AAGCCCACAT AGAGAAGGAT TTTATCGCTT TCTGCTCTTC CACACCAGAT
   AATGTTTCTT GGAGACATCC CACAATGGGC TCTGTTTTTA TTGGAAGACT CATTGAACAT
   ATGCAAGAAT ATGCCTGTTC CTGTGATGTG GAGGAAATTT TCCGCAAGGT TCGATTTTCA
   TTTGAGCAGC CAGATGGTAG AGCGCAGATG CCCACCACTG AAAGAGTGAC TTTGACAAGA
   TGTTTCTACC TCTTCCCAGG ACAT

### (2) INFORMATION FOR SEQ ID NO:16:

(i) SEQUENCE CHARACTERISTICS:
   (A) LENGTH: 195 amino acids
   (B) TYPE: amino acid
   (C) STRANDEDNESS: single
   (D) TOPOLOGY: linear
(ii) MOLECULE TYPE: peptide
(xi) SEQUENCE DESCRIPTION: SEQ ID NO:16:
   Asp Ser Gln Gly Val Leu Ser Ser Phe Pro Ala Pro Gln Ala Val Gln
   1               5                   10                  15
   Asp Asn Pro Ala Met Pro Thr Ser Ser Gly Ser Glu Gly Asn Val Lys
               20                  25                  30
   Leu Cys Ser Leu Glu Glu Ala Gln Arg Ile Trp Lys Gln Lys Ser Ala
           35                  40                  45
   Glu Ile Tyr Pro Ile Met Asp Lys Ser Ser Arg Thr Arg Leu Ala Leu
       50                  55                  60
   Ile Ile Cys Asn Glu Glu Phe Asp Ser Ile Pro Arg Arg Thr Gly Ala
   65                  70                  75                  80
   Glu Val Asp Ile Thr Gly Met Thr Met Leu Leu Gln Asn Leu Gly Tyr
                   85                  90                  95
   Ser Val Asp Val Lys Lys Asn Leu Thr Ala Ser Asp Met Thr Thr Glu
               100                 105                 110
   Leu Glu Ala Phe Ala His Arg Pro Glu His Lys Thr Ser Asp Ser Thr
           115                 120                 125
   Phe Leu Val Phe Met Ser His Gly Ile Arg Glu Gly Ile Cys Gly Lys
       130                 135                 140
   Lys His Ser Glu Gln Val Pro Asp Ile Leu Gln Leu Asn Ala Ile Phe
   145                 150                 155                 160
   Asn Met Leu Asn Thr Lys Asn Cys Pro Ser Leu Lys Asp Lys Pro Lys
                   165                 170                 175
   Val Ile Ile Ile Gln Ala Cys Arg Gly Asp Ser Pro Gly Val Val Trp
               180                 185                 190
   Phe Lys Asp
           195

### (2) INFORMATION FOR SEQ ID NO:17:

(i) SEQUENCE CHARACTERISTICS:
   (A) LENGTH: 178 amino acids
   (B) TYPE: amino acid
   (C) STRANDEDNESS: single
   (D) TOPOLOGY: linear
(ii) MOLECULE TYPE: peptide
(xi) SEQUENCE DESCRIPTION: SEQ ID NO:17:
   Asp Pro Ala Met Pro Thr Ser Ser Gly Ser Glu Gly Asn Val Lys Leu
   1 5 10 15
   Cys Ser Leu Glu Glu Ala Gln Arg Ile Trp Lys Gln Lys Ser Ala Glu
   20 25 30
   Ile Tyr Pro Ile Met Asp Lys Ser Ser Arg Thr Arg Leu Ala Leu Ile
   35 40 45
   Ile Cys Asn Glu Glu Phe Asp Ser Ile Pro Arg Arg Thr Gly Ala Glu
   50 55 60
   Val Asp Ile Thr Gly Met Thr Met Leu Leu Gln Asn Leu Gly Tyr Ser
   65 70 75 80
   Val Asp Val Lys Lys Asn Leu Thr Ala Ser Asp Met Thr Thr Glu Leu
   85 90 95
   Glu Ala Phe Ala His Arg Pro Glu His Lys Thr Ser Asp Ser Thr Phe
   100 105 110
   Leu Val Phe Met Ser His Gly Ile Arg Glu Gly Ile Cys Gly Lys Lys
   115 120 125
   His Ser Glu Gln Val Pro Asp Ile Leu Gln Leu Asn Ala Ile Phe Asn
   130 135 140
   Met Leu Asn Thr Lys Asn Cys Pro Ser Leu Lys Asp Lys Pro Lys Val
   145 150 155 160
   Ile Ile Ile Gln Ala Cys Arg Gly Asp Ser Pro Gly Val Val Trp Phe
   165 170 175
   Lys Asp

### (2) INFORMATION FOR SEQ ID NO:18:

(i) SEQUENCE CHARACTERISTICS:
   (A) LENGTH: 88 amino acids
   (B) TYPE: amino acid
   (C) STRANDEDNESS: single
   (D) TOPOLOGY: linear
(ii) MOLECULE TYPE: peptide
(xi) SEQUENCE DESCRIPTION: SEQ ID NO:18:
   Ala Ile Lys Lys Ala His Ile Glu Lys Asp Phe Ile Ala Phe Cys Ser
   1               5                   10                  15
   Ser Thr Pro Asp Asn Val Ser Trp Arg His Pro Thr Met Gly Ser Val
               20                  25                  30
   Phe Ile Gly Arg Leu Ile Glu His Met Gln Glu Tyr Ala Cys Ser Cys
           35                  40                  45
   Asp Val Glu Glu Ile Phe Arg Lys Val Arg Phe Ser Phe Glu Gln Pro
       50                  55                  60
   Asp Gly Arg Ala Gln Met Pro Thr Thr Glu Arg Val Thr Leu Thr Arg
   65                  70                  75                  80
   Cys Phe Tyr Leu Phe Pro Gly His
                   85

### (2) INFORMATION FOR SEQ ID NO:19:

(i) SEQUENCE CHARACTERISTICS:
   (A) LENGTH: 21 amino acids
   (B) TYPE amino acid
   (C) STRANDEDNESS: single
   (D) TOPOLOGY: linear
(ii) MOLECULE TYPE: peptide
(xi) SEQUENCE DESCRIPTION: SEQ ID NO:19: